(19) Europäisches Patentamt
European Patent Office
Office européen des brevets

(11) EP 0 768 898 B1

(12) EUROPÄISCHE PATENTSCHRIFT

(45) Veröffentlichungstag und Bekanntmachung des Hinweises auf die Patenterteilung:
**09.04.2003 Patentblatt 2003/15**

(21) Anmeldenummer: **95925817.9**

(22) Anmeldetag: **04.07.1995**

(51) Int Cl.⁷: **A61K 49/00**, A61K 51/06

(86) Internationale Anmeldenummer:
**PCT/EP95/02577**

(87) Internationale Veröffentlichungsnummer:
**WO 96/001655 (25.01.1996 Gazette 1996/05)**

(54) **KASKADEN-POLYMER-KOMPLEXE UND IHRE VERFAHREN**

CASCADE-POLYMER COMPLEXES AND METHODS OF PRODUCING THE SAME

COMPLEXES DE POLYMERES EN CASCADE ET LEURS PROCEDES DE PRODUCTION

(84) Benannte Vertragsstaaten:
**AT BE CH DE DK ES FR GB GR IE IT LI LU MC NL PT SE**

(30) Priorität: **07.07.1994 DE 4425857**

(43) Veröffentlichungstag der Anmeldung:
**23.04.1997 Patentblatt 1997/17**

(73) Patentinhaber: **SCHERING AKTIENGESELLSCHAFT**
**13353 Berlin (DE)**

(72) Erfinder:
- **SCHMITT-WILLICH, Heribert**
  **12161 Berlin (DE)**
- **PLATZEK, Johannes**
  **12621 Berlin (DE)**
- **NIEDBALLA, Ulrich**
  **14195 Berlin (DE)**
- **RADÜCHEL, Bernd**
  **13465 Berlin (DE)**
- **MÜHLER, Andreas**
  **Wayne, NJ 07470 (US)**
- **FRENZEL, Thomas**
  **12247 Berlin (DE)**
- **EBERT, Wolfgang**
  **12203 Berlin (DE)**

(56) Entgegenhaltungen:
**EP-A- 0 430 863** **WO-A-93/06868**
**DE-A- 4 344 460**

- **MAGNETIC RESONANCE IN MEDICINE, Bd. 32, Nr. 5, 1.November 1994 Seiten 622-628, XP 000479919 ADAM G ET AL 'DYNAMIC CONTRAST-ENHANCED MR IMAGING OF THE UPPER ABDOMEN: ENHANCEMENT PROPERTIES OF GADOBUTROL GADOLINIUM-DTPA-POLYLYSINE, AND GADOLINIUM-DTPA-CASCADE-POLYMER'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 768 898 B1

## Beschreibung

Die Erfindung betrifft den in den Patentansprüchen gekennzeichneten Gegenstand, das heißt neue Kaskaden-Polymer-Komplexe, diese Verbindungen enthaltende Mittel, die Verwendung der Komplexe in der Diagnostik und Therapie sowie Verfahren zur Herstellung dieser Verbindungen und Mittel.

Die zur Zeit klinisch eingesetzten Kontrastmittel für die modernen bildgebenden Vetfahren Kernspintomographie (MRI) und Computertomographie (CT) [Magnevist ®, Pro Hance ®, Ultravist® und Omniscan ®] verteilen sich im gesamten extrazellulären Raum des Körpers (Intravasalraum und Interstitium). Dieser Verteilungsraum umfaßt etwa 20 % des Körpervolumens.

Extrazelluläre MRI-Kontrastmittel sind klinisch zuerst erfolgreich bei der Diagnostik von zerebralen und spinalen Krankheitsprozessen eingesetzt worden, da sich hier eine ganz besondere Situation hinsichtlich des regionalen Verteilungsraumes ergibt. Im Gehirn und im Rückenmark können extrazelluläre Kontrastmittel im gesunden Gewebe aufgrund der Blut-Hirn-Schranke nicht den Intravasalraum verlassen. Bei krankhaften Prozessen mit Störung der Blut-Hirn-Schranke (z.B. maligne Tumoren, Entzündungen, demyelinisierende Erkrankungen etc.) entstehen innerhalb des Hirns dann Regionen mit erhöhter Blutgefäß-Durchlässigkeit (Permeabilität) für diese extrazellulären Kontrastmittel (Schmiedl et al., MRI of blood-brain barrier permeability in astrocytic gliomas: application of small and large molecular weight contrast media, Magn. Reson. Med. 22: 288, 1991). Durch das Ausnutzen dieser Störung der Gefäßpermeabilität kann erkranktes Gewebe mit hohem Kontrast gegenüber dem gesunden Gewebe erkannt werden.

Außerhalb des Gehirns und des Rückenmarkes gibt es allerdings eine solche Permeabilitätsbarriere für die oben genannten Kontrastmittel nicht (Canty et al., First-pass entry of nonionic contrast agent into the myocardial extravascular space. Effects on radiographic estimate of transit time and blood volume. Circulation 84: 2071, 1991). Damit ist die Anreicherung des Kontrastmittels nicht mehr abhängig von der Gefäßpermeabilität, sondern nur noch von der Größe des extrazellulären Raumes im entsprechenden Gewebe. Eine Abgrenzung der Gefäße gegenüber dem umliegenden interstitiellen Raum bei Anwendung dieser Kontrastmittel ist nicht möglich.

Besonders für die Darstellung von Gefäßen wäre ein Kontrastmittel wünschenswert, das sich ausschließlich im vasalen Raum (Gefäßraum) verteilt. Ein solches blood-pool-agent soll es ermöglichen, mit Hilfe der Kernspintomographie gut durchblutetes von schlecht durchblutetem Gewebe abzugrenzen und somit eine Ischämie zu diagnostizieren. Auch infarziertes Gewebe ließe sich aufgrund seiner Anämie vom umliegenden gesunden oder ischämischen Gewebe abgrenzen, wenn ein vasales Kontrastmittel angewandt wird. Dies ist von besonderer Bedeutung, wenn es z.B. darum geht, einen Herzinfarkt von einer Ischämie zu unterscheiden.

Bisher müssen sich die meisten der Patienten, bei denen Verdacht auf eine kardiovaskuläre Erkrankung besteht (diese Erkrankung ist die häufigste Todesursache in den westlichen Industrieländern), invasiven diagnostischen Untersuchungen unterziehen. In der Angiographie wird zur Zeit vor allem die Röntgen-Diagnostik mit Hilfe von jodhaltigen Kontrastmitteln angewandt. Diese Untersuchungen sind mit verschiedenen Nachteilen behaftet: sie sind mit dem Risiko der Strahlenbelastung verbunden, sowie mit Unannehmlichkeiten und Belastungen, die vor allem daher kommen, daß die jodhaltigen Kontrastmittel, verglichen mit NMR-Kontrastmitteln, in sehr viel höherer Konzentration angewandt werden müssen.

Es besteht daher ein Bedarf an NMR-Kontrastmitteln, die den vasalen Raum markieren können (blood-pool-agent). Diese Verbindungen sollen sich durch eine gute Verträglichkeit und durch eine hohe Wirksamkeit (hohe Steigerung der Signalintensität bei MRI) auszeichnen.

Der Ansatz, zumindest einen Teil dieser Probleme durch Verwendung von Komplexbildnern, die an Makro- oder Biomoleküle gebunden sind, zu lösen, war bisher nur sehr begrenzt erfolgreich.

So ist beispielsweise die Anzahl paramagnetischer Zentren in den Komplexen, die in den Europäischen Patentanmeldungen Nr. 0 088 695 und Nr. 0150 844 beschrieben sind, für eine zufriedenstellende Bildgebung nicht ausreichend.

Erhöht man die Anzahl der benötigten Metallionen durch mehrfache Einführung komplexierender Einheiten in ein makromolekulares Biomolekül, so ist das mit einer nicht tolerierbaren Beeinträchtigung der Affinität und/oder Spezifizität dieses Biomoleküls verbunden [J. Nucl. Med. 24, 1158 (1983)).

Makromoleküle können generell als Kontrastmittel für die Angiographie geeignet sein. Albumin-GdDTPA (Radiology 1987; 162: 205) z.B. zeigt jedoch 24 Stunden nach intravenöser Injektion bei der Ratte eine Anreicherung im Lebelgewebe, die fast 30 % der Dosis ausmacht. Außerdem werden in 24 Stunden nur 20 % der Dosis eliminiert.

Das Makromolekül Polylysin-GdDTPA (Europäische Patentanmeldung, Publikations-Nr. 0 233 619) erwies sich ebenfalls geeignet als blood-pool-agent. Diese Verbindung besteht jedoch herstellungsbedingt aus einem Gemisch von Molekülen verschiedener Größe. Bei Ausscheidungsversuchen bei der Ratte konnte gezeigt werden, daß dieses Makromolekül unverändert durch glomeruläre Filtration über die Niere ausgeschieden wird. Synthesebedingt kann Polylysin-GdDTPA aber auch Makromoleküle enthalten, die so groß sind, daß sie bei der glomerulären Filtration die Kapillaren der Niere nicht passieren können und somit im Körper zurückbleiben.

Auch makromolekulare Kontrastmittel auf der Basis von Kohlenhydraten, z.B. Dextran, sind beschrieben worden (Europäische Patentanmeldung, Publikations-Nr. 0326226).
Der Nachteil dieser Verbindungen liegt darin, daß diese in der Regel nur ca. 5 % des signalverstärkenden paramagnetischen Kations tragen.

Die in der Europäischen Patentanmeldung Nr. 0 430 863 beschriebenen Polymere stellen bereits einen Schritt auf dem Wege zu blood-pool-agents dar, da sie nicht mehr die für die vorher erwähnten Polymere charakteristische Heterogenität bezüglich Größe und Molmasse aufweisen. Sie lassen jedoch immer noch Wünsche im Hinblick auf vollständige Ausscheidung, Verträglichkeit und/oder Wirksamkeit offen.

Es bestand daher die Aufgabe, neue diagnostische Mittel vor allem zur Erkennung und Lokalisierung von Gefäßkrankheiten, die die genannten Nachteile nicht besitzen, zur Verfügung zu stellen. Diese Aufgabe wird durch die vorliegende Erfindung gelöst.

Es wurde gefunden, daß sich Komplexe, die aus stickstoffhaltigen, mit komplexbildenden Liganden versehenen Kaskaden-Polymeren, mindestens 16 Ionen eines Elements der Ordnungszahlen 20 - 29, 39, 42, 44 oder 57 - 83 sowie gegebenenfalls Kationen anorganischer und/oder organischer Basen, Aminosäuren oder Aminosäureamide bestehen und gegebenenfalls acylierte Aminogruppen enthalten, überraschenderweise hervorragend zur Herstellung von NMR- und Röntgen-Diagnostika ohne die genannten Nachteile aufzuweisen, eignen.

Die erfindungsgemäßen komplexbildenden Kaskaden-Polymere lassen sich durch die allgemeine Formel I beschreiben

$$A\text{-}\{X\text{-}[Y\{Z\text{-}\langle W\text{-}K_w\rangle_z)_y]_x\}_a \qquad \text{(I)},$$

worin

| | |
|---|---|
| A | für einen stickstoffhaltigen Kaskadenkern der Basismultiplizität a, |
| X und Y | unabhängig voneinander für eine direkte Bindung oder eine Kaskadenreproduktionseinheit der Reproduktionsmultiplizität x bzw. y, |
| Z und W | unabhängig voneinander für eine Kaskadenreproduktionseinheit der Reproduktionsmultiplizität z bzw. w, |
| K | für den Rest eines Komplexbildners, |
| a | für die Ziffern 2 bis 12, |
| x, y, z und w | unabhängig voneinander für die Ziffern 1 bis 4 stehen, |

mit der Maßgabe, daß mindestens zwei Reproduktionseinheiten unterschiedlich sind und daß für das Produkt der Multiplizitäten gilt

$$16 \le a \cdot x \cdot y \cdot z \cdot w \le 64.$$

Als Kaskadenkern A sind geeignet:

Stickstoffatom,

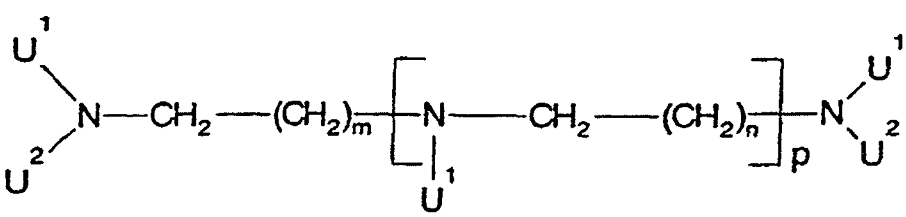

,

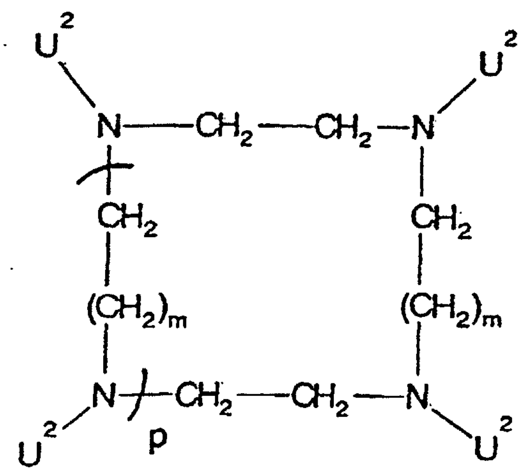

,

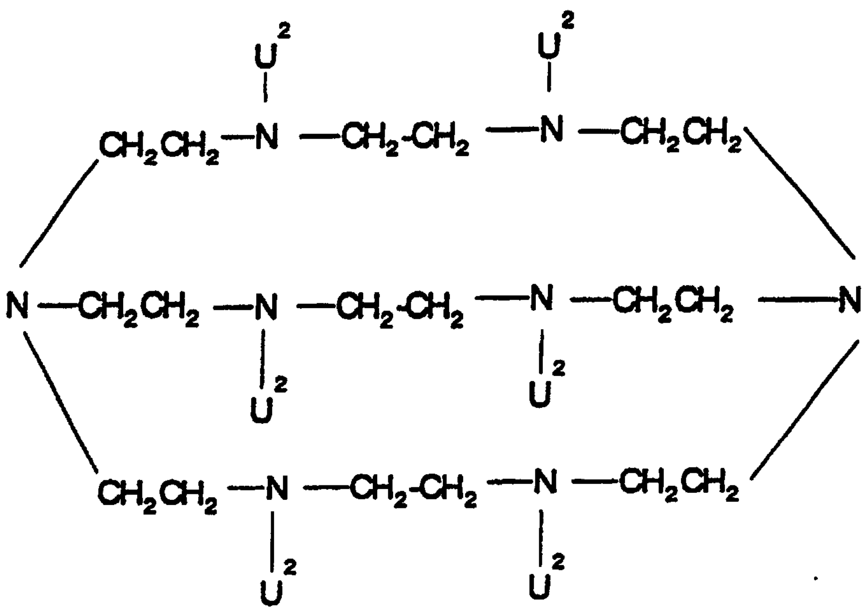

,

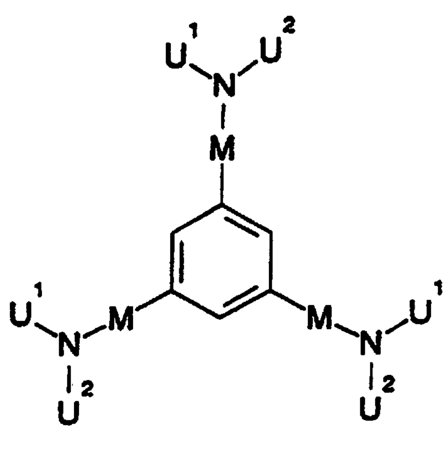

,

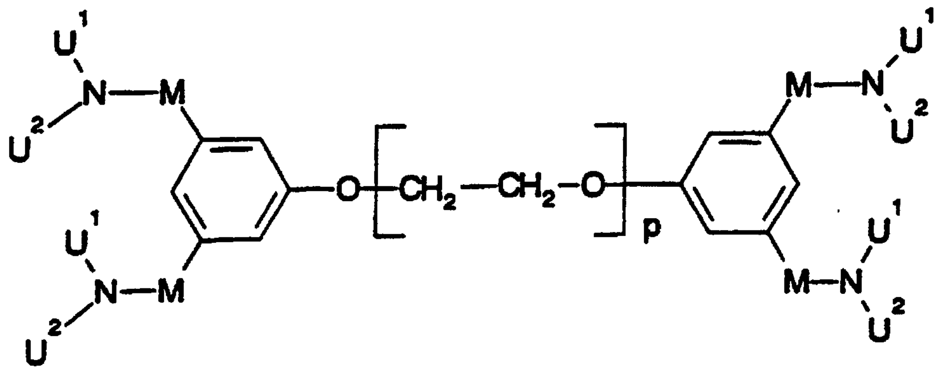

,

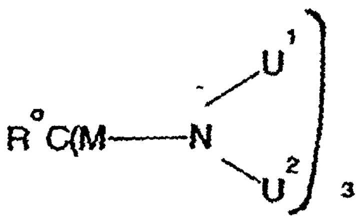

,

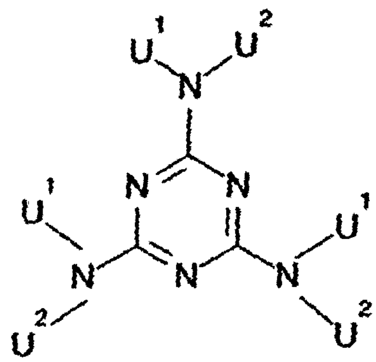

,

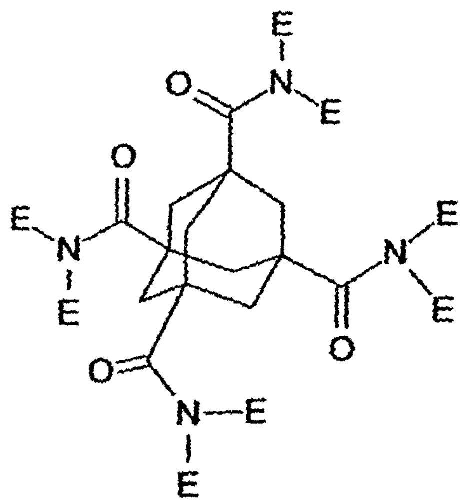

,

worin

m und n für die Ziffern 1 bis 10,
p für die Ziffern 0 bis 10,
$U^1$ für $Q^1$ oder E,
$U^2$ für $Q^2$ oder E mit

E in der Bedeutung der Gruppe

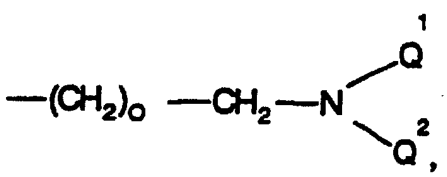

wobei

o für die Ziffern 1 bis 6,

$Q^1$ für ein Wasserstoffatom oder $Q^2$ und

$Q^2$ für eine direkte Bindung

M für eine $C_1$-$C_{10}$-Alkylenkette, die gegebenenfalls durch 1 bis 3 Sauerstoffatome unterbrochen ist und/oder gegebenenfalls mit 1 bis 2 Oxogruppen substituiert ist,

R° für einen verzweigten oder unverzweigten $C_1$-$C_{10}$-Alkylrest, eine Nitro-, Amino-, Carbonsäuregruppe oder für

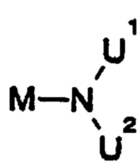

stehen, wobei die Anzahl $Q^2$ der Basismultiplizität a entspricht.

Den einfachsten Fall eines Kaskadenkerns stellt das Stickstoffatom dar, dessen drei Bindungen (Basismultiplizität a = 3) in einer ersten "inneren Schicht" (Generation 1) von drei Reproduktionseinheiten X bzw. Y (wenn X für eine direkte Bindung steht) bzw. Z (wenn X und Y jeweils für eine direkte Bindung stehen) besetzt sind; anders formuliert: die drei Wasserstoffatome des zugrundeliegenden Kaskadenstarters Ammoniak $A(H)_a$ = $NH_3$ sind durch drei Reproduktionseinheiten X bzw. Y bzw. Z substituiert worden. Die im Kaskadenkern A enthaltene Anzahl $Q^2$ gibt dabei die Basismulitplizität a wieder.

Die Reproduktionseinheiten X, Y, Z und W enthalten $-NQ^1Q^2$-Gruppen, worin $Q^1$ ein Wasserstoffatom oder $Q^2$ und $Q^2$ eine direkte Bindung bedeuten. Die in der jeweiligen Reproduktionseinheit (z.B. X) enthaltene Anzahl $Q^2$ entspricht der Reproduktionsmultiplizität dieser Einheit (z.B. x im Falle von X). Das Produkt aller Multiplizitäten $a \cdot x \cdot y \cdot z \cdot w$ gibt die Anzahl der im Kaskadenpolymeren gebundenen Komplexbildner-Reste K an. Die erfindungsgemäßen Polymere enthalten mindestens 16 und höchstens 64 Reste K im Molekül, die jeweils ein bis maximal drei (im Falle von zweiwertigen Ionen), vorzugsweise ein Ion, eines Elements der oben genannten Ordnungszahlen binden können.

Die letzte Generation, d.h. die an die Komplexbildner-Reste K gebundene Reproduktionseinheit W ist über NH-Gruppen ($-NQ^1Q^2$ mit Q1 in der Bedeutung eines Wasserstoffatoms und $Q^2$ = direkte Bindung) an K gebunden, während die vorangehenden Reproduktionseinheiten sowohl über $NHQ^2$-Gruppen (z.B. durch Acylierungsreaktionen) als auch über $NQ^2Q^2$-Gruppen (z.B. durch Alkylierungsreaktionen) miteinander verknüpft sein können.

Die erfindungsgemäßen Kaskaden-Polymer-Komplexe weisen maximal 10 Generationen auf (d.h. es können auch mehr als jeweils nur eine der Reproduktionseinheiten X, Y und Z im Molekül vorhanden sein), vorzugsweise jedoch 2 bis 4 Generationen, wobei mindestens zwei der Reproduktionseinheiten im Molekül unterschiedlich sind.

Als bevorzugte Kaskadenkerne A seien diejenigen angeführt, die unter die oben genannten allgemeinen Formeln fallen, wenn

m für die Ziffern 1 - 3, besonders bevorzugt für die Ziffer 1,
n für die Ziffern 1 - 3, besonders bevorzugt für die Ziffer 1,
p für die Ziffern 0 - 3, besonders bevorzugt für die Ziffer 1,
o für die Ziffer 1,
M für eine $-CH_2-$, -CO- oder $-CH_2CO$-Gruppe und
$R^o$ für eine $-CH_2NU^1U^2-$, $CH_3$- oder $NO_2$-Gruppe

steht.

Als weitere bevorzugte Kaskadenstarter $A(H)_a$ seien z.B. aufgeführt:
(In der Klammer wird die Basismultiplizität a angegeben für den Fall der zum Aufbau der nächsten Generation dienenden nachfolgenden Mono- bzw. Disubstitution)

| | |
|---|---|
| Tris(aminoethyl)amin | (a= 6 bzw. 3); |
| Tris(aminopropyl)amin | (a= 6 bzw.3); |
| Diethylentriamin | (a= 5 bzw. 3); |
| Triethylentetram | (a= 6 bzw. 4); |
| Tetraethylenpentamin | (a= 7 bzw. 5); |
| 1,3,5-Tris(aminomethyl)benzol | (a= 6 bzw.3); |
| Trimesinsäuretriamid | (a= 6 bzw.3); |
| 1,4,7-Triazacyclononan | (a= 3); |
| 1,4,7,10-Tetraazacyclododecan | (a= 4); |
| 1,4,7,10,13-Pentaazacyclopentadecan | (a= 5); |
| 1,4,8,11-Tetraazacyclotetradecan | (a= 4); |
| 1,4,7,10,13,16-Hexaazacyclooctadecan | (a= 6); |
| 1,4,7,10,13,16,19,22,25,28-Decaazacyclotriacontan | (a = 10); |
| Tetrakis(aminomethyl)methan | (a = 8 bzw. 4); |
| 1,1,1-Tris(aminomethyl)ethan | (a = 6 bzw. 3); |
| Tris(aminopropyl)-nitromethan | (a = 6 bzw. 3); |
| 2,4,6-Triamino-1,3,5-triazin | (a = 6 bzw. 3); |
| 1,3,5,7-Adamantantetracatbonsäureamid | (a= 8 bzw. 4); |
| 3,3',5,5'-Diphenylether-tetracarbonsäureamid | (a = 8 bzw. 4); |
| 1,2-Bis[Phenoxyethan]-3',3",5',5"-tetracarbonsäureamid | (a = 8 bzw. 4); |
| 1,4,7,10,13,16,21,24-Octaazabicyclo[8.8.8.]hexacosan | (a = 6). |

Es sei darauf hingewiesen, daß die Definition als Kaskadenkern A und damit die Trennung von Kaskadenkern und erster Reproduktionseinheit rein formal und damit unabhängig von dem tatsächlichen synthetischen Aufbau der gewünschten Kaskaden-Polymer-Komplexe gewählt werden kann. So kann man z.B. das in Beispiel 4 verwendete Tris(aminoethyl)-amin sowohl selbst als Kaskadenkern A (vergleiche die erste für A angegebene allgemeine Formel mit m = n = p = 1, $U^1$ = E mit o in der Bedeutung der Ziffer 1 und $U^1$ = $U^2$ = $Q^2$) aber auch als Stickstoffatom (= Kaskadenkern A), das als erste Generation drei Reproduktionseinheiten

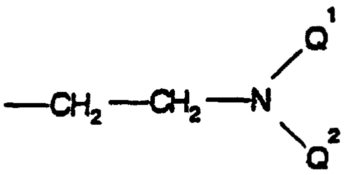

(vergleiche die Definition von E) aufweist, ansehen.

Die Kaskadenreproduktionseinheiten X, Y, Z und W werden unabhängig voneinander durch

E,

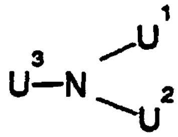

,

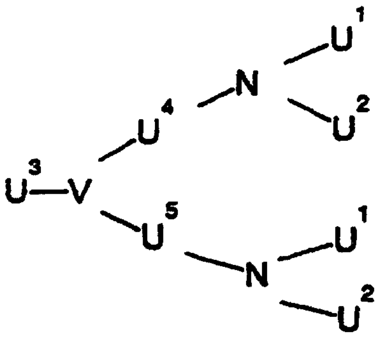

,

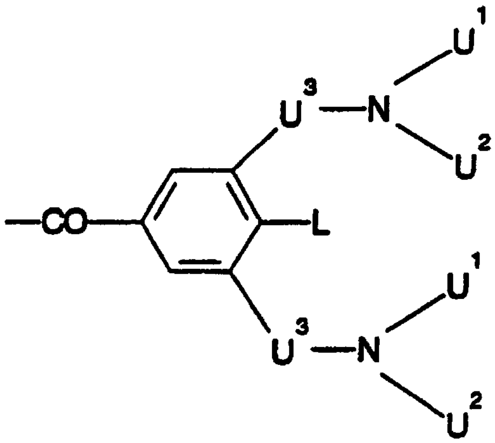

bestimmt,
worin

$U^1$ für $Q^1$ oder E,
$U^2$ für $Q^2$ oder E mit

E in der Bedeutung der Gruppe

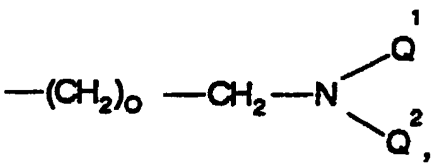

wobei

o für die Ziffern 1 bis 6,

$Q^1$ für ein Wasserstoffatom oder $Q^2$ ,

$Q^2$ für eine direkte Bindung,

$U^3$ für eine $C_1$-$C_{20}$-Alkylenkette, die gegebenenfalls durch 1 bis 10 Sauerstoffatome und/oder 1 bis 2 -$N(CO)_q$-$R^2$-, 1 bis 2 Phenylen- und/oder 1 bis 2 Phenylenoxyreste unterbrochen ist und/oder gegebenenfalls durch 1 bis 2 Oxo-, Thioxo-, Carboxy-, $C_1$-$C_5$-Alkylcarboxy-, $C_1$-$C_5$-Alkoxy-, Hydroxy-, $C_1$-$C_5$-alkylgruppen substituiert ist, wobei

q für die Ziffern 0 oder 1 und
$R^2$ für ein Wasserstoffatom, einen Methyl- oder einen Ethylrest, der gegebenenfalls mit 1 - 2 Hydroxy- oder 1 Carboaygruppe(n) substituiert ist,
L für ein Wasserstoffatom oder die Gruppe

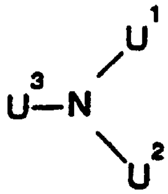

V für die Methingruppe

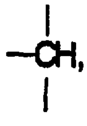

wenn gleichzeitig $U^4$ eine direkte Bindung oder die Gruppe M bedeutet und $U^5$ eine der Bedeutungen von $U^3$ besitzt oder
V für die Gruppe

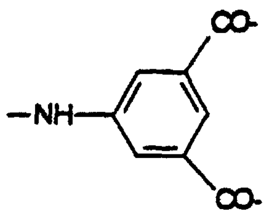

,

wenn gleichzeitig $U^4$ und $U^5$ identisch sind und die direkte Bindung oder die Gruppe M bedeuten,

stehen.

Bevorzugte Kaskadenreproduktionseinheiten X, Y, Z und W sind diejenigen, bei denen in den oben genannten allgemeinen Formeln der Rest $U^3$ für
-CO-, $-COCH_2OCH_2CO-$, $-COCH_2-$, $-CH_2CH_2-$, $-CONHC_6H_4-$, $-COCH_2CH_2CO-$, $-COCH_2-CH_2CH_2CO-$, $-COCH_2CH_2CH_2CH_2CO-$, der Rest $U^4$ für eine direkte Bindung, für $-CH_2CO-$,
der Rest $U^5$ für eine direkte Bindung, für $-(CH_2)_4-$, $-CH_2CO-$, -CH(COOH)-, $CH_2OCH_2CH_2-$, $-CH_2C_6H_4-$, $CH_2-C_6H_4OCH_2CH_2-$,
der Rest E für eine Gruppe

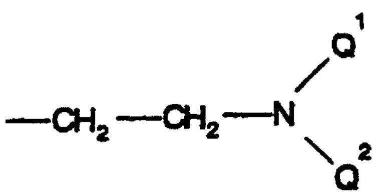

steht

Als beispielhaft genannte Kaskadenreproduktionseinheiten X, Y, Z und W seien angeführt:

$-CH_2CH_2NH-$; $-CH_2CH_2N<$;
$-COCH(NH-)(CH_2)_4NH-$; $-COCH(N<)(CH_2)_4N<$ ;
$-COCH_2OCH_2CON(CH_2CH_2NH-)_2$; $-COCH_2OCH_2CON(CH_2CH_2N<)_2$ ;
$-COCH_2N(CH_2CH_2NH-)_2$; $-COCH_2N(CH_2CH_2N< )_2$;
$-COCH_2NH-$; $-COCH_2N<$ ;
$-COCH_2CH_2CON(CH_2CH_2NH-)_2$; $-COCH_2CH_2CON(CH_2CH_2N<)_2$;
$-COCH_2OCH_2CONH-C_6H_4-CH[CH_2CON(CH_2CH_2NH-)_2]_2$;
$-COCH_2OCH_2CONH-C_6H_4-CH[CH_2CON(CH_2CH_2N< )_2]_2$ ;
$-COCH_2CH_2CO-NH-C_6H_4-CH[CH_2CON(CH_2CH_2NH-)_2]_2$ ;
$-COCH_2CH_2CO-NH-C_6H_4-CH[CH_2CON(CH_2CH_2N<)_2]_2$ ;
$-CONH-C_6H_4-CH[CH_2CON(CH_2CH_2NH-)_2]_2$ ;
$-CONH-C_6H_4-CH[CH_2CON(CH_2CH_2N<)_2]_2$ ;
-COCH(NH-)CH(COOH)NH-; -COCH(N<)CH(COOH)N< ;

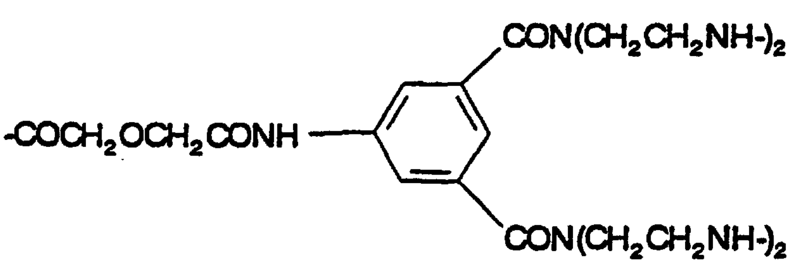

;

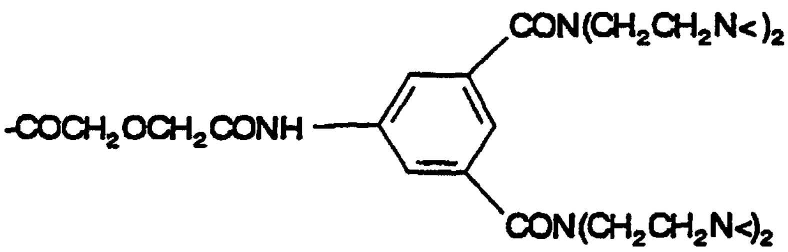

;

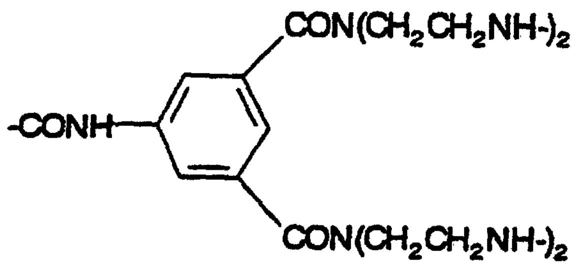

;

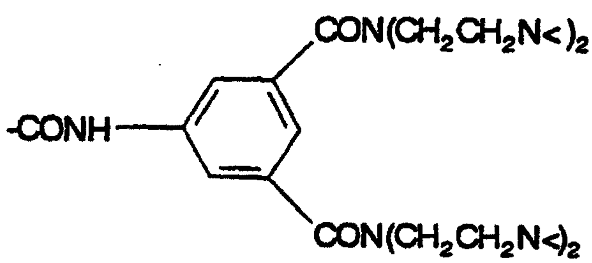

;

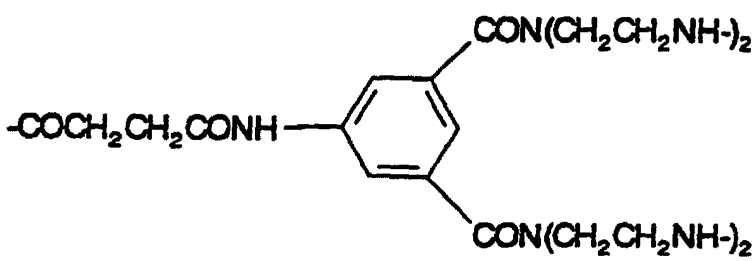

;

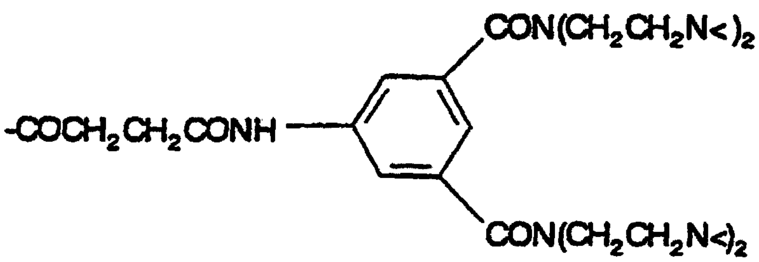

;

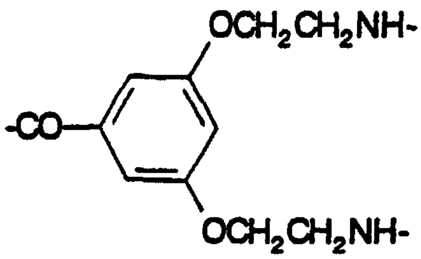

;

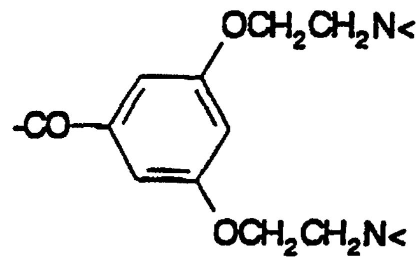

;

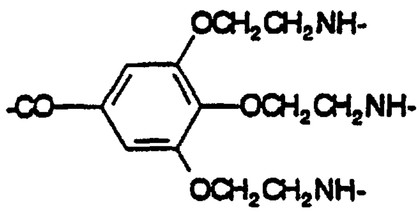

;

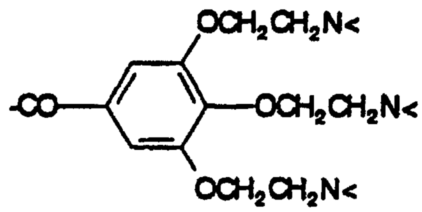

;

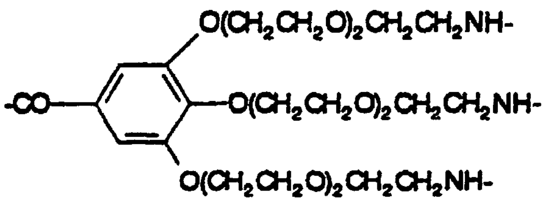

;

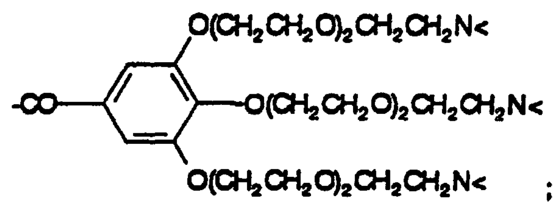

;

Die Komplexbildner-Reste K werden durch die allgemeinen Formeln IA und IB beschrieben:

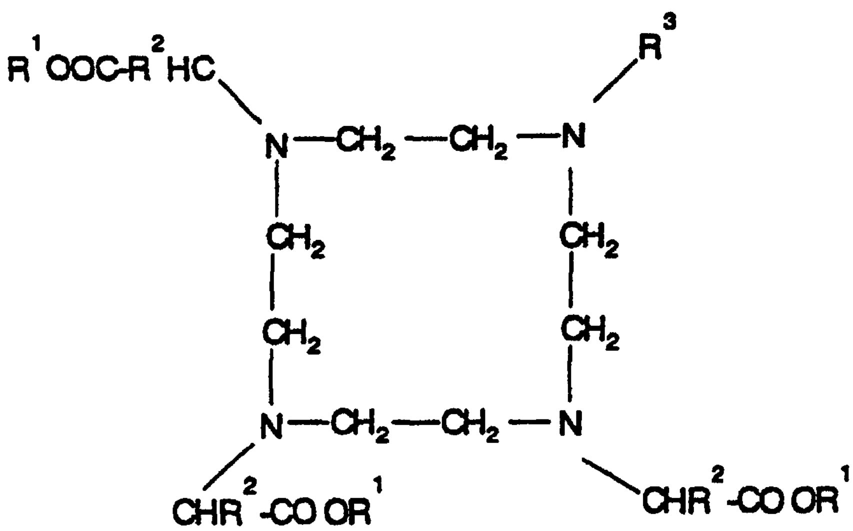

(IA),

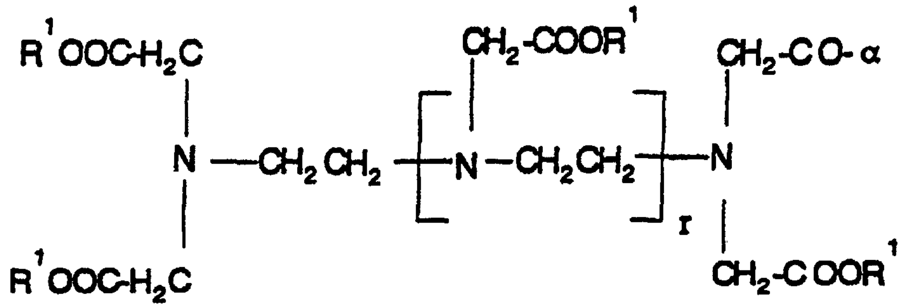

(IB)

steht, worin

$R^1$ unabhängig voneinander für ein Wasserstoffatom oder ein Metallionenäquivalent der Ordnungszahlen 20 - 29, 39, 42 - 44 oder 57 - 83,

$R^2$ für ein Wasserstoffatom, einen Methyl- oder einen Ethylrest, der gegebenenfalls mit 1 - 2 Hydroxy- oder 1 Carboxygruppe(n) substituiert ist,

$R^3$ für eine $-CH_2-CH(OH)-U^6-T-$ oder $-CH_2-CO-U^7$-Gruppe,

$U^6$ für eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1-Thioharnstoff-, 1 - 2 Carboxyalkylimino-, 1 - 2 Estergruppen, 1 - 10 Sauerstoff-, 1 - 5 Schwefel- und/oder 1 - 5 Stickstoff-Atom(e) enthaltende und/oder gegebenenfalls durch 1-5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppe(n) substituierte geradkettige, vetzweigte, gesättigte oder ungesättigte $C_1$-$C_{20}$-Alkylengruppe, wobei die gegebenenfalls enthaltenden Phenylengruppen durch 1 - 2 Carboxy-, 1 - 2 Sulfon- oder 1 - 2 Hydroxygruppen substituiert sein können,

$U^7$ für eine direkte Bindung oder den Rest $-NR^2-U^6-T$

T für eine $-CO-\alpha$, $-NHCO-\alpha-$ oder $-NHCS-\alpha$-Gruppe,

$\alpha$ für die Bindungsstelle an die terminalen Stickstoffatome der letzten Generation, der Reproduktionseinheit W und

r für die Ziffern 0, 1, 2 oder 3

stehen.

Als bevorzugte Komplexbildner-Reste K seien diejenigen genannt, bei denen in der oben angegebenen Formel IA die für $U^6$ stehende $C_1$-$C_{20}$-, bevorzugt $C_1$-$C_{12}$-, Alkylenkette die Gruppen
$-CH_2-$, $-CH_2NHCO-$, $-NHCOCH_2O-$, $-NHCOCH_2OC_6H_4-$, $-N(CH_2CO_2H)-$,
$-NHCOCH_2C_6H_4-$, $-NHCSNHC_6H_4-$, $-CH_2OC_6H_4-$, $-CH_2CH_2O-$,
enthält und/oder durch die Gruppen $-COOH$, $-CH_2COOH$ substituiert ist.

Als Beispiele für $U^6$ seien folgende Gruppen angerührt:

$-CH_2-$, $-CH_2CH_2-$, $-CH_2CH_2CH_2-$, $-C_6H_4-$, $-C_6H_{10}-$, $-CH_2C_6H_5-$,
$-CH_2NHCOCH_2CH(CH_2CO_2H)-C_6H_4-$,
$-CH_2NHCOCH_2OCH_2-$,
$-CH_2NHCOCH_2C_6H_4-$,

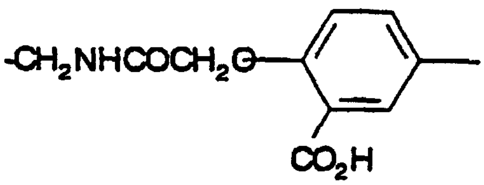

,

$-CH_2NHCSNH-C_6H_4-CH(CH_2COOH)CH_2-$,
$-CH_2OC_6H_4-N(CH_2COOH)CH_2-$,
$-CH_2NHCOCH_2O(CH_2CH_2O)_4-C_6H_4-$,
$-CH_2O-C_6H_4-$,
$-CH_2CH_2-O-CH_2CH_2-$, $-CH_2CH_2-O-CH_2CH_2-O-CH_2CH_2-$,

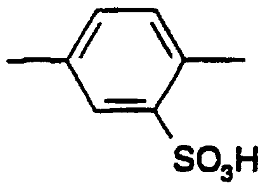

,

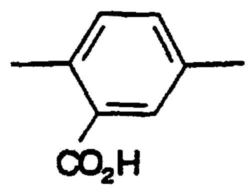

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21 - 29, 42, 44 und 58 - 70. Geeignete Ionen sind beispielsweise das Chrom(III)-, Eisen(II)-, Cobalt (II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gedolinium(III)-, Terbium(III)-, Dysprosium(III)-, Holmium (III)-, Erbium(III)-, Mangan(II)- und Eisen(III)-ion.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21 - 29, 39, 42, 44, 57 - 83 enthalten, geeignet sind; dies sind beispielsweise das Lanthan(III)-ion und die oben genannten Ionen der Lanthanidenreihe.

Die erfindungsgemäßen Kaskaden-Polymer-Komplexe enthalten mindestens 16 Ionen eines Elements der oben genannten Ordnungszahl.

Die restlichen aciden Wasserstoffatome, das heißt diejenigen, die nicht durch das Zentralion substituiert worden sind, können gegebenenfalls ganz oder teilweise durch Kationen von anorganischen und/oder organischen Basen, Aminosäuren oder Aminosäureamiden ersetzt sein.

Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Die erfindungsgemäßen Verbindungen, die ein Molekulargewicht von 10.000 - 80.000 D, vorzugsweise 15.000 - 40.000 D, besitzen, weisen die eingangs geschilderten gewünschten Eigenschaften auf. Sie enthalten die für ihre Vewendung benötigte große Anzahl von Metallionen im Komplex stabil gebunden.

Sie reichern sich in Gebieten mit erhöhter Gefäßpermeabilität, wie z.B. in Tumoren, an, erlauben Aussagen über die Perfusion von Geweben, geben die Möglichkeit, das Blutvolumen in Geweben zu bestimmen, die Relaxationszeiten bzw. Densitäten des Blutes selektiv zu verkürzen, und die Permeabilität der Blutgefäße bildlich darzustellen. Solche physiologischen Informationen sind nicht durch den Einsatz von extrazellulären Kontrastmitteln, wie z.B. Gd-DTPA [Magnevist®], zu erhalten. Aus diesen Gesichtspunkten ergeben sich auch die Einsatzgebiete bei den modernen bildgebenden Verfahren Kernspintomographie und Computertomographie: spezifischere Diagnose von malignen Tumoren, frühe Therapiekontrolle bei zytostatischer, antiphlogistischer oder vasodilatativer Therapie, frühe Erkennung von minderperfundierten Gebieten (z.B. im Myokard), Angio-graphie bei Gefäßerkrankungen, und Erkennung und Diagnose von (sterilen oder infektiösen) Entzündungen.

Als weitere Vorteile gegenüber extrazellulären Kontrastmitteln, wie z.B. Gd-DTPA [Magnevist®], muß die höhere Effektivität als Kontrastmittel für die Kernspintomographie (höhere Relaxivität) hervorgehoben werden, was zu einer deutlichen Reduktion der diagnostisch notwendigen Dosis führt. Gleichzeitig können die erfindungsgemäßen Kontrastmittel als Lösungen isoosmolar zum Blut formuliert werden und verringern dadurch die osmotische Belastung des Körpers, was sich in einer verringerten Toxizität der Substanz (höhere toxische Schwelle) niederschlägt. Geringere Dosen und höhere toxische Schwelle führen zu einer signifikanten Erhöhung der Sicherheit von Kontrastmittelanwendungen bei modernen bildgebenden Verfahren.

Im Vergleich zu den makromolekularen Kontrastmitteln auf der Basis von Kohlenhydraten, z.B. Dextran (Europäische Patentanmeldung, Publikations-Nr. 0 326 226), die - wie erwähnt - in der Regel nur ca. 5 % des signalverstärkenden paramagnetischen Kations tragen, weisen die erfindungsgemäßen Polymer-Komplexe einen Gehalt von in der Regel ca. 20 % des paramagnetischen Kations auf. Somit bewirken die erfindungsgemäßen Makromoleküle pro Molekül eine sehr viel höhere Signalverstärkung, was gleichzeitig dazu führt, daß die zur Kernspintomographie notwendige Dosis gegenüber der makromolekularer Kontrastmittel auf Kohlenhydratbasis erheblich kleiner ist.

Mit den erfindungsgemäßen Polymer-Komplexen ist es gelungen, Makromoleküle so zu konstruieren und herzustellen, daß diese ein einheitlich definiertes Molekulargewicht haben. Es ist somit überraschenderweise möglich, die Größe der Makromoleküle so zu steuern, daß diese groß genug sind, um den vasalen Raum nur langsam verlassen zu können, aber gleichzeitig klein genug, die Kapillaren der Niere, welche 300 - 800 A groß sind, noch passieren zu können.

Im Vergleich zu den anderen erwähnten Polymer-Verbindungen des Stands der Technik zeichnen sich die erfindungsgemäßen Kaskaden-Polymer-Komplexe durch verbessertes Ausscheidungsverhalten, höhere Wirksamkeit, größere Stabilität und/oder bessere Verträglichkeit aus.

Ein weiterer Vorteil der vorliegenden Erfindung liegt darin, daß nunmehr Komplexe mit hydrophilen oder lipophilen, makrocyclischen oder offenkettigen, niedermolekularen oder hochmolekularen Liganden zugänglich geworden sind. Dadurch ist die Möglichkeit gegeben, Verträglichkeit und Pharmakokinetik dieser Polymer-Komplexe durch chemische Substitution zu steuern.

Die Herstellung der erfindungsgemäßen Kaskaden-Polymer-Komplexe erfolgt dadurch, daß man Verbindungen der allgemeinen Formel I'

$$A\text{-}\{X\text{-}[Y\text{-}(Z\text{-}\langle W\text{-}\beta_w\rangle_z)_y]_x\}_a \qquad \text{(I')},$$

worin

| | |
|---|---|
| A | für einen stickstoffhaltigen Kaskadenkern der Basismultiplizität a, |
| X und Y | unabhängig voneinander für eine direkte Bindung oder eine Kaskadenreproduktionseinheit der Reproduktionsmultiplizität x bzw. y, |
| Z und W | unabhängig voneinander für eine Kaskadenreproduktionseinheit der Reproduktionsmultiplizität z bzw. w, |
| a | für die Ziffern 2 bis 12, |
| x, y, z und w | unabhängig voneinander für die Ziffern 1 bis 4 und |
| β | für die Bindungsstelle der terminalen NH-Gruppen der letzten Generation, der Reproduktionseinheit W stehen |

mit der Maßgabe, daß mindestens zwei Reproduktionseinheiten unterschiedlich sind und daß für das Produkt der Multiplizitäten gilt

$$16 \leq a \cdot x \cdot y \cdot z \cdot w \leq 64,$$

mit einem Komplex oder Komplexbildner K' der allgemeinen Formel I'A oder I'B

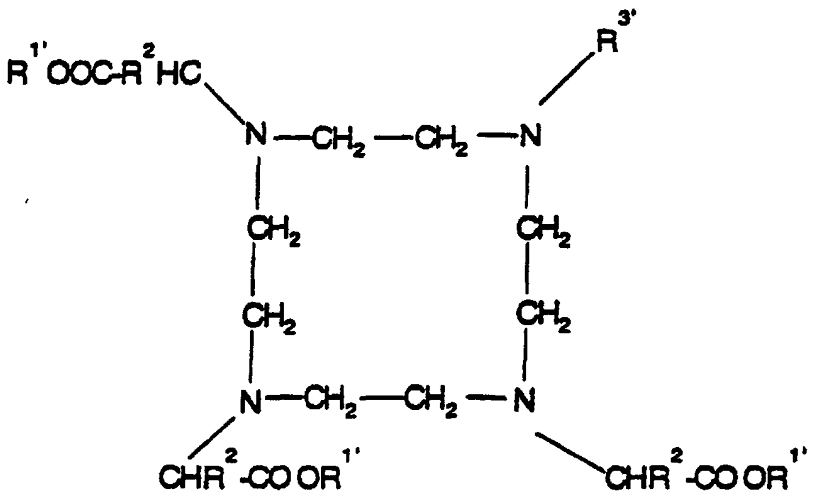

(I'A)

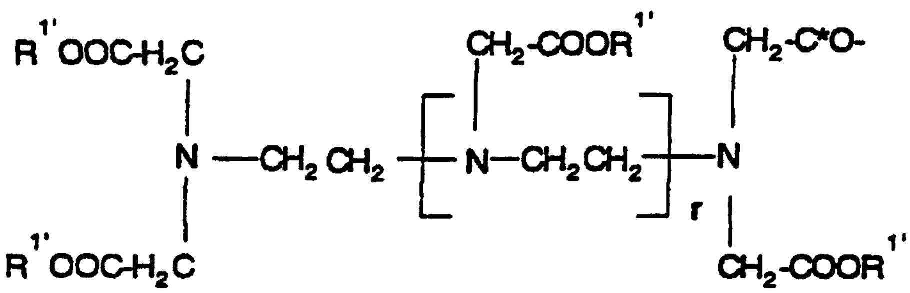

(I'B),

wobei

R1' unabhängig voneinander für ein Wasserstoffatom, ein Metallionenäquivalent der Ordnungszahlen 20 - 29, 39, 42 - 44 oder 57 - 83 oder eine Säureschutzgruppe,

$R^2$ für ein Wasserstoffatom, einen Methyl- oder einen Ethylrest, der gegebenenfalls mit 1 - 2 Hydroxy- oder 1 Carboxygruppe(n) substituiert ist,

$R^{3'}$ für eine $-CH_2-CH(OH)-U^6-T'$ oder $-CH_2CO-U^{7'}$-Gruppe,

$U^6$ für eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1-Thioharnstoff-, 1 - 2 Carboxyalkylimino-, 1 - 2 Estergruppen, 1 - 10 Sauerstoff-, 1 - 5 Schwefel- und/oder 1 - 5 Stickstoff-Atom(e) enthaltende und/oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppe(n) substituierte geradkettige, verzweigte, gesättigte oder ungesättigte $C_1$-$C_{20}$-Alkylengruppe, wobei die gegebenenfalls enthaltenden Phenylengruppen durch 1 - 2 Carboxy-, 1 - 2 Sulfon- oder 1 - 2 Hydroxygruppen substituiert sein können,

$U^{7'}$ für eine direkte Bindung oder den Rest $-NR^2-U^6-T'$,

T' für eine -C*O-, -COOH-, -N=C=O- oder -N=C=S-Gruppe,

C*O für eine aktivierte Carboxylgruppe und

r für die Ziffern 0, 1, 2 oder 3

stehen

mit der Maßgabe, daß - sofern K' für einen Komplex steht - mindestens zwei (bei zweiwertigen Metallen) bzw. drei (bei dreiwertigen Metallen) der Substituenten $R^1$ für ein Metallionenäquivalent der oben genannten Elemente stehen und daß gewünschtenfalls weitere Carboxylgruppen in Form ihrer Salze mit anorganischen und/oder organischen Basen, Aminosäuren oder Aminosäureamiden vorliegen,

umsetzt, gegebenenfalls vorhandene Schutzgruppen abspaltet, die so erhaltenen Kaskaden-Polymere - sofern K' für einen Komplexbildner steht - in an sich bekannter Weise mit mindestens einem Metalloxid oder Metallsalz eines Elements der Ordnungszahlen 20 - 29, 39, 42, 44 oder 57 - 83 umsetzt und gegebenenfalls anschließend in den so erhaltenen Kaskaden-Polymer-Komplexen noch vorhandene acide Wasserstoffatome ganz oder teilweise durch Kationen von anorganischen und/oder organischen Basen, Aminosäuren oder Aminosäureamiden substituiert und gegebenenfalls noch vorhandene freie terminale Aminogruppen gewünschtenfalls - vor oder nach der Metallkomplexierung - acyliert.

Als Beispiel für eine aktivierte Carbonylgruppe C*O in den Komplexen bzw. Komplexbildnem K' seien Anhydrid, p-Nitrophenylester, N-Hydroxysuccinimidester, Pentafluorphenylester und Säurechlorid genannt.

Die zur Einführung der Komplexbildner-Einheiten vorgenommene Addition oder Acylierung wird mit Substraten durchgeführt, die den gewünschten Substituenten K (eventuell gebunden an eine Fluchtgruppe) enthalten, oder aus denen der gewünschte Substituent durch die Reaktion generiert wird.

Als Beispiele für Additionsreaktionen seien die Umsetzung von Isocyanaten und Isothiocyanaten genannt, wobei die Umsetzung von Isocyanaten bevorzugt in aprotischen Solventien wie z.B. THF, Dioxan, DMF, DMSO, Methylenchlorid bei Temperaturen zwischen 0 und 100 °C, bevorzugt zwischen 0 und 50 °C, gegebenenfalls unter Zusatz einer organischen Base wie Triethylamin, Pyridin, Lutidin, N-Ethyldiisopropylamin, N-Methylmorpholin, durchgeführt wird. Die Umsetzung mit Isothiocyanaten wird in der Regel in Lösungsmitteln wie z.B. Wasser oder niederen Alkoholen wie z.B. Methanol, Ethanol, Isopropanol oder deren Mischungen, DMF oder Mischungen aus DMF und Wasser bei Temperaturen zwischen 0 und 100 °C, bevorzugt zwischen 0 und 50 °C, gegebenenfalls unter Zusatz einer organischen oder anorganischen Base wie z.B. Triethylamin, Pyridin, Lutidin, N-Ethyldiisopropylamin, N-Methylmorpholin oder Erdalkali-, Alkalihydroxiden wie z.B. Lithium-, Natrium-, Kalium-, Calciumhydroxid oder deren Carbonate wie z.B. Magnesiumcarbonat, durchgeführt.

Als Beispiele für Acylierungsreaktionen seien die Umsetzung von freien Carbonsäuren nach den dem Fachmann bekannten Methoden [z.B. J.P. Greenstein, M. Winitz, Chemistry of the Amino Acids, John Wiley & Sons, N.Y. (1961), S. 943 - 945] genannt. Als vorteilhaft hat sich jedoch erwiesen, die Carbonsäuregruppe vor der Acylierungsreaktion in eine aktivierte Form wie z.B. Anhydrid, Aktivester oder Säurechlorid zu überführen [z.B. E. Gross, J. Meienhofer, The Peptides, Academic Press, N.Y. (1979), Vol. 1, S. 65 - 314; N.F. Albertson, Org. React. 12, 157 (1962)].

Beispielhaft für die Umsetzung von Anhydriden sei die Umsetzung des Monoanhydrids $N^3$-(2,6-Dioxomorpholinoethyl)-$N^6$-(ethoxycarbonylmethyl)-3,6-diazaoctandisäure mit den jeweils gewünschten, terminale Aminogruppen enthaltenden Kaskaden-Polymeren in Wasser, in polaren Lösungsmitteln wie z.B. Dioxan, THF, DMF, DMSO oder Acetonitril oder deren Mischungen bei basischem pH, vorzugsweise 8 - 10, d.h. unter Zusatz von Basen wie z.B. Natrium-, Kaliumhydroxid, Triethylamin oder Pyridin, bei Temperaturen von 0 - 50 °C, vorzugsweise bei Raumtemperatur, genannt. Zur vollständigen Umsetzung in Wasser arbeitet man vorzugsweise mit einem z.B. 2- bis 3fachen Überschuß Monoanhydrid.

Im Falle der Umsetzung mit Aktivester sei auf die dem Fachmann bekannte Literatur [z.B. Houben-Weyl, Methoden der organischen Chemie, Georg Thieme Verlag, Stuttgart, Band E 5 (1985), 633] verwiesen. Sie kann unter den oben für die Anhydridreaktion angegebenen Bedingungen durchgeführt werden. Es können aber auch aprotische Lösungsmittel wie z.B. Methylenchlorid, Chloroform verwendet werden.

Im Falle der Säurechlorid-Umsetzungen werden nur aprotische Lösungsmittel wie z.B. Methylenchlorid, Chloroform, Toluol oder THF bei Temperaturen zwischen -20 bis 50 °C, bevorzugt zwischen 0 bis 30 °C, verwendet. Des weiteren sei auf die dem Fachmann bekannte Literatur [z.B. Houben-Weyl, Methoden der Organischen Chemie, Georg-Thieme-Verlag, Stuttgart, (1974), Band 15/2, S. 355 - 364] verwiesen.

Falls R1' für eine Säureschutzgruppe steht, kommen niedere Alkyl-, Aryl- und Aralkylgruppen, beispielsweise die Methyl-, Ethyl-, Propyl-, Butyl-, Phenyl-, Benzyl-, Diphenylmethyl-, Triphenylmethyl-, bis-(p-Nitrophenyl)-methylgruppe, sowie Trialkylsilylgruppen in Frage.

Die gegebenenfalls gewünschte Abspaltung der Schutzgruppen erfolgt nach den dem Fachmann bekannten Verfahren, beispielsweise durch Hydrolyse, Hydrogenolyse, alkalische Verseifung der Ester mit Alkali in wäßrig-alkoholischer Lösung bei Temperaturen von 0 °C bis 50 °C oder im Fall von tert.-Butylestern mit Hilfe von Trifluoressigsäure.

Gegebenenfalls unvollständig mit Ligand oder Komplex acylierte terminale Aminogruppen können, wenn gewünscht, in Amide oder Halbamide überführt werden. Beispielhaft genannt sei die Umsetzung mit Acetanhydrid, Bernsteinsäureanhydrid oder Diglykolsäureanhydrid.

Die Einführung der gewünschten Metallionen erfolgt in der Weise, wie sie z.B. in der Deutschen Offenlegungsschrift 34 01 052 offenbart worden ist, indem man das Metalloxid oder ein Metallsalz (beispielsweise das Nitrat,

Acetat, Carbonat, Chlorid oder Sulfat) des Elements der Ordnungszahlen 20 - 29, 42, 44, 57 - 83 in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome der Säuregruppen durch Kationen von anorganischen und/oder organischen Basen, Aminosäuren oder Aminosäureamiden substituiert.

Die Einführung der gewünschten Metallionen kann sowohl auf der Stufe der Komplexbildner I'A oder I'B, d. h. vor der Kopplung an die Kaskaden-Polymere, als auch nach Kopplung der unmetallierten Liganden I'A oder I'B erfolgen.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Omithin oder von Amiden ursprünglich neutraler oder saurer Aminosäuren, wie zum Beispiel Hippursäure, Glycinacetamid.

Zur Herstellung der neutralen Komplexverbindungen kann man beispielsweise den sauren Komplexsalzen in wäßriger Lösung oder Suspension so viel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsaize durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Die Reinigung der so erhaltenen Kaskaden-Polymer-Komplexe erfolgt, gegebenenfalls nach Einstellung des pH-Wertes durch Zusatz einer Säure oder Base auf pH6 bis 8, bevorzugt ca. 7, vorzugsweise durch Ultrafiltration mit Membranen geeigneter Posengröße (z.B. Amicon®XM30, Amicon®YM10, Amicon®YM3) oder Gelfiltration an z.B. geeigneten Sephadex®-Gelen.

Die Herstellung der für die Kopplung an die KompLexbildner K' (bzw. auch die entsprechenden metallhaltigen Komplexe) benötigten terminale Aminogruppen tragenden Kaskaden-Polymere geht im allgemeinen aus von käuflichen bzw. nach oder analog literaturbekannten Methoden herstellbaren stickstoffhaltigen Kaskadenstartem $A(H)_a$. Die Einführung der Generationen X, Y, Z und W erfolgt nach literaturbekannten Methoden [z.B. J. March, Advanced Organic Chemistry, 3$^{rd}$ ed.; John Wiley & Sons, (1985), 364 - 381] durch Acylierungs- bzw. Alkylierungsreaktionen mit die gewünschten Strukturen aufweisenden geschützten Aminen, die zur Bindung an den Kaskadenkern befähigte funktionelle Gruppen wie z.B. Carbonsäuren, Isocyanate, Isothiocyanate oder aktivierte Carbonsäuren (wie z.B. Anhydride, Aktivester, Säurechloride) bzw. Halogenide (wie z.B. Chloride, Bromide, Iodide), Aziridin, Mesylate, Tosylate oder andere dem Fachmann bekannte Fluchtgruppen enthalten.

Es sei jedoch hier nochmals betont, daß die Unterscheidung zwischen Kaskadenkern A und Reproduktionseinheiten rein formal ist. Es kann synthetisch von Vorteil sein, daß man nicht den formalen Kaskadenstarter $A(H)_a$ verwendet, sondern die per Definition zum Kaskadenkern gehörigen Stickstoffatome erst zusammen mit der ersten Generation einfuhrt. So ist es z.B. zur Synthese der in Beispiel 1b) beschriebenen Verbindung vorteilhafter, nicht den formalen Kaskadenkern Trimesinsäuretriamid mit z.B. Benzyloxycarbonylaziridin (sechsfach) zu alkylieren, sondern Trimesinsäuretrichlorid mit Bis[2-(benzyloxycarbonylamino)-ethyl]-amin (dreifach) umzusetzen.

Als Aminschutzgruppen seien die dem Fachmann geläufigen Benzyloxycarbonyl-, tertiär-Butoxycarbonyl-, Trifluoracetyl-, Fluorenylmethoxycarbonyl-, Benzyl- und Formylgruppe [Th. W. Greene, P.G.M Wuts, Protective Groups in Organic Syntheses, 2nd ed, John Wiley and Sons (1991), S. 309-385] genannt. Nach Abspaltung dieser Schutzgruppen, die ebenfalls nach literaturbekannten Methoden erfolgt, kann die nächste gewünschte Generation in das Molekül eingeführt werden. Neben diesem aus jeweils zwei Reaktionsstufen (Alkylierung bzw. Acylierung und Schutzgruppenabspaltung) bestehenden Aufbau einer Generation ist auch mit ebenfalls nur zwei Reaktionsstufen die gleichzeitige Einführung von zwei, z.B. X-[Y]x, oder mehrerer Generationen, z.B. $X\text{-}[Y\text{-}(Z)_y]_x$, möglich. Der Aufbau dieser Mehrgenerationen-Einheiten erfolgt durch Alkylierung bzw. Acylierung von die Strukturen der gewünschten Reproduktionseinheiten aufweisenden ungeschützten Aminen ("Reproduktionsamin") mit einem zweiten Reproduktionsamin, dessen Amingruppen in geschützter Form vorliegen.

Die als Kaskadenstarter benötigten Verbindungen der allgemeinen Formel $A(H)_a$ sind käuflich zu erwerben

oder nach bzw. analog literaturbekannten Methoden [z.B. Houben-Weyl, Methoden der Org. Chemie, Georg-Thieme-Verlag, Stutgart (1957), Bd. 11/1; M. Micheloni et al., Inorg. Chem. (1985), 24, 3702; TJ. Atkins et al., Org. Synth., Vol. 58 (1978), 86 - 98; The Chemistry of Heterocyclic Compounds: J.S. Bradshaw et al., Aza-Crown-Macrocycles, John Wiley & Sons, N.Y. (1993)] herstellbar. Beispielhaft angeführt seien:

Tris(aminoethyl)amin [z.B. Fluka Chemie AG, Schweiz; Aldrich-Chemie, Deutschland]; Tris(aminopropyl)amin [z. B. C. Woemer et al., Angew. Chem. Int. Ed. Engl. (1993), 32, 1306];
Diethylentriamin [z.B. Fluka; Aldrich];
Triethylentetramin [z.B. Fluka; Aldrich];
Tetraethylenpentamin [z.B. Fluka; Aldrich];
1,3,5-Tris(aminomethyl)benzol [z.B. T.M. Garrett et al., J. Am. Chem. Soc. (1991), 113, 2965];
Trimesinsäuretriamid [z.B. H. Kurihara; Jpn. Kokai Tokkyo Koho JP 04077481; CA 117, 162453];
1,4,7-Triazacyclononan [z.B. Fluka; Aldrich];
1,4,7,10,13-Pentaazacyclopentadecan [z.B. K.W. Aston, Eur. Pat. Appl. 0 524 161, CA 120, 44580];
1,4,8,11-Tctraazacyclotetradecan [z.B. Fluka; Aldrich];
1,4,7,10,13,16,19,22,25,28-Decaazacyclotriacontan [z.B. A. Andres et al., J. Chem. Soc. Dalton Trans. (1993), 3507];
1,1,1-Tris(aminomethyl)ethan [z.B. RJ. Geue et al., Aust. J. Chem. (1983), 36, 927];
Tris(aminopropyl)-nitromethan [z.B. G.R. Newkome et al., Angew. Chem. 103, 1205 (1991) analog zu R.C. Larock, Comprehensive Organic Transformations, VCH Publishers, N.Y. (1989), 419-420]
1,3,5,7-Adamantantetracarbonsäureamid [z.B. H. Stetter et al., Tetr. Lett. 1967, 1841];
1,2-Bis[Phenoxyethan]-3',3",5',5"-tetracarbonsäureamid [z.B. J.P. Collman et al.; J. Am. Chem. Soc. (1988), 110, 3477 - 86 analog zur Vorschrift für das Beispiel 1b)];
1,4,7,10,13,16,21,24-Octaazabicyclo[8.8.8.]hexacosan [z.B. P.H. Smith et al., J. Org. Chem. (1993), 58, 7939].

Die Herstellung der für den Aufbau der Generationen benötigten die oben genannten fünktionellen Gruppen enthaltenden Reproduktionsamine erfolgt nach bzw. analog den im experimentellen Teil beschriebenen Vorschriften bzw. nach literaturbekannten Verfahren.

Beispielhaft genannt seien:

$N^{\alpha}$,$N^{\varepsilon}$-Di-CO-O-$CH_2C_6H_5$-Lysin-p-nitropheylester [s. Vorschrift für Beispiel 1c)];
HOOC-$CH_2OCH_2CO$-N($CH_2CH_2NH$-CO-O-$CH_2C_6H_5)_2$ [s. Vorschrift für Beispiel 7a)];
HOOC-$CH_2N(CH_2CH_2NH$-CO-O-$CH_2C_6H_5)_2$ [s. Vorschrift für Beispiel 9e)];
HOOC-$CH_2CH_2CO$-N($CH_2CH_2NH$-$COCF_3)_2$ [herzustellen nach Vorschrift für das Beispiel 7a), indem man anstelle von Bis(benzyloxycarbonylaminoethyl)amin von Bis(trifluoracetylaminoethyl)amin und anstelle von Diglycolsäureanhydrid von Bemsteinsäureanhydrid ausgeht];
HOOC-$CH_2OCH_2CONH$-$C_6H_4$-CH[$CH_2CON(CH_2CH_2NH$-CO-O-$CH_2C_6H_5)_2]_2$ [herzustellen nach Vorschrift für Beispiel 7a), indem man anstelle von Bis(benzyloxycarbonylaminoethyl)amin von dem in Beispiel 9b) beschriebenen Amin ausgeht];
O=C=N-$C_6H_4$-CH[$CH_2CON(CH_2CH_2NH$-CO-O-$CH_2C_6H_5)_2]_2$ [s. Vorschrift für Beispiel 9c)]

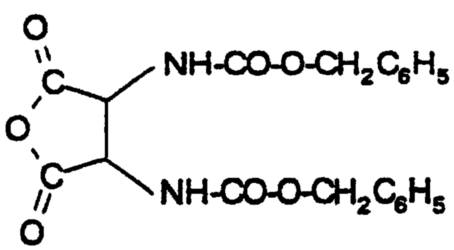

[s. Vorschrift für Beispiel 21b)]

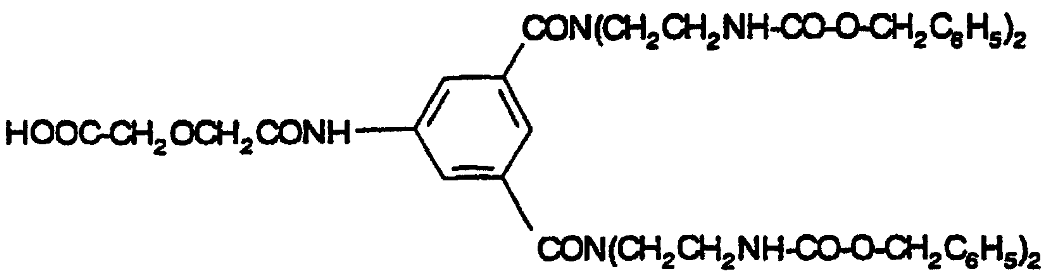

[s. Vorschrift für Beispiel 17f)]

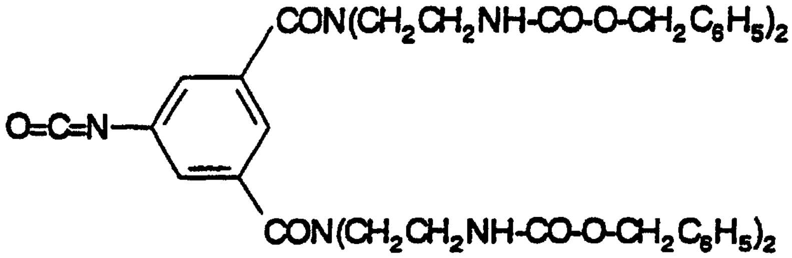

herzustellen nach Vorschrift für Beispiel 17f), indem man anstelle von Diglycolsäureanhydrid mit Triphosgen analog der Vorschrift für Beispiel 9c) umsetzt

N-Benzyloxycarbonyl-aziridin siehe Vorschrift für Beispiel 15a)

N-Benzyloxycarbonyl-glycin käuflich bei z.B. Bachem California

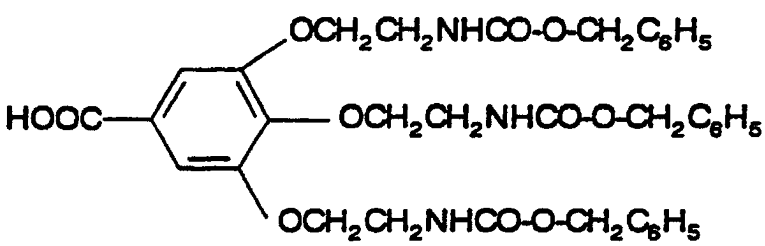

herzustellen nach CJ. Cavallito et al., J. Amer. Chem. Soc. 1943, 65, 2140, indem man anstelle von Benzylchlorid von N-CO-O-$CH_2C_6H_5$-(2-Bromethyl)amin [A.R. Jacobson et al., J. Med. Chem. (1991), 34, 2816] ausgeht.

Die Herstellung der Komplexe und Komplexbildner der allgemeinen Formel I'A und I'B erfolgt nach bzw. analog den im experimentellen Teil beschriebenen Vorschriften bzw. nach literaturbekannten Methoden (s. z.B. Europäische Patentanmeldungen Nr. 0 512 661, 0 430 863, 0 255 471 und 0 565 930.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern oder schwachen Komplexen (wie zum Beispiel Diethylentriaminpentaessigsäure oder die korrespondierenden Ca-Kaskaden-Polymer-Komplexe) oder - falls erforderlich - Elektrolyte wie zum Beispiel Natriumchlorid oder - falls erforderlich - Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) [zum Beispiel Methylcellulose, Lactose, Mannit] und/oder Tensid(en) [zum Beispiel Lecithine, Tween®, Myrj® ] und/oder Aromastoff(en) zur Geschmackskorrektur [zum Beispiel ätherischen Ölen] gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 1µMol -1 Mol/l des Komplexsalzes und werden in der Regel in Mengen von 0,0001 - 5 mMol/kg dosiert. Sie sind zur enteralen und parenteralen Applikation bestimmt. Die erfindungsgemäßen Komplexverbindungen kommen zur Anwendung

1. für die NMR- und Röntgen-Diagnostik in Form ihrer Komplexe mit den Ionen der Elemente mit den Ordnungszahlen 21 - 29, 39, 42, 44 und 57 - 83;

2. für die Radiodiagnostik und Radiotherapie in Form ihrer Komplexe mit den Radioisotopen der Elemente mit den Ordnungszahlen 27, 29, 31, 32, 37 - 39, 43, 49, 62, 64, 70, 75 und 77.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100- bis 1000fach besser wasserlöslich sein als für die NMR-Spektroskopie. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen mittel für die Anwendung als NMR-Diagnostika in Mengen von 0,0001 - 5 mMol/kg, vorzugsweise 0,005 - 0,5 mMol/kg, dosiert. Details der Anwendung werden zum Beispiel in H.-J. Weinmann et al., Am. J. of Roentgenology 142, 619 (1984) diskutiert.

Besonders niedrige Dosierungen (unter 1 mg/kg Körpergewicht) von organspezifischen NMR-Diagnostika sind zum Beispiel zum Nachweis von Tumoren und von Herzinfarkt einsetzbar.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida, beschrieben.

Eine weitere bildgebende Methode mit Radioisotopen ist die Positronen-Emissions-Tomographie, die positronenemittierende Isotope wie z.B. $^{43}Sc$, $^{44}Sc$, $^{52}Fe$, $^{55}Co$ und $^{68}Ga$ verwendet (Heiss, W.D.; Phelps, M.E.; Positron Emission Tomography of Brain, Springer Verlag Berlin, Heidelberg, New York 1983).

Da sich die erfindungsgemäßen Substanzen in malignen Tumoren anreichern (keine Diffusion in gesunde Gewebe, aber hohe Durchlässigkeit von Tumorgefäßen), können sie auch die Strahlentherapie von malignen Tumoren unterstützen. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Hierzu werden Wechselwirkungen der in den Komplexen enthaltenen Metalle (wie z.B. Eisen oder Gadolinium) mit ionisierenden Strahlungen (z.B. Röntgenstrahlen) oder mit Neutronenstrahlen ausgenutzt. Durch diesen Effekt wird die lokale Strahlendosis am Ort, wo sich der Metallkomplex befindet (z.B. in Tumoren) signifikant erhöht. Um die gleiche Strahlendosis im malignen Gewebe zu erzeugen, kann bei Anwendung solcher Metallkomplexe die Strahlenbelastung für gesunde Gewebe erheblich reduziert und damit belastende Nebenwirkungen für die Patienten vermieden werden. Die erfindungsgemäßen Metallkomplex-Konjugate eignen sich deshalb auch als radiosensibilisierende Substanz bei Strahlentherapie von malignen Tumoren (z.B. Ausnutzen von Mössbauer-Effekten

oder bei Neutroneneinfangtherapie). Geeignete β-emittierende Ionen sind zum Beispiel $^{46}Sc$, $^{47}Sc$, $^{48}Sc$, $^{72}Ga$, $^{73}Ga$ und $^{90}Y$. Geeignete geringe Halbwertzeiten aufweisende α-emittierende Ionen sind zum Beispiel $^{211}Bi$, $^{212}Bi$, $^{213}Bi$ und $^{214}Bi$, wobei $^{212}Bi$ bevorzugt ist. Ein geeignetes Photonen- und Elektronenemittierendes Ion ist $^{158}Gd$, das aus $^{157}Gd$ durch Neutronen-einfang erhalten werden kann.

Ist das erfindungsgemäße Mittel zur Anwendung in der von R.L. Mills et al. (Nature Vol. 336, (1988), S. 787] vorgeschlagenen Variante der Strahlentherapie bestimmt, so muß sich das Zentralion von einem Mößbauer-Isotop wie beispielsweise $^{57}Fe$ oder $^{151}Eu$ ableiten.

Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die erfindungsgemäßen therapeutischen Mittel werden parenteral, vorzugsweise i.v., appliziert.

Details der Anwendung von Radiotherapeutika werden z.B. in R.W. Kozak et al. TIBTEC, Oktober 1986, 262, diskutiert.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemischpharmakologischen Untersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zu zum Beispiel Meglumin-Diatrizoat in Mengen von 0,1 - 5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler "Einführung in die Röntgendiagnostik", G. Thieme, Stuttgart, New York (1977) diskutiert.

Insgesamt ist es gelungen, neue Komplexbildner, Metallkomplexe und Metallkomplexsalze zu synthetisieren, die neue Möglichkeiten in der diagnostischen und therapeutischen Medizin erschließen.

Die nachfolgenden Beispiele dienen zur näheren Erläuterung des Erfindungsgegenstands:

**Beispiel 1**

a) Bis[2-(benzyloxycarbonylamino)-ethyl]-amin

51,5 g (500 mmol) Diethylentriamin und 139 ml (1 mol) Triethylamin werden in Dichlormethan gelöst und bei -20°C mit 161 g Benzylcyanformiat (Fluka) in Dichlormethan versetzt und anschließend über Nacht bei Raumtemperatur gerührt. Nach Beendigung der Reaktion wird im Abzug eingedampft, der Rückstand in Diethylether aufgenommen, die organische Phase mit Natriumcarbonatlösung gewaschen und mit Natriumsulfat getrocknet. Das Filtrat wird mit Hexan versetzt, der Niederschlag abfiltriert und getrocknet.
Ausbeute: 163,4 g (88 % d. Th.)

| Elementaranalyse: | | | |
|---|---|---|---|
| ber. | C 64,67 | H 6,78 | N 11,31 |
| gef. | C 64,58 | H 6,83 | N 11,28 |

b) N,N,N',N',N",N"-Hexakis[2-(benzyloxycarbonylamino)-ethyl]-trimesinsäuretriamid

13,27 g (50 mmol) Trimesinsäure-trichlorid (Aldrich) und 34,7 ml (250 mmol) Triethylamin werden in Dimethylformamid (DMF) gelöst und bei 0°C mit 65,0 g (175 mmol) des in Beispiel 1a beschriebenen Amins versetzt und anschließend über Nacht bei Raumtemperatur gerührt. Die Lösung wird im Vakuum eingedampft und der Rückstand mit Ethylacetat an Kieselgel chromatographiert.
Ausbeute: 39,4 g (62 % d. Th.)

| Elementaranalyse: | | | |
|---|---|---|---|
| ber. | C 65,24 | H 5,95 | N 9,92 |
| gef. | C 65,54 | H 5,95 | N 9,87 |

c) $N^{\alpha}$, $N^{\varepsilon}$-Bis(N,N'-dibenzyloxycarbonyl-lysyl)-lysin, geschütztes "Tri-Lysin"

3,6 g (20 mmol) Lysin-Hydrochlorid und 6,95 ml (50 mmol) Triethylamin werden in DMF gelöst, mit 26,8 g (50 mmol) $N^{\alpha}$, $N^{\varepsilon}$-Dibenzyloxycarbonyl-Lysin-p-nitrophenylester (Bachem) versetzt und 2 Tage bei Raumtemperatur gerührt. Nach Beendigung der Reaktion wird im Vakuum eingedampft, der Rückstand in Ethylacetat aufgenommen und mit verdünnter Salzsäure ausgeschüttelt Die organische Phase wird mit Natriumsulfat getrocknet, das Lösungsmittel eingedampft und der Rückstand mit Ethylacetat/Ethanol in einem Stufengradienten chromatographiert.
Ausbeute: 10,7 g (57 % d. Th.)

| Elementaranalyse: | | | |
|---|---|---|---|
| ber. | C 63,95 | H 6,65 | N 8,95 |
| gef. | C 63,63 | H 6,69 | N 8,93 |

d) Vollgeschütztes Benzyloxycarbonyl-24-Polyamin auf der Basis des N,N,N',N',N",N"-Hexakis[2-(trilysyl-amino)-ethyl]-trimesinsäuretriamids

1,27 g (1 mmol) des im Beispiel 1b beschriebenen Hexa-Benzyloxycarbonylamins werden in Eisessig gelöst und unter Rühren mit 33 prozentigem Bromwasserstoff in Eisessig versetzt. Nach 60 Minuten wird mit Diethylether die begonnene Fällung vervollständigt, das entstandene Hexa-amin-hydrobromid mit Ether gewaschen, im Vakuum getrocknet und ohne weitere Reinigung in die weiter unten beschriebene Reaktion eingesetzt.
Ausbeute: 0,95 g (quantitativ)

7,0 g (7,5 mmol) des in Beispiel 1c beschriebenen geschützten "Tri-Lysins", 1,2 g (7,5 mmol) 1-Hydroxybenzotriazol und 2,4 g (7,5 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Diese Lösung wird anschließend mit 5,16 ml (30 mmol) N-Ethyldiisopropylamin und mit 0,95 g (1 mmol) des oben beschriebenen Hexa-amin-hydrobromids versetzt und über Nacht bei Raumtemperatur gerührt. Nach Beendigung der Reaktion wird im Vakuum eingedampft und der Rückstand mit Ethylacetat/Ethanol (2:1) an Kieselgel chromatographiert.
Ausbeute: 4,55 g (76 % d. Th.)

| Elementaranalyse: | | | |
|---|---|---|---|
| ber. | C 64,35 | H 6,71 | N 10,52 |
| gef. | C 64,08 | H 6,57 | N 10,29 |

e) 24-Gadolinium-DTPA-monoamid auf der Basis des N,N,N',N',N",N"-Hexakis[2-trilysyl-amino)-ethyl]-trimesinsäuretriamids

1,20 g (0,2 mmol) des in Beispiel 1d beschriebenen Poly-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 24-Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden 5,8 g (14,4 mmol) $N^3$-(2,6-Dioxomorpholinoethyl)-$N^6$-(ethoxycarbonylmethyl)-3,6-diazaoctandisäure (Beispiel 13a der EP 0331 616) in fester Form zugegeben, wobei der pH durch weitere Zugabe von Natronlauge bei 9,5 konstant gehalten wird. Nach Beendigung der Zugabe wird zur Verseifung des DTPA-ethylesters mit 5 N Natronlauge ein pH > 13 eingestellt und über Nacht bei Raumtemperatur gerührt. Dann wird mit konz. Salzsäure auf pH 5 eingestellt, mit 2,61 g (7,2 mmol) $Gd_2O_3$ versetzt, 30 Minuten bei 80°C gerührt, nach dem Abkühlen auf pH 7 eingestellt und über eine YM3 AMICON-Ultrafiltrationsmembran entsalzt. Das Retentat wird schließlich membranfiltriert und gefriergetrocknet.
Ausbeute: 3,31 g (94,6 % d. Th.)
$H_2O$-Gehalt (Karl-Fischer): 8,5 %
Gd-Bestimmung (AAS): 22,9 %

| Elementaranalyse (bezogen auf wasserfreie Substanz): | | | | | |
|---|---|---|---|---|---|
| ber. | C 34,89 | H 4,17 | Gd 23,57 | N 10,24 | Na 3,45 |
| gef. | C 35,26 | H 4,32 | Gd 22,82 | N 10,56 | Na 3,14 |

In analoger Weise erhält man mit $Yb_2O_3$ den Ytterbium-Komplex:

| Elementaranalyse (bezogen auf wasserfreie Substanz): | | | | | |
|---|---|---|---|---|---|
| ber. | C 34,08 | H 4,08 | Yb 25,34 | N 10,00 | Na 3,37 |
| gef. | C 33,90 | H 4,22 | Yb 25,06 | N 9,83 | Na 3,13 |

**Beispiel 2**

a) 10-[5-(2-Carboxyphenyl)-2-Hydroxy-5-oxo-4-aza-pentyl]-1,4,7-tris(carboxymethyl)-1,4,7,10-tetraazacyclododecan

50 g (144,3 mmol) 1,4,7-Tris(carboxymethyl)-1,4,7,10-tetraazacyclododecan (DO3A) werden in 250 ml Wasser gelöst und der pH-Wert mit 5 N Natronlauge auf pH 13 eingestellt. Dann wird innerhalb einer Stunde eine Lösung aus 38,12 g (187,6 mmol) N(2,3-Epoxypropyl)-phthalimid in 100 ml Dioxan zugetropft, 24 Stunden bei 50°C gerührt und der pH-Wert durch Zugabe von 5 N Natronlauge bei pH 13 gehalten. Die Lösung wird mit 10%iger Salzsäure auf pH 2 gestellt und im Vakuum zur Trockne eingedampft. Der Rückstand wird in etwas Wasser gelöst und an einer Ionenaustauschersäule (Reillex®= Poly-(4-vinyl)-pyridin (man eluiert mit Wasser) gereinigt. Die Hauptfraktionen werden im Vakuum eingedampft und der Rückstand durch Chromatographie an RP-18 (LiChroPrep®/Laufmittel: Gradient aus Tetrahydrofuran/Methanol/Wasser) endgereinigt. Nach Eindampfen der Hauptfraktionen erhält man 63,57 g (71 % d. Th.) eines amorphen Feststoffes.
Wassergehalt: 8,5 %

| Analyse (bezogen auf wasserfreie Substanz): | | | |
|---|---|---|---|
| ber. | C 52,90 | H 6,57 | N 12,34 |
| gef. | C 52,65 | H 6,68 | N 12,15 |

b) 10-(3-Amino-2-hydroxy-propyl)-1,4,7-tris(carboxymethyl)-1,4,7,10-tetraazacyclo-dodecan

50 g (88,1 mmol) der Titelverbindung aus Beispiel 2a werden in 300 ml konz. Salzsäure 24 Stunden unter Rückfluß erhitzt. Man dampft zur Trockne ein, löst den Rückstand in etwas Wasser und reinigt an einer Ionenaustauschersäule (Reillex®=Poly-(4-vinyl)pyridin (man eluiert mit Wasser). Die Hauptfraktionen werden zur Trockne eingedampft. Ausbeute: 39,0 g (95 % d. Th.) eines glasigen Feststoffes
Wassergehalt: 10.3%

| Analyse (bezogen auf wasserfreie Substanz): | | | |
|---|---|---|---|
| ber. | C 48,68 | H 7,93 | N 16,70 |
| gef. | C 48,47 | H 8,09 | N 16,55 |

c) Gadolinium-Komplex des 10-(3-Amino-2-hydroxy-propyl)-1,4,7-tris(carboxymethyl)-1,4,7,10-tetraazacyclododecans

38 g (90,6 mmol) der Titelverbindung aus Beispiel 2b werden in 300 ml Wasser gelöst und 16,42 g (45,3 mmol) Gadoliniumoxid zugesetzt. Man erwärmt 3 Stunden auf 90°C Die abgekühlte Lösung wird mit je 5 ml saurem Ionenaustauscher (IR-120/$H^+$-Form) und 5 ml basischem Austauscher (IRA-410/$OH^-$-Form) eine Stunde bei Raumtemperatur gerührt. Man filtriert vom Austauscher ab. Gefriertrocknung des Filtrats liefert 57,23 g (98 % d.Th.) ,eines amorphen Feststoffes.
Wassergehalt: 11,3 %

| Analyse (bezogen auf wasserfreie Substanz): | | | | |
|---|---|---|---|---|
| ber. | C 35,59 | H 5,27 | Gd 27,41 | N 12,21 |
| gef. | C 35,32 | H 5,38 | Gd 27,20 | N 12,31 |

d) Gadolinium-Komplex des 10-[7-(4-Nitrophenyl)-2-hydroxy-5-oxo-7-(carboxymethyl)-4-aza-heptyl]-1,4,7-tris(carboxymethyl)-1,4,7,10-tetraazacyclododecans

Zu 20 g (34,86 mmol) der Titelverbindung aus Beispiel 2c in 200 ml Dimethylformamid/20 ml Triethylamin gibt man 9,84 g (41,8 mmol) 3-(4-Nitrophenyl)-glutarsäureanhydrid (Journal of Org. Chem., Vol. 26, 3856 (1961)) und rührt über Nacht bei Raumtemperatur. Man dampft im Vakuum zur Trockne ein. Der Rückstand wird aus Isopropanol/Essigsäure 95:5 umkristallisiert.
Ausbeute: 27,46 g (94 % d. Th.) eines gelblichen Feststoffes
Wassergehalt: 3,4 %

| Analyse (bezogen auf wasserfreie Substanz): | | | | |
|---|---|---|---|---|
| ber. | C 41,58 | H 4,86 | Gd 19,44 | N 10,39 |
| gef. | C 41,38 | H 4,97 | Gd 19,28 | N 10,17 |

e) Gadolinium-Komplex des 10-[7-(4-Aminophenyl)-2-hydroxy-5-oxo-7-(carboxymethyl)-4-aza-heptyl]-1,4,7-tris(carboxymethyl)-1,4,7,10-tetraazacyclododecans

25 g (30,9 mmol) der Titelverbindung aus Beispiel 2d werden in 250 ml Methanol gelöst und 5 g Palladium-Katalysator (10 % Pd auf C) zugegeben. Man hydriert über Nacht bei Raumtemperatur. Der Katalysator wird abfiltriert und das Filtrat im Vakuum zur Trockne eingedampft.
Ausbeute: 24,07 g (97 % d. Th.) eines cremefarbenen Feststoffes
Wassergehalt: 3,0 %

| Analyse (bezogen auf wasserfreie Substanz): | | | | |
|---|---|---|---|---|
| ber. | C 43,18 | H 5,31 | Gd 20,19 | N 10,79 |
| gef. | C 43,27 | H 5,48 | Gd 20,02 | N 10,61 |

f) Gadolinium-Komplex des 10-[7-(4-Isothiocyanatophenyl)-2-hydroxy-5-oxo-7-(carboxymethyl)-4-aza-heptyl]-1,4,7-tris(carboxymethyl)-1,4,7,10-tetraazacyclododecans

15 g (19,26 mmol) der Titelverbindung aus Beispiel 2e werden in 100 ml Wasser gelöst und 6,64 g (57,8 mmol) Thiophosgen in 50 ml Chloroform zugegeben. Man rührt 1 Stunde bei 50°C. Man kühlt auf Raumtemperatur, trennt die organische Phase ab und schüttelt die wässrige Phase 2 mal mit 100 ml Chloroform aus. Die wässrige Phase wird zur Trockne eingedampft und der Rückstand in 100 ml Isopropanol bei Raumtemperatur ausgerührt. Der Feststoff wird abfiltriert und mit Ether gewaschen. Nach Trocknen über Nacht im Vakuum (40°C) erhält man 15,9 g (98 % d. Th.) eines cremefarbenen Feststoffes.
Wassergehalt: 3,5 %

| Analyse (bezogen auf wasserfreie Substanz): | | | | | |
|---|---|---|---|---|---|
| ber. | C 42,43 | H 4,79 | Gd 19,15 | N 10,24 | S 3,91 |
| gef. | C 42,23 | H 4,90 | Gd 19,01 | N 10,05 | S 3,96 |

g) 24-Thio-Harnstoff-Konjugat aus dem Gd-Komplex des 10-[7-(4-Isothiocyanatophenyl)-2-hydroxy-5-oxo-7-(carboxymethyl)-4-aza-heptyl]-1,4,7-tris(carboxymethyl)-1,4,7,10-tetraazacyclododecans mit dem 24-Amin N,N,N',N',N",N"-Hexakis[2-(trilysyl-amino)-ethyl]-trimesinsäuretriamid

1,20 g (0,2 mmol) des in Beispiel 1d beschriebenen 24-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 24-Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden portionsweise 6,08 g (7,2 mmol) des im vorstehenden Beispiel 2f beschriebenen Isothiocyanats in fester Form zugegeben, wobei der pH durch weitere Zugabe von Natronlauge bei 9,5 konstant gehalten wird. Nach Beendigung der Zugabe wird über Nacht bei Raumtemperatur gerührt und anschließend mit verd. Salzsäure neutralisiert. Die Lösung wird ultrafiltriert (Amicon YM3) und das Retentat gefriergetrocknet.

Ausbeute: 4,16 g (83 % d. Th.)
$H_2O$-Gehalt (Karl-Fischer): 8,2 %
Gd-Bestimmung (AAS): 14,7 %

| Elementaranalyse (berechnet auf wasserfreie Substanz): | | | | | | |
|---|---|---|---|---|---|---|
| ber. | C 43,08 | H 5,11 | Gd 16,41 | N 11.51 | Na 2,40 | S 3,35 |
| gef. | C 43,47 | H 5,32 | Gd 16,19 | N 11,23 | Na 2,04 | S 3,07 |

In analoger Weise erhält man mit N-Methyl-D-glucamin anstelle von Natronlauge das N-Methyl-D-glucaminsalz:

| | | | | | |
|---|---|---|---|---|---|
| ber. | C 43,91 | H 5,93 | Gd 13,89 | N 10,98 | S 2,83 |
| gef. | C 43,73 | H 6,19 | Gd 13,56 | N 11,22 | S 2,60 |

**Beispiel 3**

a) Gadolinium-Komplex des 10-(8-carboxy-2-hydroxy-5-oxo-4-aza-7-oxa-octyl)-1,4,7-tris(carboxymethyl)-1,4,7,10-tetraazacyclododecans

10 g (17,43 mmol) der Titelverbindung aus Beispiel 2c werden in 100 ml Wasser gelöst und 20 ml Triethylamin zugegeben. Bei 0°C setzt man 5,80 g (50 mmol) Diglycolsäureanhydrid zu und rührt 3 Stunden bei dieser Temperatur. Es wird im Vakuum zur Trockne eingedampft und der Rückstand an Kieselgel chromatographiert (Laufmittel: Methanol/Ammoniak (25 % aqu.) 20:1). Die produkthaltigen Fraktionen werden zur Trockne eingedampft, mit 100 ml Wasser aufgenommen, und der pH-Wert der Lösung durch Zugabe von saurem Ionenaustauscher (IR 120 $H^+$) auf pH 2,46 gestellt. Man filtriert vom Austauscher ab, spült zweimal mit wenig Wasser nach und dampft zur Trockne ein. Der Rückstand wird aus Methanol/Aceton umkristallisiert. Nach Trocknen im Vakuum (125°C/über Nacht) erhält man 7,57 g (63 % d. Th.) eines farblosen kristallinen Feststoffs.
Wassergehalt: 3,1 %

| Elementaranalyse (berechnet auf wasserfreie Substanz): | | | | |
|---|---|---|---|---|
| ber. | C 36,57 | H 4,97 | Gd 22,80 | N 10,15 |
| gef. | C 36,43 | H 5,06 | Gd 22,70 | N 10,01 |

b) 24-amid-Konjugat aus dem Gd-Komplex des 10-[8-carboxy-2-hydroxy-5-oxo-4-aza-7-oxaoctyl]-1,4,7-tris(carboxymethyl)-1,4,7,10-tetraazacyclododecans mit dem 24-Amin N,N,N',N',N",N"-Hexakis[2-(trilysyl-amino)-ethyl]-trimesinsäuretriamid

1,20 g (0,2 mmol) des in Beispiel 1d beschriebenen 24-Benzyloxycarbonylamins werden in Eisessig gelöst und unter Rühren mit 33 prozentigem Bromwassetstoff in Eisessig versetzt. Nach 3 Stunden wird mit Diethylether die begonnene Fällung vervollständigt, das entstandene 24-Amin-hydrobromid mit Diethylether gewaschen, im Vakuum getrocknet und ohne weitere Reinigung in die weiter unten beschriebene Reaktion eingesetzt.
Ausbeute: 0,95 g (quantitativ)

4,14 g (6 mmol) der in Beispiel 3a beschriebenen Komplexsäure, 0,96 g (6 mmol) 1-Hydroxybenzotriazol (HOBt) und 1,92 g (6 mmol) 2-(1H-Benzotriazol-1-yl)-1,1,3,3-tetramethyl-uronium tetrafluorborat (TBTU; Peboc Limited, UK) werden in DMF gelöst und 15 Minuten gerührt. Diese Lösung wird anschließend mit 4,13 ml (24 mmol) N-Ethyldiiso propylamin und mit 0,95 g (0,2 mmol) des oben beschriebenen 24-Amin-hydrobromids versetzt und über Nacht bei Raumtemperatur gerührt. Nach Beendigung der Reaktion werden 0,23 g (2 mmol) Diglykolsäureanhydrid zugegeben und noch eine Stunde gerührt. Dann wird im Vakuum eingedampft, der Rückstand in Wasser aufgenommen und über eine AMICON YM10-Ultrafiltrationsmembran gereinigt. Das Retentat wird, falls notwendig, auf pH 7 eingestellt, membranfiltriert und schließlich gefriergetrocknet.
Ausbeute: 3,62 g (88 % d. Th.)
$H_2O$-Gehalt (Karl Fischer): 8,1 %
Gd-Bestimmung (AAS): 18,7 %

| Elementaranalyse (bezogen auf wasserfreie Substanz): | | | | |
|---|---|---|---|---|
| ber. | C 40,24 | H 5,46 | Gd 19,97 | N 12,23 |
| gef. | C 40,07 | H 5,63 | Gd 20,20 | N 12,17 |

**Beispiel 4**

a) N,N,N-Tris[2-($N^{\alpha}$,$N^{\varepsilon}$-dibenzyloxycarbonyl-lysylamino)-ethyl]amin

1,46 g (10 mmol) Tris(2-aminoethyl)amin und 21,4 g (40 mmol) $N^{\alpha}$,$N^{\varepsilon}$-Dibenzyloxy-carbonyl-Lysin-p-nitrophenylester werden in DMF gelöst und über Nacht bei Raumtemperatur gerührt. Nach Beendigung der Reaktion wird im Vakuum eingedampft, der Rückstand mit Diethylether verrührt, der Niederschlag abgesaugt und aus Ethylacetat umkristallisiert.
Ausbeute: 12,55 g (94 % d. Th.)

| Elementatanalyse: | | | |
|---|---|---|---|
| ber. | C 64,75 | H 6,79 | N 10,49 |
| gef. | C 64,48 | H 6,88 | N 10,26 |

b) Vollgeschütztes Benzyloxycarbonyl-24-Polyamin auf der Basis des N,N,N-Tris[2-($N^{\alpha}$, $N^{\varepsilon}$-bis{trilysyl}-lysylamino)-ethyl]amins

1,33 g (1 mmol) des in Beispiel 4a beschriebenen Hexa-Benzyloxycarbonylamins werden in Eisessig gelöst und unter Rühren mit 33 prozentigem Bromwasserstoff in Eisessig versetzt. Nach 60 Minuten wird mit Diethylether die begonnene Fällung vervollständigt, das entstandene Hexamin-hydrobromid mit Ether gewaschen, im Vakuum getrocknet und ohne weitere Reinigung in die weiter unten beschriebene Reaktion eingesetzt.
Ausbeute: 1,02 g (quantitativ)

7,0 g (7,5 mmol) des in Beispiel 1c beschriebenen $N^{\alpha}$,$N^{\varepsilon}$-Bis(N,N'-dibenzyloxycarbonyllysyl)-lysins (geschütztes "Tri-Lysin") werden analog zu Beispiel 1d mit TBTU und N-Hydroxybenzotriazol aktiviert und in analoger Weise mit 1,02 g (1 mmol) des oben beschriebenen N,N,N-Tris[2-(lysylamino)ethyl]-amin-hexahydrobromids und 5,16 ml (30 mmol) N-Ethyldlisopropylamin umgesetzt und in gleicher Weise, wie dort beschrieben, aufgearbeitet.
Ausbeute: 4,42 g (73 % d. Th.)

| Elementaranalyse: | | | |
|---|---|---|---|
| ber. | C 64,25 | H 6,89 | N 10,64 |
| gef. | C 64,06 | H 7,04 | N 10,69 |

c) 24-Gadolinium-DTPA-monoamid auf der Basis des 24-Amins N,N,N-Tris[2-($N^{\alpha}$,$N^{\varepsilon}$-bis{trilysyl-lysylamino)-ethyl]amin

1,21 g (0,2 mmol) des in Beispiel 4b beschriebenen 24-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 24-Amin-hydrobromid mit Diethylether gewaschen und im Vakuum getrocknet. Der Rückstand wird analog der in Beispiel 1e gegebenen Vorschrift mit 5,8 g (14,4 mmol) $N^3$-(2,6-Dioxomorpholinoethyl)-$N^6$-(ethoxycarbonylmethyl)-3,6-diazaoctandisäure acyliert und mit 2,61 g (7,2 mmol) $Gd_2O_3$ komplexiert.
Man erhält 3,30 g (92 % d. Th.) farbloses Lyophilisat
$H_2O$-Gehalt (Karl-Fischer): 10,3 %
Gd-Bestimmung (AAS) : 20,7 %

| Elementaranalyse (bezogen auf wasserfreie Substanz): | | | | | |
|---|---|---|---|---|---|
| ber. | C 34,97 | H 4,25 | Gd 23,48 | N 10,28 | Na 3,43 |
| gef. | C 34,69 | H 4,31 | Gd 23,20 | N 10,37 | Na 3,05 |

In analoger Weise erhält man mit $Dy_2O_3$ den Dysprosium-Komplex:

| Elementaranalyse (bezogen auf wasserfreie Substanz): | | | | | |
|---|---|---|---|---|---|
| ber. | C 34,70 | H 4,22 | Dy 24,07 | N 10,20 | Na 3,41 |
| gef. | C 34,50 | H 4,42 | Dy 23,81 | N 10,06 | Na 3,02 |

**Beispiel 5**

a) 24-Thio-Harnstoff-Konjugat aus dem Gd-Komplex des 10-[7-(4-Isothiocyanatophenyl)-2-hydroxy-5-oxo-7-(carboxymethyl)-4-aza-heptyl]-1,4,7-tris(carboxymethyl)-1,4,7,10-tetraazacyclododecans mit dem aus N,N,N-Tris[2-(lysylamino)ethyl]amin mit $N^{\alpha}$, $N^{\varepsilon}$-bis(lysyl) Lysin ("Tri-Lysin") kondensierten 24-Amin

1,21 g (0,2 mmol) des in Beispiel 1d beschriebenen 24-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 24-Amin-hydrobromid mit Diethylether gewaschen und im Vakuum getrocknet.
Der Rückstand wird anschließend in Wasser aufgenommen und analog der in Beispiel 2g gegebenen Vorschrift bei pH 9,5 mit 6,08 g (7,2 mmol) des Gadolinium-Komplexes des 10-[7-(4-Isothiocyanatophenyl)-2-hydroxy-5-oxo-7-(carboxymethyl)-4-azaheptyl]-1,4,7-tris(carboxymethyl)-1,4,7,10-tetraazacyclododecans umgesetzt und nach Ultrafiltration gefriergetrocknet.
Ausbeute: 4,31 g (87 % d. Th.)
$H_2O$-Gehalt (Karl-Fischer): 6,9 %
Gd-Bestimmung (AAS): 15,0 %

| Elementaranalyse (bezogen auf wasserfreie Substanz): | | | | | | |
|---|---|---|---|---|---|---|
| ber. | C 43,11 | H 5,16 | Gd 16,36 | N 11,54 | Na 2,39 | S 3,34 |
| gef. | C 42,89 | H 5,35 | Gd 16,09 | N 11,67 | Na 2,18 | S 3,23 |

**Beispiel 6**

a) 24-Amidkonjugat aus dem Gd-Komplex des 10-[8-carboxy-2-hydroxy-5-oxo-4-aza-7-oxaoctyl]-1,4,7-tris(carboxymethyl)-1,4,7,10-tetraazacyclododecans mit dem aus N,N,N-Tris[2-(lysylamino)ethyl]amin mit $N^{\alpha}$,$N^{\varepsilon}$-bis(lysyl)Lysin ("Tri-Lysin") kondensierten 24 Amin

1,21 g (0,2 mmol) des in Beispiel 4b beschriebenen 24-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 24-Amin-hydrobromid mit Diethylether gewaschen, im Vakuum getrocknet und ohne weitere Reinigung in die weiter unten beschriebene Reaktion eingesetzt.
Ausbeute: 0,96 g (quantitativ)
4,14 g (6 mmol) der in Beispiel 3a beschriebenen Gadoliniumkomplexsäure werden analog der für Beispiel 3b gegebenen Vorschrift mit TBTU und HOBt in DMF aktiviert und mit dem oben beschriebenen 24-Amin-hydrobromid unter Basenzusatz gekuppelt und in analoger Weise aufgearbeitet.
Ausbeute: 3,83 g (92 % d. Th.)
$H_2O$-Gehalt (Karl-Fischer): 8,8 %
Gd-Bestimmung (AAS): 18,5 %

| Elementaranalyse (bezogen auf wasserfreie Substanz): | | | | |
|---|---|---|---|---|
| ber. | C 40,29 | H 5,52 | Gd 19,90 | N 12,26 |
| gef. | C 39,97 | H 5,71 | Gd 19,55 | N 12,21 |

**Beispiel 7**

a) N,N'-Bis(benzyloxycarbonyl)-3-[carboxymethoxyacetyl]-3-azapentan-1,5-diamin

37,14 g (100 mmol) des in Beispiel 1a beschriebenen Bis(benzyloxycaibonyl-aminoethyl) amins werden in DMF gelöst, im Eisbad mit 17,4 g (150 mmol) Diglykolsäureanhydrid (Janssen Chimica) und 21 ml (150 mmol) Triethylamin versetzt und anschließend über Nacht bei Raumtemperatur gerührt. Die Lösung wird im Vakuum eingedampft, der Rückstand in Ethylacetat aufgenommen und mit verdünnter Salzsäure ausgeschüttelt. Die organische Phase wird mit Natriumsulfat getrocknet und nach Filtration vom Trocknungsmittel durch Zugabe von Hexan kristallisiert.
Ausbeute: 41,4 g (85 % d. Th.)

| Elementaranalyse: | | | |
|---|---|---|---|
| ber. | C 59,13 | H 6,00 | N 8,62 |
| gef. | C 58,99 | H 5,93 | N 8,70 |

b) N,N',N",N"'-Tetrakis{8-(Benzyloxycarbonylamino)-6-[2-(benzyloxycarbonylamino-ethyl]-5-oxo-3-oxaoctanoyl}cyclen

345 mg (2 mmol) 1,4,7,10-Tetraazacyclododecan (Cyclen; Fluka) werden mit Toluol azeotrop entwässert. Zu der abgekühlten Lösung von Cyclen in Toluol wird bei Raumtemperatur eine Lösung von 4,88 g (10 mmol) N,N'-Bis (benzyloxycarbonyl)-3-[carboxymethoxyacetyl]-3-azapentan-1,5-diamin (Beispiel 7a) in Tetrahydrofuran (THF) sowie 2,47 g (10 mmol) 2-Ethoxy-1-ethoxycarbonyl-1,2-dihydrochinolin (EEDQ; Fluka) zugegeben und über Nacht gerührt. Nach Beendigung der Reaktion wird das Produkt durch Zugabe von Hexan ausgefällt, vom Lösungsmittel abdekantiert und noch einmal aus THF/Hexan und anschließend aus THF/Toluol umgefällt. Man erhält nach Trocknung im Vakuum 2,78 g (68 % d. Th.) eines blaßgelben Feststoffs.

| Elementaranalyse: | | | |
|---|---|---|---|
| ber. | C 60,93 | H 6,29 | N 10,93 |
| gef. | C 60,68 | H 6,40 | N 10,97 |

c) Vollgeschütztes Benzyloxycarbonyl-32-Polyamin auf der Basis des aus N,N',N",N"'-Tetrakis{8-Benzyloxycarbonylamino)-6-[2-(benzyloxycarbonylamino)-ethyl]-5-oxo-3-oxaoctanoyl}cyclen mit $N^{\alpha}$,$N^{\varepsilon}$-bis(lysyl)-Lysin("Tri-Lysin") kondensierten 32-Amins

2,05 g (1 mmol) des im Beispiel 7b beschriebenen Okta-Benzyloxycarbonylamins werden in Eisessig gelöst und unter Rühren mit 33 prozentigem Bromwasserstoff in Eisessig versetzt. Nach 90 Minuten wird mit Diethylether die begonnende Fällung vervollständigt, das entstandene Okta-amin-hydrobromid mit Ether gewaschen, im Vakuum getrocknet und ohne weitere Reinigung in die weiter unten beschriebene Reaktion eingesetzt.
Ausbeute: 1,6 g (quantitativ)

9,4 g (10 mmol) des in Beispiel 1c beschriebenen geschützten "Tri-Lysins", 1,5 g (10 mmol) 1-Hydroxybenzotriazol und 3,2 g (10 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Diese Lösung wird anschließend mit 5,16 ml (30 mmol) N-Ethyldiisopropylamin und mit 1,6 g (1 mmol) des oben beschriebenen Okta-amin-hydrobromids versetzt und über Nacht bei Raumtemperatur gerührt. Nach Beendigung der Reaktion wird im Vakuum eingedampft und der Rückstand mit Dichlormethan/Methanol (10:1) an Kieselgel chromatographiert.
Ausbeute: 6,0 g (72 % d. Th.)

| Elementaranalyse: | | | |
|---|---|---|---|
| ber. | C 63,32 | H 6,76 | N 10,74 |
| gef. | C 62,98 | H 6,91 | N 10,43 |

d) 32-Gadolinium-DTPA-monoamid auf der Basis des deblockierten 32-Amins aus Beispiel 7c

1,67 g (0,2 mmol) des in Beispiel 7c beschriebenen Poly-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 32-Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden 7,7 g (19,2 mmol) $N^3$-(2,6-Dioxomorpholinoethyl)-$N^6$-(ethoxycarbonylmethyl)-3,6-diazaoctandisäure (Beispiel 13a der EP 0331 616) in fester Form zugegeben, wobei der pH durch weitere Zugabe von Natronlauge bei 9,5 konstant gehalten wird. Nach Beendigung der Zugabe wird zur Verseifung des DTPA-ethylesters

mit 5 N Natronlauge ein pH > 13 eingestellt und über Nacht bei Raumtemperatur gerührt. Dann wird mit konz. Salzsäure auf pH 5 eingestellt, mit 3,48 g (9,6 mmol) $Gd_2O_3$ versetzt, 30 Minuten bei 80°C gerührt, nach dem Abkühlen auf pH 7 eingestellt und über eine YM3 AMICON-Ultrafiltrationsmembran entsalzt. Das Retentat wird schließlich membranfiltriert und gefriergetrocknet.
Ausbeute: 4,27 g (91 % d. Th.)
$H_2O$-Gehalt (Karl-Fischer): 7,5 %
Gd-Bestimmung (AAS): 22,6 %

| Elementaranalyse (berechnet auf wasserfreie Substanz): | | | | | |
|---|---|---|---|---|---|
| ber. | C 34,98 | H 4,24 | Gd 23,19 | N 10,33 | Na 3,39 |
| gef. | C 34,67 | H 4,15 | Gd 22,86 | N 10,25 | Na 3,14 |

In analoger Weise erhält man mit $Eu_2O_3$ den Europium-Komplex:

| Elementaranalyse (bezogen auf wasserfreie Substanz): | | | | | |
|---|---|---|---|---|---|
| ber. | C 35,25 | H 4,27 | Eu 22,58 | N 10,41 | Na 3,42 |
| gef. | C 35,08 | H 4,17 | Eu 22,16 | N 10,53 | Na 3,22 |

**Beispiel 8**

a) 32-Thio-Harnstoff-Konjugat aus dem Gd-Komplex des 10-[7-(4-Isothiocyanatophenyl)-2-hydroxy-5-oxo-7-(carboxymethyl)-4-aza-heptyl]-1,4,7-tris(carboxymethyl)-1,4,7,10-tetraazacyclododecans mit dem deblockierten 32-Amin aus Beispiel 7c

1,67 g (0,2 mmol) des in Beispiel 7c beschriebenen 32-Benzyloxycarbonylamins werden in Eisessig gelöst und unter Rühren mit 33 prozentigem Bromwasserstoff in Eisessig versetzt. Nach 3 Stunden wird mit Ether die begonnene Fällung vervollständigt, das entstandene 32-Amin-hydrobromid mit Diethylether gewaschen und im Vakuum getrocknet.
Der Rückstand wird anschließend in Wasser aufgenommen und durch Zugabe von 1 N Natronlauge auf pH 9,5 eingestellt. In diese Lösung werden portionsweise 8,1 g (9,6 mmol) des im Beispiel 2f beschriebenen Isothiocyanats in fester Form zugegeben,
wobei der pH durch weitere Zugabe von Natronlauge bei 9,5 konstant gehalten wird. Nach Beendigung der Zugabe wird über Nacht bei Raumtemperatur gerührt und anschließend mit verd. Salzsäure neutralisiert. Die Lösung wird ultrafiltriert (Amicon YM3) und das Retentat gefriergetrocknet.
Ausbeute: 5,8 g (85 % d. Th.)
$H_2O$-Gehalt (Karl-Fischer): 9,1 %
Gd-Bestimmung (AAS): 15,2 %

| Elementaranalyse (berechnet auf wasserfreie Substanz): | | | | | | |
|---|---|---|---|---|---|---|
| ber. | C 43,05 | H 5,15 | Gd 16,22 | N 11,56 | Na 2,37 | S 3,31 |
| gef. | C 42,84 | H 5,36 | Gd 16,00 | N 11,84 | Na 2,10 | S 2,97 |

**Beispiel 9**

a) 1,11-Bis(benzyloxycarbonylamino)-3,9-bis[2-(benzyloxycarbonylamino)ethyl]-4,8-dioxo-6-(4-nitrophenyl)-3,9-diazaundecan

6,33 g (25 mmol) 3-(4-Nitrophenyl)-glutarsäure (J. Org. Chem., 26, 3856 (1961)), 6,33 g (55 mmol) N-Hydroxysuccinimid und 20,43 g (55 mmol) des in Beispiel 1a beschriebenen Bis(benzyloxycarbonyl-aminoethyl)amins werden in DMF gelöst und nach Zugabe von 11,35 g (55 mmol) Dicyclohexylcarbodiimid 3 Tage bei Raumtemperatur gerührt. Dann wird vom ausgefallenen Dicyclohexylharnstoff abfiltriert, das Filtrat im Vakuum zur Trockene eingedampft und an Kieselgel mit Ethylacetat als Laufmittel chromatographisch gereinigt.
Ausbeute: 17,3 g (72 % d. Th.)

| Elementaranalyse: | | | |
|---|---|---|---|
| ber. | C 63,81 | H 5,98 | N 10,21 |
| gef. | C 63,94 | H 5,77 | N 10,26 |

b) 1,11-Bis(benzyloxycarbonylamino)-3,9-bis-[2-(benzyloxycarbonylamino)ethyl]-4,8-dioxo-6-(4-aminophenyl)-3,9-diazaundecan

15,57 g (56 mmol) $FeSO_4$ x 7 $H_2O$ werden in Wasser gelöst und mit 7,68 g (8 mmol) der in Beispiel 9a beschriebenen Nitroverbindung im gleichen Volumen Ethanol vereinigt und zum Sieden erhitzt. Bei dieser Temperatur werden langsam 24 ml konz. Ammoniak zugetropft,
wobei sich ein schwarzer Niederschlag bildet. Man läßt die Suspension unter Rühren langsam abkühlen, dann wird filtriert, der Niederschlag mit Ethylacetat nachgewaschen und die vereinigten Filtrate im Vakuum zur Trockene eingedampft. Der Rückstand wird mit Ethylacetat/Ethanol (98:2) an Kieselgel chromatographiert.
Ausbeute: 4,84 g (65 % d. Th.)

| Elementaraanalyse: | | | |
|---|---|---|---|
| ber. | C 65,86 | H 6,39 | N 10,54 |
| gef. | C 65,68 | H 6,31 | N 10,62 |

c) N,N',N'',N'''-Cyclen-Tetra-Ureido-Konjugat aus 1,4,7,10-Tetraazacyclododecan mit 1,11-Bis(benzyloxycarbonylamino)-3,9-bis[2-(benzyloxycarbonylamino)ethyl]-4,8-dioxo-6-(4-Isocyanatophenyl)-3,9-diazaundecan

4,65 g (5 mmol) des in Beispiel 9b beschriebenen Phenylamins werden in Toluol gelöst, zur Entwässerung zweimal zur Trockene eingedampft und mit jeweils frischem Toluol wieder aufgenommen. Die auf diese Weise getrocknete Lösung des Amins in Toluol wird bei 10°C mit 0,54 g (1,8 mmol) Triphosgen versetzt, eine Stunde bei 10°C und über Nacht bei Raumtemperatur gerührt. Zu dieser Suspension werden 0,17 g (1 mmol) wasserfreies 1,4,7,10-Tetraazacyclododecan (Cyclen) (Aldrich) in Toluol/Pyridin (10:1) zugegeben und über Nacht bei Raumtemperatur gerührt. Dann wird im Vakuum zur Trockene eingedampft und der Rückstand in Ethylacetat an Kieselgel chromatographiert.
Ausbeute: 3,55 g (89 % d. Th.)

| Elementaranalyse: | | | |
|---|---|---|---|
| ber. | C 64,98 | H 6,16 | N 11,23 |
| gef. | C 64,70 | H 6,29 | N 11,04 |

d) N,N-Bis[2-(benzyloxycarbonylamino)-ethyl]-glycin-tert.-butylester

18,6 g (50 mmol) des in Beispiel 1a beschriebenen Bis[2-(benzyloxycarbonylamino)-ethyl]amins in Tetrahydrofuran/Wasser (25:1) werden mit 4,2 g (30 mmol) Kaliumcarbonat versetzt. Bei Raumtemperatur werden zu dieser Suspension 11,7 g (60 mmol) tert.-Butylbromacetat zugetropft und anschließend bei dieser Temperatur über Nacht gerührt. Es wird vom Ungelösten abfiltriert, das Filtrat zur Trockne eingedampft und an Kieselgel chromatographiert (Diethylether/Diisopropylether 1:1).
Ausbeute: 20,4 g (84 % d. Th.)

| Elementaranalyse: | | | |
|---|---|---|---|
| ber. | C 64,31 | H 7,26 | N 8,65 |
| gef. | C 64,35 | H 7,00 | N 8,58 |

e) N,N-Bis[2-(benzyloxycarbonylamino)-ethyl]-glycin

19,4 g (40 mmol) des in Beispiel 9d beschriebenen t-Butylesters werden mit 150 ml Trifluoressigsäure versetzt und 20 Minuten bei Raumtemperatur gerührt. Anschließend wird das Produkt durch Zugabe von Diethylether ausgefällt und noch je einmal mit Ether und schließlich mit Wasser ausgerührt. Der Niederschlag wird abfiltriert und getrocknet.
Ausbeute: 13,4 g (78 % d. Th.)

| Elementaranalyse: | | | |
|---|---|---|---|
| ber. | C 61,52 | H 6,34 | N 9,79 |
| gef. | C 61,64 | H 6,20 | N 9,94 |

f) Vollgeschütztes Benzyloxycarbonyl-32-Polyamin aus dem mit N,N-Bis[2-(benzyloxycarbonylamino)-ethyl]-glycin kondensierten N,N',N",N"'-Cyclen-Tetra-Ureido-Konjugat aus Beispiel 9c

3,99 g (1 mmol) des im Beispiel 9c beschriebenen Hexadeca-Benzyloxycarbonylamins werden in Eisessig gelöst und unter Rühren mit 33 prozentigem Bromwasserstoff in Eisessig versetzt. Nach 90 Minuten wird mit Diethylether die begonnene Fällung vervollständigt, das entstandene Hexadeca-amin-hydrobromid mit Ether gewaschen, im Vakuum getrocknet und ohne weitere Reinigung in die weiter unten beschriebene Reaktion eingesetzt.
Ausbeute: 3,2 g (quantitativ)

8,6 g (20 mmol) des in Beispiel 9e beschriebenen N,N-Bis[2-(benzyloxycarbonylamino)-ethyl]-glycins, 3,2 g (20 mmol) 1-Hydroxybenzotriazol und 6,4 g (20 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Diese Lösung wird anschließend mit 13,76 ml (80 mmol) N-Ethyldiisopropylamin und mit 3,2 g (1 mmol) des oben beschriebenen Hexadeca-amin-hydrobromids versetzt und über Nacht bei Raumtemperatur gerührt. Nach Beendigung der Reaktion wird im Vakuum eingedampft und der Rückstand mit Dichlormethan/Methanol (9:1) an Kieselgel chromatographiert.
Ausbeute: 7,3 g (87 % d. Th.)

| Elementaranalyse: | | | |
|---|---|---|---|
| ber. | C 62,69 | H 6,55 | N 13,29 |
| gef. | C 62,37 | H 6,72 | N 13,38 |

g) 32-Gadolinium-DTPA-monoamid auf der Basis des deblockierten 32-Amins aus Beispiel 9f

1,69 g (0,2 mmol) des in Beispiel 9f beschriebenen Poly-Benzyloxycarbonylamins werden in Eisessig gelöst unter Rühren mit 33 prozentigem Bromwasserstoff in Eisessig versetzt. Nach 3 Stunden wird mit Diethylether die begonnene Fällung vervollständigt, das entstandene 32-Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden 7,3 g (19,2 mmol) $N^3$-(2,6-Dioxomorpholinoethyl)-$N^6$-(ethoxycarbonylmethyl)-3,6-diazaoctandisäure (Beispiel 13a der EP 0331 616) in fester Form zugegeben, wobei der pH durch weitere Zugabe von Natronlauge bei 9,5 konstant gehalten wird. Nach Beendigung der Zugabe wird zur Verseifung des DTPA-ethylesters mit 5 N Natronlauge ein pH > 13 eingestellt und über Nacht bei Raumtemperatur gerührt. Dann wird mit konz. Salzsäure auf pH 5 eingestellt, mit 3,48 g (9,6 mmol) $Gd_2O_3$ versetzt, 30 Minuten bei 80°C gerührt, nach dem Abkühlen auf pH 7 eingestellt und über eine YM3 AMICON-Ultrafiltrationsmembran entsalzt. Das Retentat wird schließlich membranfiltriert und gefriergetrocknet.
Ausbeute: 4,29 g (91 % d. Th.)
$H_2O$-Gehalt (Karl-Fischer): 9,1 %
Gd-Bestimmung (AAS): 22,7 %

| Elementaranalyse (berechnet auf wasserfreie Substanz): | | | | | |
|---|---|---|---|---|---|
| ber. | C 35,41 | H 4,31 | Gd 23,48 | N 11,50 | Na 1,72 |
| gef. | C 35,39 | H 4,20 | Gd 23,22 | N 11,69 | Na 1,59 |

**Beispiel 10**

a) 32-Thio-Harnstoff-Konjugat aus dem Gd-Komplex des 10-[7-(4-Isothiocyanatophenyl)-2-hydroxy-5-oxo-7-(carboxymethyl)-4-aza-heptyl]-1,4,7-tris(carboxymethyl)-1,4,7,10-tetraazacyclododecans mit dem deblockierten 32-Amin aus Beispiel 9f

1,69 g (0,2 mmol) des in Beispiel 9f beschriebenen 32-Benzyloxycarbonylamins werden in Eisessig gelöst und unter Rühren mit 33 prozentigem Bromwasserstoff in Eisessig versetzt. Nach 3 Stunden wird mit Ether die begonnene Fällung vervollständigt, das entstandene 32-Amin-hydrobromid mit Diethylether gewaschen und im Vakuum getrocknet.
Der Rückstand wird anschließend in Wasser aufgenommen und durch Zugabe von 1 N Natronlauge auf pH 9,5 eingestellt. In diese Lösung werden portionsweise 8,1 g (9,6 mmol) des im Beispiel 2f beschriebenen Isothiocyanats in fester Form zugegeben,
wobei der pH durch weitere Zugabe von Natronlauge bei 9,5 konstant gehalten wird. Nach Beendigung der Zugabe wird über Nacht bei Raumtemperatur gerührt und anschließend mit verd. Salzsäure neutralisiert. Die Lösung wird ultrafiltriert (Amicon YM3) und das Retentat gefriergetrocknet.
Ausbeute: 5,7 g (85 % d. Th.)
$H_2O$-Gehalt (Karl-Fischer): 8,7 %
Gd-Bestimmung (AAS): 15,3 %

| Elementaranalyse (berechnet auf wasserfreie Substanz): | | | | | | |
|---|---|---|---|---|---|---|
| ber. | C 43,47 | H 5,10 | Gd 16,38 | N 12,40 | Na 1,20 | S 3,34 |
| gef. | C 43,25 | H 5,27 | Gd 16,11 | N 12,46 | Na 1,05 | S 3,62 |

**Beispiel 11**

a) N,N',N'',N'''-Tetrakis{N,N-Bis[2-(benzyloxycarbonylamino)-ethyl]-glycyl}-cyclen

345 mg (2 mmol) 1,4,7,10-Tetraazacyclododecan (Cyclen; Fluka) werden mit Toluol azeotrop entwässert. Zu der abgekühlten Lösung von Cyclen in Toluol wird bei Raumtemperatur eine Lösung von 4,29 g (10 mmol) des in Beispiel 9e beschriebenen N,N-Bis[2-(benzyloxycarbonylamino)-ethyl]-glycins in Tetrahydrofuran (THF) sowie 2,47 g (10 mmol) 2-Ethoxy-1-ethoxycarbonyl-1,2-dihydrochinolin (EEDQ; Fluka) zugegeben und über Nacht gerührt. Die Lösung wird im Vakuum zur Trockne eingedampft und in Ethylacetat/Methanol (10:1) an Kieselgel chromatographiert.
Ausbeute: 2,69 g (74 % d.Th.)

| Elementaranalyse: | | | |
|---|---|---|---|
| ber. | C 63,42 | H 6,65 | N 12,33 |
| gef. | C 63,17 | H 6,80 | N 12,28 |

b) Vollgeschütztes Benzyloxycarbonyl-32-Polyamin auf der Basis des N,N',N'',N'''-Tetralds{N,N-Bis[2-(trilysylamino)-ethyl]-glycyl}-cyclens

1,82 g (1 mmol) des im Beispiel 11a beschriebenen Okta-Benzyloxycarbonylamins werden in Eisessig gelöst und unter Rühren mit 33 prozentigem Bromwasserstoff in Eisessig versetzt. Nach 90 Minuten wird mit Diethylether die begonnende Fällung vervollständigt, das entstandene Okta-amin-hydrobromid mit Ether gewaschen, im Vakuum getrocknet und ohne weitere Reinigung in die weiter unten beschriebene Reaktion eingesetzt.
Ausbeute: 1,39 g (quantitativ)

9,4 g (10 mmol) des in Beispiel 1c beschriebenen geschützten "Tri-Lysins", 1,5 g (10 mmol) 1-Hydroxybenzotriazol und 3,2 g (10 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Diese Lösung wird anschließend mit 5,16 ml (30 mmol) N-Ethyldiisopropylamin und mit 1,39 g (1 mmol) des oben beschriebenen Okta-amin-hydrobromids versetzt und über Nacht bei Raumtemperatur gerührt. Nach Beendigung der Reaktion wird im Vakuum eingedampft und der Rückstand mit Dichlormethan/Methanol (10:1) an Kieselgel chromatographiert.
Ausbeute: 6,0 g (74 % d. Th.)

| Elementaranalyse: | | | |
|---|---|---|---|
| ber. | C 63,95 | H 6,86 | N 11,05 |
| gef. | C 64,23 | H 7,01 | N 10,92 |

c) 32-Gadolinium-DTPA-monoamid auf der Basis des deblockierten 32-Amins aus Beispiel 11b

1,62 g (0,2 mmol) des in Beispiel 11b beschriebenen Poly-Benzyloxycarbonylamins werden in Eisessig gelöst unter Rühren mit 33 prozentigem Bromwasserstoff in Eisessig versetzt. Nach 3 Stunden wird mit Diethylether die

begonnene Fällung vervollständigt, das entstandene 32-Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden 7,7 g (19,2 mmol) $N^3$-(2,6-Dioxomorpholinoethyl)-$N^6$-(ethoxycarbonylmethyl)-3,6-diazaoctandisäure (Beispiel 13a der EP 0331 616) in fester Form zugegeben, wobei der pH durch weitere Zugabe von Natronlauge bei 9,5 konstant gehalten wird. Nach Beendigung der Zugabe wird zur Verseifung des DTPA-ethylesters mit 5 N Natronlauge ein pH > 13 eingestellt und über Nacht bei Raumtemperatur gerührt. Dann wird mit konz. Salzsäure auf pH 5 eingestellt, mit 3,48 g (9,6 mmol) $Gd_2O_3$ versetzt, 30 Minuten bei 80°C gerührt, nach dem Abkühlen auf pH 7 eingestellt und über eine YM3 AMICON-Ultrafiltrationsmembran entsalzt. Das Retentat wird schließlich membranfiltriert und gefriergetrocknet.
Ausbeute: 4,0 g (89 % d. Th.)
$H_2O$-Gehalt (Karl-Fischer): 4,9 %
Gd-Bestimmung (AAS): 22,1 %

| Elementaranalyse (berechnet auf wasserfreie Substanz): | | | | | |
|---|---|---|---|---|---|
| ber. | C 35,05 | H 4,28 | Gd 23,58 | N 10,48 | Na 3,01 |
| gef. | C 34,82 | H 4,40 | Gd 23,19 | N 10,46 | Na 2,79 |

**Beispiel 12**

a) 32-Thio-Harnstoff-Konjugat aus dem Gd-Komplex des 10-[7-(4-Isothiocyanatophenyl)-2-hydroxy-5-oxo-7-(carboxymethyl)-4-aza-heptyl]-1,4,7-tris(carboxymethyl)-1,4,7,10-tetraazacyclododecans mit dem deblockierten 32-Amin aus Beispiel 11b

1,62 g (0,2 mmol) des in Beispiel 11b beschriebenen 32-Benzyloxycarbonylamins werden in Eisessig gelöst und unter Rühren mit 33 prozentigem Bromwasserstoff in Eisessig versetzt. Nach 3 Stunden wird mit Ether die begonnene Fällung vervollständigt, das entstandene 32-Amin-hydrobromid mit Diethylether gewaschen und im Vakuum getrocknet.
Der Rückstand wird anschließend in Wasser aufgenommen und durch Zugabe von 1 N Natronlauge auf pH 9,5 eingestellt. In diese Lösung werden portionsweise 8,1 g (9,6 mmol) des im Beispiel 2f beschriebenen Isothiocyanats in fester Form zugegeben,
wobei der pH durch weitere Zugabe von Natronlauge bei 9,5 konstant gehalten wird. Nach Beendigung der Zugabe wird über Nacht bei Raumtemperatur gerührt und anschließend mit verd. Salzsäure neutralisiert. Die Lösung wird ultrafiltriert (Amicon YM3) und das Retentat gefriergetrocknet.
Ausbeute: 5,6 g (88 % d. Th.)
$H_2O$-Gehalt (Karl-Fischer): 3,5 %
Gd-Bestimmung (AAS): 15,9 %

| Elementaranalyse (berechnet auf wasserfreie Substanz): | | | | | | |
|---|---|---|---|---|---|---|
| ber. | C 43,18 | H 5,19 | Gd 16,39 | N 11,68 | Na 2,10 | S 3,34 |
| gef. | C 42,95 | H 5,33 | Gd 16,02 | N 11,90 | Na 1,71 | S 2,98 |

**Beispiel 13**

a) N,N,N',N',N",N"-Hexakis{2-[N,N-bis(2-benzyioxycarbonylamino-ethyl)amino]ethyl}-trimesinsäuretriamid

1,27 g (1 mmol) des in Beispiel 1b beschriebenen Hexa-Benzyloxycarbonylamins werden in Eisessig gelöst und unter Rühren mit 33 prozentigem Bromwasserstoff in Eisessig versetzt. Nach 60 Minuten wird mit Diethylether die begonnene Fällung vervollständigt, das entstandene Hexamin-hydrobromid mit Ether gewaschen, im Vakuum getrocknet und ohne weitere Reinigung in die weiter unten beschriebene Reaktion eingesetzt.
Ausbeute: 0,95 g (quantitativ)
0,95 g (1 mmol) des so hergestellten Hydrobromids werden in 50 ml Acetonitril suspendiert und 3 ml Triethylamin zugegeben. Dann werden 3,54 g (20 mmol) N-Benzyloxycarbonyl-aziridin (hergestellt nach J. Chem. Soc. Perkin Trans. 1, 21-26, 1993) zugegeben und 5 Tage unter Stickstoff unter Rückfluß erhitzt. Man dampft zur Trockne ein, nimmt mit 100 ml Methylenchlorid auf und wäscht 2 mal mit je 100 ml 5 prozentiger Kaliumcarbonat-Lösung. Die Methylenchloridphase wird über Magnesiumsulfat getrocknet und eingedampft. Der Rückstand wird an Kieselgel chromatographiert (Laufmittel: Methylenchlorid/Methanol/Triethylamin 20:1:0,1).
Ausbeute: 2,31 g (89 % d. Th.) eines blaßgelben zähen Öls

| Elementaranalyse: | | | |
|---|---|---|---|
| ber. | C 65,34 | H 6,65 | N 11,35 |
| gef. | C 65,12 | H 6,80 | N 11,19 |

b) N,N,N',N',N",N"-Hexakis{2-[N,N-bis(2-aminoethyl)amino]ethyl}-trimesinsäuretriamid

2,2 g (0,85 mmol) der Titelverbindung aus Beispiel 13a werden in 100 ml Methanol gelöst und 3 g Pearlman-Katalysator (Palladiumhydroxid auf Aktivkohle; Fluka) zugegeben. Man hydriert 10 Stunden bei 5 bar (Raumtemperatur). Es wird vom Katalysator abfiltriert und das Filtrat zur Trockne eingedampft.
Ausbeute: 825 mg (99 % d. Th.)

| Elementaranalyse: | | | |
|---|---|---|---|
| ber. | C 55,02 | H 10,16 | N 26,94 |
| gef. | C 54,87 | H 10,25 | N 29,85 |

c) Vollgeschütztes Benzyloxycarbonyl-24-Polyamin auf der Basis des N,N,N',N',N",N"-Hexakis{2-[N,N-bis(2-(N,N-bis-aminoethyl)amino)ethyl]-aminoethyl}-trimesinsäure-triamids

800 mg (0,814 mmol) der Titelverbindung aus Beispiel 13b werden in 150 ml Acetonitril gelöst und 5,77 g (32,56 mmol) N-Benzyloxycarbonylaziridin (hergestellt nach J. Chem. Soc., Perkin Trans., 1, 21-26, 1993) zugegeben und 5 Tage (unter Stickstoff) unter Rückfluß erhitzt. Man dampft im Vakuum zur Trockne ein und chromatographiert an Kieselgel (Laufmittel: Methylenchlorid/Methanol/Triethylamin 20:1:0,1).
Ausbeute: 3,62 g (85 % d. Th.) eines glasigen Feststoffs

| Elementaranalyse: | | | |
|---|---|---|---|
| ber. | C 65,39 | H 6,99 | N 12,04 |
| gef. | C 65,21 | H 7,10 | N 11,90 |

d) Vollgeschütztes Benzyloxycarbonyl-48-Polyamin auf der Basis des 24-Lysyl-Derivats von Beispiel 13c

1,05 g (0,2 mmol) der Titelverbindung aus Beispiel 13c werden in 30 ml Methanol gelöst und 1 g Pearlman-Katalysator (Palladiumhydroxid auf Aktivkohle) zugegeben. Man hydriert 10 Stunden bei 5 bar (Raumtemperatur). Es wird vom Katalysator abfiltriert, das Filtrat zur Trockne eingedampft und in die weiter unten beschriebene Reaktion eingesetzt.
Ausbeute: 0,40 g (quantitativ).

3,11 g (7,5 mmol) $N_{\alpha}$,$N_{\varepsilon}$-Bis(benzyloxycarbonyl)-Lysin (Bachem, Schweiz), 1,2 g (7,5 mmol) 1-Hydroxybenzotriazol und 2,4 g (7,5 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Diese Lösung wird anschließend mit 5,16 ml (30 mmol) N-Ethyldiisopropylamin und mit 0,40 g (0,2 mmol) des oben beschriebenen 24-Polyamins versetzt und über Nacht bei Raumtemperatur gerührt. Nach Beendigung der Reaktion wird im Vakuum eingedampft und der Rückstand mit Ethylacetat/Ethanol/Triethylamin (2:1:0,2) an Kieselgel chromatographiert.
Ausbeute: 1,36 g (59 % d. Th.)

| Elementaranalyse: | | | |
|---|---|---|---|
| ber. | C 64,69 | H 6,95 | N 11,30 |
| gef. | C 64,42 | H 7,11 | N 11,19 |

e) 48-Gadolinium-DTPA-monoamid auf der Basis des deblockierten 48-Amins aus Beispiel 13d

1,15 g (0,1 mmol) der Titelverbindung aus Beispiel 13d werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 48-Amin-hydrobromid mit Ether gewaschen und im Vakuum getrocknet.

Der Rückstand wird anschließend in Wasser aufgenommen und durch Zugabe von 1 N Natronlauge auf pH 9,5 eingestellt. In diese Lösung werden 5,8 g (14,4 mmol) $N^3$-(2,6-Dioxomorpholinoethyl)-$N^6$-(ethoxycarbonylmethyl)-3,6-diazaoctandisäure (Beispiel 13a der EP 0 331 616) in fester Form zugegeben, wobei der pH durch weitere Zugabe von Natronlauge bei 9,5 konstant gehalten wird. Nach Beendigung der Zugabe wird zur Verseifung des DTPA-ethylesters mit 5 N Natronlauge ein pH > 13 eingestellt und über Nacht bei Raumtemperatur gerührt. Dann wird mit konz. Salzsäure auf pH 5 eingestellt, mit 2,61 g (7,2 mmol) $Gd_2O_3$ versetzt, 30 Minuten bei 80°C gerührt, nach dem Abkühlen auf pH 7 eingestellt und über eine YM3 AMICON-Ultrafiltrationsmembran entsalzt. Das Retentat wird schließlich membranfiltriert und gefriergetrocknet.
Ausbeute: 3,15 g (92 % d. Th.)
$H_2O$-Gehalt (Karl-Fischer): 8,9 %
Gd-Bestimmung (AAS): 21,9 %

| Elementaranalyse (berechnet auf wasserfreie Substanz): | | | | | |
|---|---|---|---|---|---|
| ber. | C 35,03 | H 4,34 | Gd 24,22 | N 10,65 | Na 2,21 |
| gef. | C 34,84 | H 4,50 | Gd 23,63 | N 10,87 | Na 2,04 |

**Beispiel 14**

a) 48-Thio-Harnstoff-Konjugat aus dem Gd-Komplex des 10-[7-(4-Isothiocyanato-phenyl)2-hydroxy-5-oxo-7-(carboxymethyl)-4-aza-heptyl]-1,4,7-tris(carboxymethyl)-1,4,7,10-tetraazacyclododecans mit dem deblockierten 48-Amin aus Beispiel 13d

1,15 g (0,1 mmol) der Titelverbindung aus Beispiel 13d werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 48-Amin-hydrobromid mit Ether gewaschen und im Vakuum getrocknet.

Der Rückstand wird anschließend in Wasser aufgenommen und durch Zugabe von 1 N Natronlauge auf pH 9,5 eingestellt. In diese Lösung werden portionsweise 6,08 g (7,2 mmol) des im Beispiel 2f beschriebenen Isothiocyanats in fester Form zugegeben,
wobei der pH durch weitere Zugabe von Natronlauge bei 9,5 konstant gehalten wird. Nach Beendigung der Zugabe wird über Nacht bei Raumtemperatur gerührt und anschließend mit verd. Salzsäure neutralisiert. Die Lösung wird ultrafiltriert (Amicon YM3) und das Retentat gefriergetrocknet.
Ausbeute: 3,91 g (82 % d. Th.)
$H_2O$-Gehalt (Karl-Fischer): 4,5 %
Gd-Bestimmung (AAS): 16,6 %

| Elementaranalyse (berechnet auf wasserfreie Substanz): | | | | | | |
|---|---|---|---|---|---|---|
| ber. | C 43,33 | H 5,24 | Gd 16,71 | N 11,82 | Na 1,53 | S 3,41 |
| gef. | C 43,04 | H 5,54 | Gd 16,19 | N 12,10 | Na 1,26 | S 3,89 |

**Beispiel 15**

a) 1,4,7,10,13,16-Hexakis[2-(Benzyloxycarbonylamino)-ethyl]-1,4,7,10,13,16-Hexaazacyclooctadecan

1,29 g (5 mmol) 1,4,7,10,13,16-Hexaazacyclooctadecan (Hexacyclen; Fluka) werden mit Toluol azeotrop entwässert. Zu der auf Raumtemperatur abgekühlten Lösung von Hexacyclen in Toluol wird eine Lösung von 10,63 g (60 mmol) N-Benzyloxycarbonylaziridin (J. Chem. Soc., Perkin Trans. 1, 21-26, 1993) in Acetonitril zugegeben und 3 Tage unter Stickstoff unter Rückfluß erhitzt. Man dampft zur Trockne ein und chromatographiert den Rückstand in Ethylacetat/Methanol/Triethylamin (8;2:0,5) an Kieselgel.
Ausbeute: 5,68 g (86 % d. Th.) blaßgelbes, zähes Öl

| Elementaranalyse: | | | |
|---|---|---|---|
| ber. | C 65,43 | H 7,32 | N 12,72 |
| gef. | C 65,20 | H 7,51 | N 12,49 |

b) Vollgeschütztes Benzyloxycarbonyl-24-Polyamin auf der Basis des 1,4,7,10,13,16-Hexakis-[2-(trilysylamino)-ethyl]-hexacyclens

1,32 g (1 mmol) des in Beispiel 15a beschriebenen Hexa-Benzyloxycarbonylamins werden in Eisessig gelöst und unter Rühren mit 33 prozentigem Bromwasserstoff in Eisessig versetzt. Nach 60 Minuten wird mit Diethylether die begonnene Fällung vervollständigt, das entstandene Hexaamin-hydrobromid mit Ether gewaschen, im Vakuum getrocknet und ohne weitere Reinigung in die weiter unten beschriebene Reaktion eingesetzt.
Ausbeute: 1,48 g (quantitativ)

7,0 g (7,5 mmol) des in Beispiel 1c beschriebenen geschützten "Tri-Lysins", 1,2 g (7,5 mmol) 1-Hydroxybenzotriazol und 2,4 g (7,5 mmol) Z-(1H-Benzotriazol-1-yl)-1,1,3,3-tetramethyluronium tetrafluorborat (TBTU; Peboc Limited, UK) werden in DMF gelöst und 15 Minuten gerührt. Diese Lösung wird anschließend mit 5,16 ml (30 mmol) N-Ethyldiisopropylamin und mit 1,48 g (1 mmol) des oben beschriebenen Hexa-amin-hydrobromids versetzt und über Nacht bei Raumtemperatur gerührt. Nach Beendigung der Reaktion wird im Vakuum eingedampft und der Rückstand mit Dichlormethan/Methanol (10:1) an Kieselgel chromatographiert.
Ausbeute: 5,02 g (83 % d. Th.)

| Elementaranalyse: | | | |
|---|---|---|---|
| ber. | C 64,40 | H 7,00 | N 11,13 |
| gef. | C 64,16 | H 6,82 | N 10,88 |

c) 24-Gadolinium-DTPA-monoamid auf der Basis des 1,4,7,10,13,16-Hexakis[2-(trilysyl-amino)ethyl]-hexacyclens

1,21 g (0,2 mmol) des in Beispiel 15b beschriebenen Poly-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 24-Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden 5,8 g (14,4 mmol) $N^3$-(2,6-Dioxomorpholinoethyl)-$N^6$-(ethoxycarbonylmethyl)-3,6-diazaoctandisäure (Beispiel 13a der EP 0331 616) in fester Form zugegeben, wobei der pH durch weitere Zugabe von Natronlauge bei 9,5 konstant gehalten wird. Nach Beendigung der Zugabe wird zur Verseifung des DTPA-ethylesters mit 5 N Natronlauge ein pH > 13 eingestellt und über Nacht bei Raumtemperatur gerührt. Dann wird mit konz. Salzsäure auf pH 5 eingestellt, mit 2,61 g (7,2 mmol) $Gd_2O_3$ versetzt, 30 Minuten bei 80°C gerührt, nach dem Abkühlen auf pH 7 eingestellt und über eine YM3 AMICON-Ultrafiltrationsmembran entsalzt. Das Retentat wird schließlich membranfiltriert und gefriergetrocknet.
Ausbeute: 3,17 g (92,5 % d. Th.)
$H_2O$-Gehalt (Karl-Fischer): 8,7 %
Gd-Bestimmung (AAS): 21,9 %

| Elementaranalyse (berechnet auf wassetfreie Substanz): | | | | | |
|---|---|---|---|---|---|
| ber. | C 35,94 | H 4,45 | Gd 24,13 | N 10,75 | Na 2,65 |
| gef. | C 36,19 | H 4,26 | Gd 24,39 | N 10,48 | Na 2,29 |

**Beispiel 16**

a) 24-Thio-Harnstoff-Konjugat aus dem Gd-Komplex des 10-[7-(4-Isothiocyanatophenyl)-2-hydroxy-5-oxo-7-(carboxymethyl)-4-aza-heptyl]-1,4,7-tris-(carboxymethyl)-1,4,7,10-tetraazacyclododecans mit dem 24-Amin 1,4,7,10,13,16-Hexakis[2-trilysylamino)-ethyl]hexacyclen

1,21 g (0,2 mmol) des in Beispiel 15b beschriebenen 16-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 24-Amin-hydrobromid mit Ether gewaschen und im Vakuum getrocknet.

Der Rückstand wird anschließend in Wasser aufgenommen und durch Zugabe von 1 N Natronlauge auf pH 9,5 eingestellt. In diese Lösung werden portionsweise 6,08 g (7,2 mmol) des im Beispiel 2f beschriebenen Isothiocyanats in fester Form zugegeben,
wobei der pH durch weitere Zugabe von Natronlauge bei 9,5 konstant gehalten wird. Nach Beendigung der Zugabe

wird über Nacht bei Raumtemperatur gerührt und anschließend mit verd. Salzsäure neutralisiert. Die Lösung wird ultrafiltriert (Amicon YM3) und das Retentat gefriergetrocknet.
Ausbeute: 4,33 g (89 % d. Th.)
$H_2O$-Gehalt (Karl-Fischer): 7,0 %
Gd-Bestimmung (AAS): 15,7 %

| Elementaranalyse (berechnet auf wasserfreie Substanz): | | | | | | |
|---|---|---|---|---|---|---|
| ber. | C 43,94 | H 5,32 | Gd 16,67 | N 11,88 | Na 1,83 | S 3,40 |
| gef. | C 43,70 | H 5,16 | Gd 16,21 | N 12,05 | Na 1,49 | S 3,78 |

**Beispiel 17**

a) Gadolinium-Komplex von 10-[6-(4-Nitrophenyl)-2-hydroxy-5-oxo-4-azahexyl]-1,4,7-tris(carboxymethyl)-1,4,7,10-tetraazacyclododecan

20 g (34,86 mmol) der Titelverbindung aus Beispiel 2c werden in 200 ml Wasser gelöst und der pH-Wert mit 2 normaler Natronlauge auf pH 10 gebracht. Bei 0°C tropft man hierzu eine Lösung aus 13,97 g (70 mmol)4-Nitrophenylessigsäurechlorid in 50 ml Dioxan zu und hält den pH-Wert durch Zugabe von 2 normaler Natronlauge bei pH 10. Man läßt 2 Stunden bei Raumtemperatur nachrühren. Dann wird mit 10 %iger Salzsäure auf pH 7 gestellt und die wässrige Lösung 2 mal mit 200 ml Essigester extrahiert. Die wässrige Phase wird zur Trockne eingedampft und der Rückstand durch Chromatographie gereinigt (RP-18/LiChroPrep®/Laufmittel: Gradient aus Tetrahydrofuran/Methanol/ Wasser). Nach Eindampfen der Hauptfraktionen erhält man 22,5 g (81 % d. Th.) eines cremefarbenen Feststoffes.
Wassergehalt: 7,5 %

| Analyse (bezogen auf wasserfreie Substanz): | | | | |
|---|---|---|---|---|
| ber. | C 50,75 | H 4,79 | Gd 21,34 | N 11,41 |
| gef. | C 40,61 | H 4,89 | Gd 21,15 | N 11,30 |

b) Gadolinium-Komplex von 10-[6-(4-Aminophenyl)-2-hydroxy-5-oxa-4-aza-hexyl]-1,4,7-tris(carboxymethyl)-1,4,7,10-tetraazacyclododecan

21 g (28,5 mmol) der Titelverbindung aus Beispiel 17a werden in 200 ml Methanol gelöst und 4 g Palladiumkatalysator (10 % Pd auf C) zugegeben. Man hydriert 6 Stunden bei Raumtemperatur. Der Katalysator wird abfiltriert und das Filtrat zur Trockne eingedampft. Ausbeute: 20,56 g (99 % d. Th.) gelblicher, glasiger Feststoff
Wassergehalt: 13,1 %

| Analyse (bezogen auf wasserfreie Substanz): | | | | |
|---|---|---|---|---|
| ber. | C 42,48 | H 5,28 | Gd 22,25 | N 11,89 |
| gef. | C 42,31 | H 5,41 | Gd 22,07 | N 11,67 |

c) Gadolinium-Komplex von 10-[6-(4-Isothiocyanatophenyl)-2-hydroxy-5-oxo-4-aza-hexyl]-1,4,7-tris(carboxymethyl)-1,4,7,10-tetraazacyclododecan

20 g (28,29 mmol) der Titelverbindung aus Beispiel 17b werden in 100 ml Wasser gelöst und 9,76 g (84,9 mmol) Thiophosgen in 50 ml Chloroform zugegeben. Man rührt 1 Stunde bei 50°C. Man kühlt auf Raumtemperatur ab, trennt die organische Phase ab und schüttelt die wässrige Phase 2 mal mit 100 ml Chloroform aus. Die wässrige Phase wird zur Trockne eingedampft, und der Rückstand in 200 ml Isopropanol bei Raumtemperatur ausgerührt. Man filtriert den Feststoff ab, wäscht mit Ether nach und trocknet über Nacht im Vakuum bei 40°C.
Ausbeute: 21,7 g (99 % d. Th.) eines leicht cremefarbenen Feststoffes
Wassergehalt: 3,4 %

| Analyse (bezogen auf wasserfreie Substanz): | | | | | |
|---|---|---|---|---|---|
| ber. | C 41,70 | H 4,71 | Gd 21,00 | N 11,22 | S 4,28 |
| gef. | C 41,55 | H 4,85 | Gd 20,85 | N 11,11 | S 4,20 |

d) N,N,N',N'-Tetrakis[2-(benzyloxycarbonylamino)-ethyl]-5-nitro-isophthalsäure-diamid

14,86 g (40 mmol) des in Beispiel 1a beschriebenen Bis[2-(benzyloxycarbonylamino)-ethyl]-amins und 20,24 g (200 mmol) Triethylamin werden in Dimethylformamid gelöst und mit 4,96 g (20 mmol) 5-Nitro-isophthalsäuredichlorid (J. Chem. Soc. 1957, 1172-1175) versetzt und über Nacht bei Raumtemperatur gerührt. Die Lösung wird im Vakuum eingedampft und der Rückstand mit Ethylacetat an Kieselgel chromatographiert.
Ausbeute: 11,5 g (62,6 %) eines zähen Öls

| Elementaranalyse: | | | |
|---|---|---|---|
| ber. | C 62,80 | H 5,60 | N 10,68 |
| gef. | C 62,62 | H 5,77 | N 10,41 |

e) N,N,N',N'-Tetrakis[2-(benzyloxycarbonylamino)-ethyl]-5-amino-isophthalsäure-diamid

9,18 g (10 mmol) der im vorstehenden Beispiel beschriebenen Nitroverbindung in 200 ml Ethanol werden zu einer Lösung von 27,8 g (100 mmol) $FeSO_4$ x 7 $H_2O$ in 200 ml Wasser zugegeben und zum Sieden erhitzt. Nach Zugabe von 50 ml konz. Ammoniak wird weitere 90 Minuten unter Rückfluß gerührt. Man läßt die Suspension abkühlen, dann wird das Ethanol im Vakuum eingeengt und die wässrige Phase mit Ethylacetat ausgeschüttelt. Man erhält nach Trocknung über Natriumsulfat 8,61 g (97 % d. Th.) eines fast farblosen zähen Öls.

| Elementaranalyse: | | | |
|---|---|---|---|
| ber. | C 64,93 | H 6,02 | N 11,04 |
| gef. | C 65,10 | H 5,96 | N 10,89 |

f) N,N,N',N'-Tetrakis[2-(benzyloxycarbonylamino)-ethyl]-5-(carboxymethoxyacetylamino)-isophthalsäure-diamid

4,44 g (5 mmol) der im vorstehenden Beispiel beschriebenen Aminoverbindung in Dimethylformamid werden im Eisbad mit 5,05 g (50 mmol) Triethylamin und 2,9 g (25 mmol) Diglycolsäureanhydrid (Fluka) versetzt und 2 Stunden bei 0°C gerührt. Es wird im Vakuum zur Trockne eingedampft, der Rückstand wird zwischen Ethylacetat und 1 M Zitronensäure verteilt, mit Wasser neutral gewaschen und die organische Phase getrocknet.
Ausbeute: 4,62 g (92 % d. Th.) eines zähen Öls

| Elementaranalyse: | | | |
|---|---|---|---|
| ber. | C 62,20 | H 5,72 | N 9,77 |
| gef. | C 62,03 | H 5,60 | N 9,89 |

g) Vollgeschütztes Benzyloxycarbonyl-Hexadeca-amin auf der Basis des N,N',N'',N'''-Tetrakis-(trilysytaminomethyl]-methans

3,0 g (22,69 mmol) Tetrakis(aminomethyl)methan (hergestellt nach US 4,485,237 A, 1984), 127,85 g (136,15 mmol) der Titelverbindung aus Beispiel 1c und 15,67 g (136,15 mmol) N-Hydroxysuccinimid werden in 300 ml Dimethylformamid gelöst. Bei 0°C gibt man 28,09 g (136,15 mmol) Dicyclohexylcarbodiimid hinzu und rührt 1 Stunde bei 0°C, dann 2 Tage bei Raumtemperatur. Man gibt 300 ml Ethylacetat zu, filtriert vom ausgefallenen Harnstoff ab und engt das Filtrat im Vakuum zur Trockne ein. Der Rückstand wird an Kieselgel. chromatographiert (Laufmittel: Methylenchlorid/Isopropanol 20:1)
Ausbeute: 58,02 g (67 % d. Th.) eines farblosen zähen Öls

| Elementaranalyse: | | | |
|---|---|---|---|
| ber. | C 64,52 | H 6,76 | N 10,28 |
| gef. | C 64,41 | H 6,91 | N 10,05 |

h) Vollgeschütztes Benzyloxycarbonyl-64-Amin aus dem mit der Tetraamin-monocarbon-säure 17f kondensierten 16-AminN,N',N'',N'''-Tetrakis-(trilysylaminomethyl)-methan

1,91 g (0,5 mmol) des in Beispiel 17g beschriebenen 16-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 16-Amin-hydrobromid mit Ether gewaschen, im Vakuum getrocknet und ohne weitere Reinigung in die folgende Reaktion eingesetzt.
Ausbeute: 1,48 g (quantitativ) 12,05 g (12 mmol) der in Beispiel 17f beschriebenen Tetraamin-monocarbonsäure, 1,92 g (12 mmol) 1-Hydroxybenzotriazol (HOBt) und 3,84 g (12 mmol) 2-(1H-Benzotriazol-1-yl)-1,1,3,3-tetramethyl-uronium tetrafluorborat (TBTU; Peboc Limited, UK) werden in DMF gelöst und 15 Minuten gerührt. Diese Lösung wird anschließend mit 6,20 ml (36 mmol) N-Ethyldiisopropylamin und mit 1,48 g (0,5 mmol) des oben beschriebenen 16-Amin-hydrobromids versetzt und über Nacht bei Raumtemperatur gerührt. Nach Beendigung der Reaktion wird im Vakuum eingedampft und der Rückstand mit Ethylacetat/Ethanol (2:1) an Kieselgel chromatographiert.
Ausbeute: 4,54 g (52 % d. Th.) eines hellgelben Öls

| Elementaranalyse: | | | |
|---|---|---|---|
| ber. | C 62,58 | H 6,01 | N 11,24 |
| gef. | C 62,39 | H 6,22 | N 11,00 |

i) 64-Thio-Harnstoff-Konjugat aus dem Gadolinium-Komplex des 10-[6-(4-Isothiocyanatophenyl)-2-hydroxy-5-oxo-4-aza-hexyl]-1,4,7-tris(carboxymethyl)-1,4,7,10-tetraazacyclododecans mit dem deblockierten 64-Polyamin aus Beispiel 17h

1,74 g (0,1 mmol) des in Beispiel 17h beschriebenen 64-Benzyloxycarbonylamins werden in Eisessig gelöst und unter Rühren mit 33 prozentigem Bromwassetstoff in Eisessig versetzt. Nach 3 Stunden wird mit Diethylether die begonnene Fällung vervollständigt, das entstandene 64-Amin-hydrobromid mit Ether gewaschen und im Vakuum getrocknet. Der Rückstand wird anschließend in Wasser aufgenommen und durch Zugabe von 1 N Natronlauge auf pH 9,5 eingestellt. In diese Lösung werden portionsweise 7,20 g (9,6 mmol) des im Beispiel 17c beschriebenen Isothiocyanats in fester Form zugegeben, wobei der pH durch weitere Zugabe von Natronlauge bei 9,5 konstant gehalten wird. Nach Beendigung der Zugabe wird über Nacht bei Raumtemperatur gerührt und anschließend mit verd. Salzsäure neutralisiert. Die Lösung wird ultrafiltriert (Amicon YM3) und das Retentat gefriergetrocknet.
Ausbeute: 5,31 g (86 % d. Th.)
$H_2O$-Gehalt (Karl-Fischer): 8,0 %
Gd-Bestimmung (AAS): 16,2 %

| Elementaranalyse (berechnet auf wasserfreie Substanz): | | | | | |
|---|---|---|---|---|---|
| ber. | C 43,59 | H 5,14 | Gd 17,72 | N 12,92 | S 3,61 |
| gef. | C 43,36 | H 5,41 | Gd 17,18 | N 13,20 | S 3,04 |

**Beispiel 18**

a) 2-Nitro-5-hydroxy-benzoesäurebenzylester

40 g (218,4 mmol) 2-Hydroxy-5-nitro-benzoesäure, 236,17 g (2,184 mol) Benzylalkohol und 1 g p-Toluolsulfonsäure in 1000 ml Toluol werden 2 Tage am Wasserabscheider erhitzt. Man dampft zur Trockne ein und chromatographiert den Rückstand an Kieselgel (Laufmittel: Methylenchlorid/Hexan/Aceton= 15:5:1).
Ausbeute: 50,72 g (85 % d. Th.) eines gelben Feststoffes

| Analyse: | | | |
|---|---|---|---|
| ber. | C 61,54 | H 4,06 | N 5,13 |
| gef. | C 61,31 | H 4,17 | N 5,05 |

b) 2-[4-Nitro-2-(benzyloxycarbonyl)-phenoxy]-essigsäure-tert.-butylester

Zu 50 g (183 mmol) der Titelverbindung aus Beispiel 18a, 20,5 g (366 mmol) feingepulvertem Kaliumhydroxid

und 500 mg Tetrabutylammoniumhydrogensulfat in 500 ml Toluol gibt man bei 0°C 42,8 g (220 mmol) Bromessigsäure-tert.-butylester zu und rührt 3 Stunden bei 0°C. Man versetzt mit 500 ml Eiswasser, rührt 2 Minuten kräftig durch und trennt die organische Phase ab. Die organische Phase wird über Magnesiumsulfat getrocknet und im Vakuum eingedampft. Der Rückstand wird an Kieselgel chromatographiert (Laufmittel: Methylenchlorid/Hexan/Aceton= 15:10:1).
Ausbeute: 47,5 g (67 % d. Th.) eines gelben zähen Öls

| Analyse: | | | |
|---|---|---|---|
| ber. | C 62,01 | H 5,46 | N 3,62 |
| gef. | C 62,13 | H 5,55 | N 3,50 |

c) 2-[4-Nitro-2-(benzyloxycarbonyl)-phenoxy]-essigsäure

40 g (103,26 mmol) der Titelverbindung aus Beispiel 18b werden in 300 ml Methylenchlorid gelöst. Bei 0°C tropft man 100 ml Trifluoressigsäure hinzu und läßt auf Raumtemperatur kommen. Nach 4 Stunden dampft man zur Trockne ein. Der Rückstand wird aus wenig Ether/Hexan umkristallisiert.
Ausbeute: 32,5 g (95 % d. Th.) eines leicht gelblichen Feststoffes

| Analyse: | | | |
|---|---|---|---|
| ber. | C 58,01 | H 3,96 | N 4,23 |
| gef. | C 58,17 | H 3,81 | N 4,18 |

d) N-Hydroxysuccinimidester von 2-[4-Nitro-2-(benzyloxycarbonyl)-phenoxy]-essigsäure

10 g (30,19 mmol) der Titelverbindung aus Beispiel 18c und 4,17 g (36,28 mmol) N-Hydroxysuccinimid werden in 20 ml Chloroform gelöst und bei 0°C 7,48 g (36,23 mmol) Dicyclohexylcarbodiimid zugesetzt. Man rührt 24 Stunden bei Raumtemperatur. Es wird im Eisbad gekühlt und vom ausgefallenen Harnstoff abfiltriert. Das Filtrat wird im Vakuum zur Trockne eingedampft und der Rückstand aus wenig Isopropanol umkristallisiert.
Ausbeute: 12,03 g (93 % d. Th.) eines leicht gelblichen kristallinen Feststoffes

| Analyse: | | | |
|---|---|---|---|
| ber. | C 56,08 | H 3,76 | N 6,54 |
| gef. | C 56,17 | H 3,84 | N 6,41 |

e) Gadolinium-Komplex von 10-[6-(4-Nitro-2-(benzyloxycarbonyl)-phenoxy)-2-hydroxy-5-oxo-4-aza-hexyl]-1,4,7-tris (carboxymethyl)-1,4,7,10-tetraazacyclododecaa

Zu 11 g (25,68 mmol) der Titelverbindung aus Beispiel 2b in 100 ml Dimethylformamid/20 ml Triethylamin gibt man bei Raumtemperatur 13,26 g (23,11 mmol) der Titelverbindung aus Beispiel 18d zu und rührt über Nacht. Man dampft im Vakuum zur Trockne ein und chromatographiert den Rückstand an RP-18 (LiChroPrep®/Laufmittel: Wasser/Tetrahydrofuran-Gradient).
Ausbeute: 19,02 g (81 % d. Th.) eines amorphen Feststoffes
Wassergehalt: 3,1 %

| Analyse (bezogen auf wasserfreie Substanz): | | | | |
|---|---|---|---|---|
| ber. | C 44,69 | H 4,66 | Gd 17,73 | N 9,47 |
| gef. | C 44,48 | H 4,80 | Gd 17,56 | N 9,28 |

f) Gadolinium-Komplex von 10-[6-(4-Amino-2-carboxy-phenoxy)-2-hydroxy-5-oxo-4-aza-hexyl]-1,4,7-tris(carboxymethyl)-1,4,7,10-tetraazacyclododecan

18 g (20,3 mmol) der Titelverbindung aus Beispiel 18e werden in 200 ml Methanol gelöst und 5 g Palladium-Katalysator (10 % Pd auf C) zugegeben. Man hydriert 8 Stunden bei Raumtemperatur. Der Katalysator wird abfiltriert und das Filtrat zur Trockne eingedampft. Ausbeute: 15,88 g (98 % d. Th.) eines cremefarbenen Feststoffes
Wassergehalt: 4,1 %

| Analyse (bezogen auf wasserfreie Substanz): | | | | |
|---|---|---|---|---|
| ber. | C 40,72 | H 4,86 | Gd 20,51 | N 10,96 |
| gef. | C 40,51 | H 4,97 | Gd 20,32 | N 10,73 |

g) Gadolinium-Komplex von 10-[6-(4-Isothiocyanato-2-carboxy-phenoxy)-2-hydroxy-5-oxo-4-aza-hexyl]-1,4,7-tris(carboxymethyl)-1,4,7,10-tetraazacyclododecan

15 g (19,56 mmol) der Titelverbindung aus Beispiel 18f werden in 100 ml Wasser gelöst und 6,75 g (58,7 mmol) Thiophosgen in 50 ml Chloroform zugegeben. Man rührt 1 Stunde bei 50°C. Man kühlt auf Raumtemperatur ab, trennt die organische Phase ab und schüttelt die wässrige Phase 2 mal mit 100 ml Chloroform aus. Die wässrige Phase wird zur Trockne eingedampft und der Rückstand in 100 ml Isopropanol bei Raumtemperatur ausgerührt. Der Feststoff wird abfiltriert und mit Ether gewaschen. Nach Trocknen über Nacht im Vakuum (40°C) erhält man 16,5 g (98 % d. Th.) eines cremefarbenen Feststoffes.
Wassergehalt: 5,8 %

| Analyse (bezogen auf wasserfreie Substanz): | | | | | |
|---|---|---|---|---|---|
| ber. | C 40,09 | H 4,36 | Gd 19,44 | N 10,39 | S 3,96 |
| gef. | C 40,15 | H 4,45 | Gd 19,23 | N 10,19 | S 3,87 |

h) 64-Thio-Harnstoff-Konjugat aus dem Gadolinium-Komplex des 10-[6-(4-Isothiocyanato-2-carboxy-phenoxy)-2-hydroxy-5-oxo-4-aza-hexyl]-1,4,7-tris(carboxymethyl)-1,4,7,10-tetraazacyclododecans mit dem deblockiertem 64-Polyamin aus Beispiel 17h

1,74 g (0,1 mmol) des in Beispiel 17h beschriebenen 64-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 64-Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden portionsweise 7,76 g (9,6 mmol) des im vorstehenden Beispiel 18g beschriebenen Isothiocyanats in fester Form zugegeben, wobei der pH durch weitere Zugabe von Natronlauge bei 9,5 konstant gehalten wird. Nach Beendigung der Zugabe wird über Nacht bei Raumtemperatur gerührt und anschließend mit verd. Salzsäure neutralisiert. Die Lösung wird ultrafiltriert (Amicon YM3) und das Retentat gefriergetrocknet.
Ausbeute: 5,21 g (79 % d. Th.)
$H_2O$-Gehalt (Karl-Fischer): 6,0 %
Gd-Bestimmung (AAS): 15,1 %

| Elementaranalyse (berechnet auf wasserfreie Substanz): | | | | | | |
|---|---|---|---|---|---|---|
| ber. | C 41,14 | H 4,60 | Gd 16,22 | N 11,83 | Na 2,37 | S 3,31 |
| gef. | C 40,89 | H 4,70 | Gd 16,65 | N 11,90 | Na 2,08 | S 3,73 |

**Beispiel 19**

a) 3-(4-Isothiocyanatophenyl)-glutarsäure

Zu 22,12 g (100 mmol) 3-(4-Aminophenyl)-glutarsäure in 300 Chloroform gibt man 11,5 g (100 mmol) Thiophosgen und erwärmt 50 Minuten bei 40°C Man dampft zur Trockne ein und kristallisiert den Rückstand aus wenig Isopropanol/Ether um.
Ausbeute: 22,5 g (85 % d. Th.) eines cremefarbenen kristallinen Feststoffes

| Analyse: | | | | |
|---|---|---|---|---|
| ber. | C 54,33 | H 4,18 | N 5,28 | S 12,09 |
| gef. | C 54,17 | H 4,29 | N 5,14 | S 12,15 |

b) Gadolinium-Komplex des 10-{6-[4-(2-carboxy-1-carboxymethyl-ethyl)-phenyl]-2-hydroxy-5-thioxo-4,6-diazahexyl]-1,4,7-tris(carboxymethyl)-1,4,7,10-tetraazacyclododecans

20 g (37,2 mmol) der Titelverbindung aus Beispiel 2c werden in 200 ml Dimethylformamid/50 ml Triethylamin gelöst und 11,84 g (44,63 mmol) der Titelverbindung aus Beispiel 19a zugegeben. Man rührt über Nacht bei Raumtemperatur. Man dampft im Vakuum zur Trockne ein, löst den Rückstand in 200 ml Ethanol/30 ml Essigsäure auf und dampft erneut ein. Dieser Rückstand wird an RP-18 (LiChroPrep®/Laufmittel: Gradient aus Wasser/Tetrahydrofuran) chromatographiert.
Ausbeute: 26,22 g (81 % d. Th.) eines cremefarbenen Feststoffes
Wassergehalt: 3,6 %

| Analyse (bezogen auf wasserfreie Substanz): | | | | | |
|---|---|---|---|---|---|
| ber. | C 41,52 | H 4,93 | Gd 18,74 | N 10,02 | S 3,82 |
| gef. | C 41,33 | H 5,05 | Gd 18,61 | N 10,17 | S 3,75 |

c) Gd-Komplex des 10-{6-[4-(2,6-dioxo-3,4,5,6-tetrahydro-2H-pyran-4-yl)-phenyl]-2-hydroxy-5-thioxo-4,6-diazahexyl}-1,4,7-tris(carboxymethyl)-1,4,7,10-tetraazacyclo-dodecans

10 g (11,92 mmol) der Titelverbindung aus Beispiel 19b werden in 50 ml Dimethylformamid gelöst und bei 0°C 3,7 g (17,88 mmol) Dicyclohexylcarbodiimid zugegeben. Man rührt über Nacht bei Raumtemperatur. Es wird auf 0°C abgekühlt und vom ausgefallenem Harnstoff abfiltriert. Zum Filtrat tropft man unter Rühren 500 ml Ether nach. Nach Trocknen über Nacht im Vakuum (50°C) erhält man 9,6 g (95 % d. Th.) der Titelverbindung als leicht gelblichen Feststoff.
Wassergehalt: 2,7 %

| Analyse (bezogen auf wasserfreie Substanz): | | | | | |
|---|---|---|---|---|---|
| ber. | C 42,43 | H 4,79 | Gd 19,15 | N 10,24 | S 3,91 |
| gef. | C 42,28 | H 4,93 | Gd 19,03 | N 10,05 | S 3,83 |

d) 64-Amid-Konjugat aus dem Gadolinium-Komplex des 10-{6-[4-(2,6-dioxo-3,4,5,6-tetrahydro-2H-pyran-4-yl)-phenyl]-2-hydroxy-5-thioxo-4,6-diazahexyl}-1,4,7-tris-(carboxymethyl)-1,4,7,10-tetraazacyclododecans mit dem deblokkiertem 64-Polyamin aus Beispiel 17h

1,74 g (0,1 mmol) des in Beispiel 17h beschriebenen 64-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 64-Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden portionsweise 15,76 g (19,2 mmol) des im Beispiel 19c beschriebenen Anhydrids in fester Form zugegeben, wobei der pH durch weitere Zugabe von Natronlauge bei 9,5 konstant gehalten wird. Nach Beendigung der Zugabe wird über Nacht bei Raumtemperatur gerührt und anschließend mit verd. Salzsäure neutralisiert. Die Lösung wird ultrafiltriert (Amicon YM3) und das Retentat gefriergetrocknet.
Ausbeute: 5,64 g (83 % d. Th.)
$H_2O$-Gehalt (Karl-Fischer): 7,6 %
Gd-Bestimmung (AAS): 14,7 %

| Elementaranalyse (berechnet auf wasserfreie Substanz): | | | | | | |
|---|---|---|---|---|---|---|
| ber. | C 43,08 | H 4,96 | Gd 16,02 | N 11,68 | Na 2,34 | S 3,27 |
| gef. | C 43,03 | H 4,97 | Gd 15,86 | N 11,69 | Na 2,05 | S 3,11 |

**Beispiel 20**

a) 10-[3-(4-Aminophenoxy)-2-hydroxypropyl]-1,4,7-tris(carboxymethyl)-1,4,7,10-tetraazacyclododecan

15 g (27,7 mmol) 10-[3-(4-Nitrophenoxy)-2-hydroxypropyl]-1,4,7-tris(carboxymethyl)-1,4,7,10-tetraazacyclo-

dodecan (EP 0485045 (Schering AG), Beispiel 12 a) werden in 250 ml Methanol gelöst und 5 g Palladium-Katalysator (10 % Pd auf Aktivkohle) zugegeben. Man hydriert über Nacht bei Raumtemperatur. Der Katalysator wird abfiltriert und das Filtrat im Vakuum zur Trockne eingedampft.
Ausbeute: 14,43 g (98 % d. Th.) eines cremefarbenen Feststoffes
Wassergehalt: 3,8 %

| Analyse (bezogen auf wasserfreie Substanz): | | | |
|---|---|---|---|
| ber. | C 54,00 | H 7,29 | N 13,69 |
| gef. | C 53,88 | H 7,41 | N 13,51 |

b) 10-[3-{4-Bis(carboxymethyl)-aminophenoxy}-2-hydroxypropyl]-1,4,7-tris(carboxy-methyl)-1,4,7,10-tetraazacyclododecan

14 g (27,37 mmol) der Titelverbindung aus Beispiel 20a, 15,21 g (109 mmol) Bromessigsäure und 41,5 g (300 mmol) Kaliumcarbonat in 200 ml Ethanol werden 2 Tage unter Rückfluß erhitzt. Man filtriert vom Feststoff ab und gibt zum Filtrat 50 ml konz. Salzsäure zu. Es wird im Vakuum zur Trockne eingedampft. Der Rückstand wird an RP-18 (LiChroPrep®/Laufmittel: Tetrahydrofuran/Wasser-Gradient) gereinigt.
Ausbeute: 7,81 g (43 % d. Th.) eines cremefarbenen Feststoffes
Wassergehalt: 5,4 %

| Analyse (bezogen auf wasserfreie Substanz): | | | |
|---|---|---|---|
| ber. | C 51,67 | H 6,58 | N 11,16 |
| gef. | C 51,48 | H 6,71 | N 11,03 |

c) Gadolinium-Komplex des 10-[3-{4-Bis(carboxymethyl)-aminophenoxy}-2-hydroxy-propyl]-1,4,7-tris(carboxymethyl)-1,4,7,10-tetraazacyclododecans

Zu 7,5 g (11,95 mmol) der Titelverbindung aus Beispiel 20b und 2,17 g (5,97 mmol) Gadoliniumoxid werden 100 ml Wasser gegeben und 3 Stunden bei 90°C gerührt. Man läßt auf Raumtemperatur abkühlen und rührt anschließend eine Stunde mit 3 ml saurem Kationenaustauscher (IR-120/$H^+$-Form). Der Austauscher wird abfiltriert und das Filtrat gefriergetrocknet.
Ausbeute: 10,18 (97 % d. Th.) eines amorphen Pulvers
Wassergehalt: 11,3 %

| Analyse (bezogen auf wasserfreie Substanz): | | | | |
|---|---|---|---|---|
| ber. | C 41,48 | H 4,90 | Gd 20,11 | N 8,96 |
| gef. | C 41,27 | H 4,98 | Gd 19,93 | N 8,85 |

d) Gadolinium-Komplex des 10-{3-[4-(2,6-dioxomorpholino)-phenoxy]-2-hydroxypropyl}-1,4,7,-tris(carboxymethyl)-1,4,7,10-tetraazacyclododecans

10 g (12,79 mmol) der Titelverbindung aus Beispiel 20c werden in 50 ml Dimethylformamid gelöst und bei 0°C 5,28 g (25,58 mmol) Dicyclohexylcarbodiimid zugegeben. Man rührt über Nacht bei Raumtemperatur. Es wird auf 0°C abgekühlt und vom ausgefallenen Harnstoff abfiltriert. Zum Filtrat tropft man unter Rühren 500 ml Ether zu, wobei die Titelverbindung auskristallisiert. Man filtriert ab und wäscht mit Ether nach. Nach Trocknen über Nacht im Vakuum (50°C) erhält man 9,67 g (69 % d. Th.) der Titelverbindung als leicht gelblichen amorphen Feststoff.
Wassergehalt: 3,0 %

| Elementaranalyse (bezogen auf wasserfreie Substanz): | | | | |
|---|---|---|---|---|
| ber. | C 42,46 | H 4,75 | Gd 20,59 | N 9,17 |
| gef. | C 42,27 | H 4,91 | Gd 20,38 | N 9,03 |

e) 64-Amid-Konjugat aus dem Gadolinium-Komplex des 10-{3-[4-(2,6-dioxomorpholino)-phenoxy]-2-hydroxypropyl}-1,4,7-tris(carboxymethyl)-1,4,7,10-tetraazacyclododecans mit dem deblockierten 64-Polyamin aus Beispiel 17h

1,74 g (0,1 mmol) des in Beispiel 17h beschriebenen 64-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 64-Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden portionsweise 14,68 g (19,2 mmol) des im vorstehenden Beispiel 20d beschriebenen Anhydrids in fester Form zugegeben, wobei der pH durch weitere Zugabe von Natronlauge bei 9,5 konstant gehalten wird. Nach Beendigung der Zugabe wird über Nacht bei Raumtemperatur gerührt und anschließend mit verd. Salzsäure neutralisiert. Die Lösung wird ultrafiltriert (Amicon YM3) und das Retentat gefriergetrocknet.
Ausbeute: 5,00 g (78 % d. Th.)
$H_2O$-Gehalt (Karl-Fischer): 7,7 %
Cd-Bestimmung (AAS): 15,7 %

| Elementaranalyse (berechnet auf wasserfreie Substanz): | | | | | |
|---|---|---|---|---|---|
| ber. | C 43,15 | H 4,93 | Gd 17,01 | N 10,89 | Na 2,49 |
| gef. | C 43,17 | H 4,89 | Gd 16,80 | N 10,54 | Na 2,33 |

**Beispiel 21**

a) meso-2,3-Bis(benzyloxycarbonylamino)bernsteinsäure

14,81 g (100 mmol) Diaminobernsteinsäure (meso-Form) werden in 300 ml Tetrahydrofuran suspendiert und mit 2 N Natronlauge auf pH 9 gestellt. Bei 0°C tropft man unter starkem Rühren 42,65 g (250 mmol) Chlorameisensäurebenzylester, gelöst in 50 ml Tetrahydrofuran zu und hält unter gleichzeitiger Zugabe von 2 N Natronlauge den pH-Wert bei 9. Anschließend läßt man 5 Stunden bei pH 9 und Raumtemperatur nachrühren. Man stellt mit 10 %iger Salzsäure auf pH 2 und gibt 300 ml gesättigte Kochsalzlösung zu. Die organische Phase wird abgetrennt, im Vakuum zur Trockne eingedampft und der Rückstand an Kieselgel chromatographiert (Laufmittel: Methylenchlorid/Methanol 10:1 (2 % Essigsäure).
Ausbeute: 27,07 g (65 % d. Th.) eines farblosen Feststoffs

| Elementaranalyse: | | | |
|---|---|---|---|
| ber. | C 57,69 | H 4,84 | N 6,73 |
| gef. | C 57,59 | H 4,93 | N 6,66 |

b) 2,3 Bis(benzyloxycarbonylamino)-bernsteinsäureanhydrid

25 g (60,04 mmol) der Titelverbindung aus Beispiel 21a werden in 200 ml Acetanhydrid 12 Stunden bei 50°C gerührt. Man kühlt im Eisbad auf 0°C und tropft langsam 600 ml Diethyletherzu. Man filtriert die Kristalle ab, wäscht 2 mal mit je 100 ml Ether nach und trocknet über Nacht im Vakuum (50°C).
Ausbeute: 23,44 g (98 % d. Th.) farblose Blättchen

| Elementaranalyse: | | | |
|---|---|---|---|
| ber. | C 60,30 | H 4,55 | N 7,03 |
| gef. | C 60,15 | H 4,65 | N 6,94 |

c) Vollgeschütztes Benzyloxycarbonyl-32-Amin auf der Basis des (diamino)-succinylierten N,N',N'',N'''-Tetrakis[trilysylaminoethyl]-methans

10 g (2,62 mmol) der Titelverbindung aus Beispiel 17d werden in 100 ml Eisessig gelöst und unter Rühren 33 % Bromwasserstoff in Eisessig zugegeben (100 ml). Nach 2 Stunden wird die begonnene Fällung mit Diethylether vervollständigt, das entstandene Hexadeca-Bromid abfiltriert und mit Diethylether gewaschen und ohne weitere Aufreinigung in die weitere Reaktion eingesetzt (quantitative Ausbeute). Das so erhaltene Hydrobromid wird in 200 ml

Pyridin und 20 ml Triethylamin gelöst und 1 g (8,18 mmol) 4-Dimethylaminopyridin zugegeben. Bei 0°C tropft man 33,40 g (83,84 mmol) der Titelverbindung 21b gelöst in 100 ml Dimethylformamid hinzu und rührt anschließend über Nacht bei Raumtemperatur. Man dampft im Vakuum zur Trockne ein, nimmt den Rückstand in 300 ml Methylenchlorid auf und wäscht 3 mal mit je 100 ml 5 %iger Kaliumcarbonat-Lösung. Die organische Phase wird über Magnesiumsulfat getrocknet und im Vakuum eingeengt. Der Rückstand wird an Kieselgel chromatographiert (Laufmittel: Methylenchlorid/Methanol 20:1)
Ausbeute: 17,07 g (81 % d. Th.) eines farblosen Feststoffs

| Elementaranalyse: | | | |
|---|---|---|---|
| ber. | C 59,28 | H 5,61 | N 10,45 |
| gef. | C 59,13 | H 5,79 | N 10,29 |

d) 1-Hydroxy-11-(4-nitrophenoxy)-3,6,9-trioxaundecan

Zu 100 g (515,0 mmol) Tetraethylenglykol, 73 g (1300 mmol) feingepulverten Kaliumhydroxid und 500 mg Tetrabutylammoniumhydrogensulfat in 500 ml Toluol gibt man bei 0°C 18,16 g (128,7 mmol) 4-Fluornitrobenzol und rührt über Nacht bei Raumtemperatur. Man setzt 1000 ml gesättigte Kochsalz-Lösung zu, trennt die organische Phase ab und trocknet sie über Magnesiumsulfat. Es wird im Vakuum zur Trockne eingedampft und der Rückstand in Kieselgel chromatographiert (Laufmittel: Methylenchlorid/Isopropanol= 15:1).
Ausbeute: 27,19 g (67 % d. Th.) eines gelblichen Öls, das beim Stehenlassen erstarrt

| Analyse: | | | |
|---|---|---|---|
| ber. | C 53,33 | H 6,71 | N 4,44 |
| gef. | C 53,20 | H 6,85 | N 4,28 |

e) 1-tert-Butoxy-13-(4-nitrophenoxy)-2,5,8,11-tetraoxa-tridecan

Zu 25 g (79,28 mmol) der Titelverbindung aus Beispiel 21d, 8,9 g (158,6 mmol) feingepulvertem Kaliumhydroxid und 200 mg Tetrabutylammoniumhydrogensulfat in 300 ml Toluol gibt man bei 0°C 18,6 g (95,1 mmol) Bromessigsäure-tert.-butylester zu und rührt 3 Stunden bei 0°C. Man setzt 200 ml Wasser zu, trennt die organische Phase ab und trocknet sie über Magnesiumsulfat. Es wird im Vakuum zur Trockne eingedampft und der Rückstand an Kieselgel chromatographiert (Laufmittel: Methylenchlorid/Aceton=10:1).
Ausbeute: 29,6 g (87 % d. Th.) eines gelblichen, zähen Öls

| Analyse: | | | |
|---|---|---|---|
| ber. | C 55,93 | H 7,28 | N 3,26 |
| gef. | C 55,78 | H 7,41 | N 3,15 |

f) 1-Carboxy-13-(4-nitrophenoxy)-2,5,8,11-tetraoxa-tridecan

28 g (65,2 mmol) der Titelverbindung aus Beispiel 21e werden in 200 ml Methylenchlorid gelöst. Bei 0°C tropft man 150 ml Trifluoressigsäure hinzu und läßt auf Raumtemperatur kommen. Nach 1 Stunde dampft man zur Trockne ein. Der Rückstand wird aus wenig Ether/Hexan umkristallisiert.
Ausbeute: 23,4 g (96 % d. Th.) eines leicht gelblichen Feststoffes

| Analyse: | | | |
|---|---|---|---|
| ber. | C 51,47 | H 6,21 | N 3,75 |
| gef. | C 51,38 | H 6,31 | N 3,61 |

g) N-Hydroxysuccinimid-Estervon 1-Carboxy-13-(4-nitrophenoxy)-2,5,8,11-tetraoxatridecan

10 g (26,78 mmol) der Titelverbindung aus Beispiel 21f und 3,7 g (32,14 mmol) N-Hydroxysuccinimid werden in 20 ml Chloroform gelöst und bei 0°C 6,63 g (32,14 mmol) Dicyclohexylcarbodiimid zugesetzt. Man rührt 24 Stunden bei Raumtemperatur. Es wird im Eisbad gekühlt und vom ausgefallenen Harnstoff abfiltriert. Das Filtrat wird im Vakuum

zur Trockne eingedampft und der Rückstand aus wenig Isopropanol umkristallisiert,
Ausbeute: 11,21 g (89 % d. Th.) eines leicht gelblich, kristallinen Feststoffes

| Analyse: | | | |
|---|---|---|---|
| ber. | C 51,06 | H 5,57 | N 5,95 |
| gef. | C 51,17 | H 5,61 | N 5,83 |

h) Gadolinium-Komplex von 10-[18-(4-Nitrophenoxy)-2-hydroxy-5-oxo-4-aza-7,10,13,16-tetraaza-octadecyl]-1,4,7-tris (carboxymethyl)-1,4,7,10-tetraazacyclododecan

Zu 10 g (17,43 mmol) der Titelverbindung aus Beispiel 2c in 100 ml Dimethylformamid/20 ml Triethylamin gibt man bei Raumtemperatur 9,84 g (20,92 mmol) der Titelverbindung aus Beispiel 21g zu und rührt über Nacht. Man dampft im Vakuum zur Trockne ein und chromatographiert den Rückstand an RP-18 (LiChroPrep®/Laufmittel: Wasser/ Tetrahydrofuran-Gradient).
Ausbeute: 14,3 g (83 % d. Th.) eines amorphen Feststoffes
Wassergehalt: 5,8 %

| Analyse (bezogen auf wasserfreie Substanz): | | | | |
|---|---|---|---|---|
| ber. | C 42,66 | H 5,53 | Gd 16,93 | N 9,05 |
| gef. | C 42,51 | H 5,68 | Gd 16,80 | N 9,15 |

i) Gadolinium-Komplex von 10-[18-(4-Aminophenoxy)-2-hydroxy-5-oxo-4-aza-7,10,13,16-tetraoxa-octadecyl]-1,4,7-tris(carboxymethyl)-1,4,7,10-tetraazacyclododecan

14 g (15,07 mmol) der Titelverbindung aus Beispiel 21h werden in 200 ml Methanol gelöst und 4 g Palladium-Katalysator (10 % Pd auf C) zugegeben. Man hydriert 8 Stunden bei Raumtemperatur. Der Katalysator wird abfiltriert und das Filtrat zur Trockne eingedampft.
Ausbeute: 13,41 g (99 % d. Th.) eines cremefarbenen Feststoffes
Wassergehalt: 6,1 %

| Analyse (bezogen auf wasserfreie Substanz): | | | | |
|---|---|---|---|---|
| ber. | C 44,09 | H 5,94 | Gd 17,49 | N 9,35 |
| gef. | C 43,93 | H 6,10 | Gd 17,28 | N 9,22 |

k) Gadolinium-Komplex von 10-[18-(4-isothiocyanatophenoxy)-2-hydroxy-5-oxo-4-aza-7,10,13,16-tetraoxaoctadecyl]-1,4,7-tris(carboxymethyl)-1,4,7,10-tetraazacyclododecan

10 g (11,12 mmol) der Titelverbindung aus Beispiel 21i werden in 100 ml Wasser gelöst und 3,84 g (33,36 mmol) Thiophosgen in 50 ml Chloroform zugegeben. Man rührt 1 Stunde bei 50°C. Man kühlt auf Raumtemperatur, trennt die organische Phase ab und schüttelt die wässrige Phase 2 mal mit 100 ml Chloroform aus. Die wässrige Phase wird zur Trockne eingedampft und der Rückstand in 100 ml Aceton bei Raumtemperatur ausgerührt. Der Feststoff wird abfiltriert und mit Ether gewaschen. Nach Trocknen über Nacht im Vakuum (40°C) erhält man 10,47 g (97 % d. Th.) eines cremefarbenen Feststoffes.
Wassergehalt: 3,0 %

| Analyse (bezogen auf wasserfreie Substanz): | | | | | |
|---|---|---|---|---|---|
| ber. | C 43,39 | H 5,46 | Gd 16,71 | N 8,93 | S 3,41 |
| gef. | C 43,20 | H 5,60 | Gd 16,53 | N 8,75 | S 3,36 |

l) 32-Thio-Harnstoff-Konjugat aus dem Gadolinium-Komplex des 10-[18-(4-Isothiocyanatophenoxy)-2-hydroxy-5-oxo-4-aza-7,10,13,16-tetraoxaoctadecyl]-1,4,7-tris(carboxymethyl)-1,4,7,10-tetraazacyclododecans mit dem deblockierten 32-Amin aus Beispiel 21c

1,61 g (0,2 mmol) des in Beispiel 21c beschriebenen 32-Benzyloxycarbonylamins werden in Eisessig gelöst

und unter Rühren mit 33 prozentigem Bromwasserstoff in Eisessig versetzt. Nach 3 Stunden wird mit Diethylether die begonnene Fällung vervollständigt, das entstandene 32-Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden portionsweise 9,03 g (9,6 mmol) des im vorstehenden Beispiel 21k beschriebenen Isothiocyanats in fester Form zugegeben, wobei der pH durch weitere Zugabe von Natronlauge bei 9,5 konstant gehalten wird. Nach Beendigung der Zugabe wird über Nacht bei Raumtemperatur gerührt und anschließend mit verd. Salzsäure neutralisiert. Die Lösung wird ultraßltriert (Amicon YM3) und das Retentat gefriergetrocknet.
Ausbeute: 6,10 g (86 % d. Th.)
$H_2O$-Gehalt (Karl-Fischer): 3,5 %
Gd-Bestimmung (AAS): 14,0 %

| Elementaranalyse (berechnet auf wasserfreie Substanz): | | | | | | |
|---|---|---|---|---|---|---|
| ber. | C 43,14 | H 5,51 | Gd 14,70 | N 10,31 | Na 1,07 | S 3,00 |
| gef. | C 42,96 | H 5,72 | Gd 14,50 | N 10,39 | Na 0,94 | S 2,76 |

**Beispiel 22**

32-Thio-Harnstoff-Konjugat aus dem Gadolinium-Komplex des 10-[3-(4-Isothiocyanatophenoxy)-2-hydroxypropyl]-1,4,7-tris(carboxymethyl)-1,4,7,10-tetraazacyclododecans mit dem deblockierten 32-Amin aus Beispiel 21c
1,61 g (0,2 mmol) des in Beispiel 21c beschriebenen 32-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 32-Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden portionsweise 6,80 g (9,6 mmol) des in Beispiel 12d der EP-485045 (8.11.90) beschriebenen Isothiocyanats in fester Form zugegeben, wobei der pH durch weitere Zugabe von Natronlauge bei 9,5 konstant gehalten wird. Nach Beendigung der Zugabe wird über Nacht bei Raumtemperatur gerührt und anschließend mit verd. Salzsäure neutralisiert. Die Lösung wird ultrafiltriert (Amicon YM3) und das Retentat gefriergetrocknet.
Ausbeute: 4,56 g (82 % d. Th.)
$H_2O$-Gehalt (Karl-Fischer): 4,0 %
Gd-Bestimmung (AAS): 17.9 %

| Elementaranalyse (berechnet auf wasserfreie Substanz): | | | | | | |
|---|---|---|---|---|---|---|
| ber. | C 40,81 | H 4,76 | Gd 18,81 | N 11,52 | Na 1,37 | S 3,84 |
| gef. | C 40,67 | H 4,98 | Gd 18,60 | N 11,70 | Na 1,21 | S 3,47 |

**Beispiel 23**

a) Hexakis-N-[2-Aminoethyl]-[1,3,5]-triazin-2,4,6-triyltriamin

18,41 g (100 mmol) 2,4,6-Trichlor-s-triazin und 92,99 g (315 mmol) Bis-(2-trifluoracetylaminoethyl)-amin (hergestellt nach US-Patent Nr. 4,415,737) werden in einer Mischung aus 300 ml Wasser und 300 ml Dioxan suspendiert. Unter Rühren wird die Temperatur auf 50°C gesteigert. Durch Zutropfen einer gesättigten Lösung von Kaliumhydrogencarbonat wird der pH bei 7-8 gehalten. Wenn sich der pH-Wert nicht mehr ändert, wird durch Zugabe von 1 N Kalilauge der pH-Wert auf 9 erhöht und die Temperatur für 30 Minuten auf 70°C erhöht. Anschließend engt man im Vakuum auf 1/10 des ursprünglichen Volumens ein, kühlt im Eisbad und trennt den Niederschlag durch Absaugen ab. Der Rückstand wird aus Isopropylalkohol umkristallisiert. Man erhält 29 g der Titelverbindung, Fp. 168-172°C.

| Elementaranalyse (bezogen auf wasserfreie Substanz): | | | |
|---|---|---|---|
| ber. | C 46,85 | H 9,44 | N 43,71 |
| gef. | C 46,62 | H 9,80 | N 43,92 |

b) Vollgeschütztes Benzyloxycarbonyl-24-Polyamin auf der Basis des Hexakis-N-[2-(trilysyl-amino)-ethyl]-[1,3,5]-triazin-2,4,6-triyltriamin

7,0 g (7,5 mmol) des in Beispiel 1c beschriebenen geschützten "Tri-Lysins", 1,2 g (7,5 mmol) 1-Hydroxybenzotriazol und 2,4 g (7,5 mmol) 2-(1H-Benzotriazol-1-yl)-1,1,3,3-tetramethyluronium tetrafluoroborat (TBTU) werden in 100 ml DMF gelöst und 15 Minuten gerührt. Die Lösung wird anschließend mit 5,16 ml (30 mmol) N-Ethyldiisopropylamin und 385 mg (1 mmol) Hexakis-N-[2-Aminoethyl]-[1,3,5]-triazin-2,4,6-triyltriamin (Beispiel 23a) versetzt und über Nacht bei Raumtemperatur gerührt. Nach Beendigung der Reaktion wird im Vakuum eingedampft und der Rückstand mit Ethylacetat/Ethanol (3:1) an Kieselgel chromatographiert.
Ausbeute: 4,02 g (68 % d. Th.)

| Elementaranalyse: | | | |
|---|---|---|---|
| ber. | C 63,98 | H 6,80 | N 11,37 |
| gef. | C 64,15 | H 7,19 | N 11,51 |

c) 24-Gadolinium-DTPA-monoamid auf der Basis des Hexakis-N-[2-(trilysyl-amino)-ethyl]-[1,3,5]-triazin-2,4,6-triyltriamin

1,18 g (0,2 mmol) des in Beispiel 23b beschriebenen Poly-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 24 Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden 5,8 g (14,4 mmol) $N^3$-(2,6-Dioxomorpholinoethyl)-$N^6$-(ethoxycarbonylmethyl)-3,6-diazaoctandisäure (Beispiel 13a der EP 0331616) in fester Form zugegeben, wobei der pH durch weitere Zugabe von Natronlauge bei 9,5 konstant gehalten wird. Nach Beendigung der Zugabe wird zur Verseifung des DTPA-ethylesters mit 5 N Natronlauge ein pH > 13 eingestellt und über Nacht bei Raumtemperatur gerührt. Dann wird mit konz. Salzsäure auf pH 5 eingestellt, mit 2,61 g (7,2 mmol) $Gd_2O_3$ versetzt, 30 Minuten bei 80°C gerührt, nach dem Abkühlen auf pH 7 eingestellt und über eine YM3 AMICON-Ultrafiltrationsmembran entsalzt. Das Retentat wird schließlich membranfiltriert und gefriergetrocknet.
Ausbeute: 3,38 g (88 % d. Th.)
$H_2O$-Gehalt (Karl-Fischer): 7,3 %
Gd-Bestimmung (AAS): 20,1 %

| Elementaranalyse (bezogen auf wasserfreie Substanz): | | | | | |
|---|---|---|---|---|---|
| ber. | C 40,78 | H 4,37 | Gd 19,68 | N 8,77 | Na 2,88 |
| gef. | C 41,03 | H 4,61 | Gd 19,82 | N 8,98 | Na 3,01 |

In analoger Weise erhält man mit $Dy_2O_3$ den Dysprosiumkomplex:

| Elementaranalyse (bezogen auf wasserfreie Substanz): | | | | | |
|---|---|---|---|---|---|
| ber. | C 40,51 | H 4,34 | Dy 20,21 | N 8,71 | Na 2,86 |
| gef. | C 40,39 | H 4,60 | Dy 19,90 | N 8,95 | Na 2,62 |

**Beispiel 24**

a) 1,4,7,10,13,16-Hexakis[Benzyloxycarbonylglycyl]-1,4,7,10,13,16-Hexaazacycloocta-decan

516 mg (2 mmol) 1,4,7,10,13,16-Hexaazacyclooctadecan (Hexacyclen; Fluka) werden mit Toluol azeotrop entwässert. Zu der abgekühlten Lösung von Hexacyclen in Toluol wird bei Raumtemperatur eine Lösung von 3,14 g (15 mmol) Benzyloxycarbonyl-glycin (Fluka) in Tetrahydrofuran (THF) sowie 3,71 g (15 mmol) 2-Ethoxy-1-ethoxycarbonyl-1,2-dihydrochinolin (EEDQ; Fluka) zugegeben und über Nacht gerührt. Nach Beendigung der Reaktion wird das Produkt durch Zugabe von Hexan ausgefällt und der Niederschlag mit Dichlormethan/Hexan/Isopropanol (20:10:1) an Kieselgel chromatographiert.
Ausbeute: 1,83 g (65 %)

| Elementaranalyse: | | | |
|---|---|---|---|
| ber. | C 61,53 | H 6,02 | N 11,96 |
| gef. | C 61,40 | H 5,96 | N 12,08 |

b) 1,4,7,10,13,16-Hexakis[N,N-Bis(benzyloxycarbonylaminoethyl)-glycyl]-1,4,7,10,13,16-hexaazacyclooctadecan

1,41 g(1 mmol) des im vorstehenden Beispiel beschriebenen Hexa-Benzyloxycarbonylamins werden in Eisessig gelöst und unter Rühren mit 33 prozentigem Bromwasserstoff in Eisessig versetzt. Nach 60 Minuten wird mit Diethylether die begonnene Fällung vervollständigt, das entstandene Hexamin-hydrobromid mit Ether gewaschen, im Vakuum getrocknet und ohne weitere Reinigung in die weiter unten beschriebene Reaktion eingesetzt. Ausbeute: 1,09 g (quantitativ)

1,09 g (1 mmol) des so hergestellten Hydrobromids werden in 50 ml Acetonitril suspendiert und 3 ml Triethylamin zugegeben. Dann werden 3,54 g (20 mmol) N-Benzyloxycarbonyl-aziridin ((hergestellt nach J. Chem. Soc. Perkin Trans. 1, 21-26, 1993) zugegeben und 5 Tage unter Stickstoff unter Rückfluß erhitzt. Man dampft zur Trockne ein, nimmt mit 100 ml Methylenchlorid auf und wäscht 2 mal mit je 100 ml 5 prozentiger Kaliumcarbonat-Lösung. Die Methylenchloridphase wird über Magnesiumsulfat getrocknet und eingedampft. Der Rückstand wird an Kieselgel chromatographiert (Laufmittel: Methylenchlorid/Methanol/Triethylamin 20:1:0,1).
Ausbeute: 2,29 g (84 % d. Th.) eines blaßgelben zähen Öls

| Elementaranalyse: | | | |
|---|---|---|---|
| ber. | C 63,42 | H 6,65 | N 12,33 |
| gef. | C 63,29 | H 6,60 | N 12,47 |

c) Vollgeschütztes Benzyloxycarbonyl-24-Polyamin aus dem mit Benzyloxycarbonylaziridin alkylierten 12-Amin 1,4,7,10,13,16-Hexakis[N,N-bis(aminoethyl)glycyl]-1,4,7,10,13,16-hexaazacyclooctadecan

1,36 g (0,5 mmol) der Titelverbindung aus Beispiel 24b werden in 100 ml Methanol gelöst und 3 g Peariman-Katalysator (Palladiumhydroxid auf Aktivkohle; Fluka) zugegeben. Man hydriert 10 Stunden bei 5 bar (Raumtemperatur). Es wird vom Katalysator abfiltriert, das Filtrat zur Trockne eingedampft und ohne weitere Reinigung in die folgende Reaktion eingesetzt.
Ausbeute: 0,56 g (quantitativ)

Zu 0,56 g (0,5 mmol) des so hergestellten freien 12-Amins in 50 ml Acetonitril werden 3,54 g (20 mmol) N-Benzyloxycarbonylaziridin (hergestellt nach J. Chem. Soc., Perkin Trans. 1, 21-26, 1993) zugegeben und 5 Tage unter Stickstoff unter Rückfluß erhitzt. Man dampft zur Trockne ein, nimmt mit 100 ml Methylenchlorid auf und wäscht 2 mal mit je 100 ml 5 prozentiger Kaliumcarbonat-Lösung. Die Methylenchloridphase wird über Magnesiumsulfat getrocknet und eingedampft. Der Rückstand wird an Kieselgel chromatographiert (Laufmittel: Methylenchlorid/Methanol/Triethylamin 20:1:0,1).
Ausbeute: 2,23 g (83 % d. Th.) eines blaßgelben zähen Öls

| Elementaranalyse: | | | |
|---|---|---|---|
| ber. | C 64,41 | H 6,98 | N 12,52 |
| gef. | C 64,20 | H 7,15 | N 12,44 |

d) 24-Gadolinium-DTPA-monoamid auf der Basis des deblockierten 24-Amins aus Beispiel 24c

1,07 g (0,2 mmol) der Titelverbindung aus Beispiel 24c werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 24-Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden 5,8 g (14,4 mmol) $N^3$-(2,6-Dioxomorpholinoethyl)-$N^6$-(ethoxycarbonylmethyl)-3,6-diazaoctandisäure (Beispiel 13a der EP 0331 616) in fester Form zugegeben, wobei der pH durch weitere Zugabe von Natronlauge bei 9,5 konstant gehalten wird. Nach Beendigung der Zugabe wird zur Verseifung des DTPA-ethylesters mit 5 N Natronlauge ein pH > 13 eingestellt und über Nacht bei Raumtemperatur gerührt. Dann wird mit konz. Salzsäure auf pH 5 eingestellt, mit 2,61 g (7,2 mmol) $Gd_2O_3$ versetzt, 30 Minuten bei 80°C gerührt, nach dem Abkühlen auf pH

7 eingestellt und über eine YM3 AMICON-Ultrafiltrationsmembran entsalzt. Das Retentat wird schließlich membranfiltriert und gefriergetrocknet.
Ausbeute: 2,90 g (89 % d. Th.)
$H_2O$-Gehalt (Karl-Fischer): 8,0 %
Gd-Bestimmung (AAS): 23,4 %

| Elementaranalyse (berechnet auf wasserfreie Substanz): | | | | | |
|---|---|---|---|---|---|
| ber. | C 34,61 | H 4,40 | Gd 25,17 | N 11,21 | Na 0,92 |
| gef. | C 34,34 | H 4,50 | Gd 24,93 | N 11,06 | Na 1,10 |

**Beispiel 25**

Beispiel für die Darreichungsform

a) 24-Calcium-DTPA-monoamid auf der Basis des N,N,N',N',N",N"-Hexakis[2-(trilysyl-amino)-ethyl]trimesinsäuretriamids

1,20 g (0,2 mmol) des in Beispiel 1d beschriebenen Poly-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 24-Amin-hydrobromid mit Ether gewaschen und im Vakuum getrocknet.
Der Rückstand wird anschließend in Wasser aufgenommen und durch Zugabe von 1 N Natronlauge auf pH 9,5 eingestellt. In diese Lösung werden 5,8 g (14,4 mmol) $N^3$-(2,6-Dioxomorpholinoethyl)-$N^6$-(ethoxycarbonylmethyl)-3,6-diazaoctandisäure (Beispiel 13a der EP 0331 616) in fester Form zugegeben, wobei der pH durch weitere Zugabe von Natronlauge bei 9,5 konstant gehalten wird. Nach Beendigung der Zugabe wird zur Verseifung des DTPA-ethylesters mit 5 N Natronlauge ein pH > 13 eingestellt und über Nacht bei Raumtemperatur gerührt. Dann wird durch Zugabe von Kationenaustauscher (Amberlite IR 120 ($H^+$-Form) auf pH 4,5 gestellt, vom Austauscher abfiltriert und das Filtrat mit 1,07 g Calciumhydroxid (14,4 mmol) versetzt. Die Lösung wird anschließend mit verdünnter Natronlauge auf pH 7 gestellt und über eine YM3 AMICON-Membran ultrafiltriert, das Retentat wird schließlich membranfiltriert und gefriergetrocknet.
Ausbeute: 2,76 g (95 % d.Th.)
$H_2O$-Gehalt (Karl-Fischer): 7,2 %

| Elementaranalyse (bezogen auf wasserfreie Substanz und berechnet als 1,5 Natriumsalz pro Komplex): | | | | | |
|---|---|---|---|---|---|
| ber. | C 41,42 | H 5,04 | Ca 7,13 | N 12,15 | Na 6,14 |
| gef. | C 41,09 | H 5,33 | Ca 6,97 | N 12,30 | Na 6,52 |

b) Herstellung einer Lösung von dem in Beispiel 1 beschriebenen Gadolinium-Kaskaden-polymer

16,68 g (0,025 mol Gadolinium) der in Beispiel 1e beschriebenen Verbindung werden in 60 ml Wasser pro injectione (p.i.) gelöst. Nach Zugabe von 702 mg (1,25 mmol Calcium) des im vorstehenden Beispiel beschriebenen Ca-Komplexes und 121 mg Trishydroxymethylaminomethan stellt man mit verdünnter Salzsäure auf pH 7,0 und füllt mit Wasser p.i. auf 100 ml auf. Die Lösung wird ultrafiltriert, in Flaschen abgefüllt und hitzesterilisiert.

**Beispiel 26**

a) 4,7,13,16,21,24-Hexakis{8-(Benzyloxycarbonylamino)-6-[2-(benzyloxycarbonylamino-ethyl]-5-oxo-3-oxaoctanoyl} Octaazabicyclo [8.8.8] hexacosan

741 mg (2 mmol) 1,4,7,10,13,16,21,24-Octaazabicyclo[8.8.8.]hexacosan [P.H. Smith et al., J. Org. Chem. (1993), 58, 7939] werden mit Toluol azeotrop entwässert. Zu der abgekühlten Lösung des Bicyclus' in Toluol wird bei Raumtemperatur eine Lösung von 7,32 g (15 mmol) N,N'-Bis(benzyloxycarbonyl)-3-[carboxymethoxyacetyl]-3-azapentan-1,5-diamin (Beispiel 7a) in Tetrahydrofuran (THF) sowie 3,71 g (15 mmol) 2-Ethoxy-1-ethoxycarbonyl-1,2-dihydrochinolin (EEDQ; Fluka) zugegeben und über Nacht gerührt. Nach Beendigung der Reaktion wird das Produkt durch Zugabe von Hexan ausgefällt, vom Lösungsmittel abdekantiert und noch einmal aus THF/Hexan und anschließend aus THF/Toluol umgefällt. Man erhält nach Trocknung im Vakuum 4,53 g (71 % d. Th.) eines blaßgelben Feststoffs.

| Elementaranalyse: | | | |
|---|---|---|---|
| ber. | C 61,04 | H 6,45 | N 11,42 |
| gef. | C 60,89 | H 6,55 | N 11,61 |

b) Vollgeschütztes 48-mer Benzyloxycarbonyl-Polyamin auf der Basis des aus 4,7,13,16,21,24-Hexakis{8-Benzyloxycarbonylamino)-6-[2-(benzyloxycarbonylamino)-ethyl]-5-oxo-3-oxaoctanoyl}octaazabicyclo[8.8.8.]hexacosan mit $N^{\alpha}$, $N^{\varepsilon}$-bis(lysyl)-Lysin("Tri-Lysin") kondensierten 48mer-Amins

3,19 g (1 mmol) des im Beispiel 26a beschriebenen 12-mer-Benzyloxycarbonylamins werden in Eisessig gelöst und unter Rühren mit 33 prozentigem Bromwasserstoff in Eisessig versetzt. Nach 90 Minuten wird mit Diethylether die begonnene Fällung vervollständigt, das entstandene Dodeka-amin-hydrobromid mit Ether gewaschen, im Vakuum getrocknet und ohne weitere Reinigung in die weiter unten beschriebene Reaktion eingesetzt.
Ausbeute: 2,7 g (quantitativ)

14,1 g (5 mmol) des in Beispiel 1c beschriebenen geschützten "Tri-Lysins", 2.25 g (15 mmol) 1-Hydroxybenzotriazol und 4,8 g (15 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Diese Lösung wird anschließend mit 7,74 ml (45 mmol) N-Ethyldiisopropylamin und mit 2,7 g (1 mmol) des oben beschriebenen Dodeka-amin-hydrobromids versetzt und über Nacht bei Raumtemperatur gerührt. Nach Beendigung der Reaktion wird im Vakuum eingedampft und der Rückstand mit Dichlormethan/Methanol (10:1) an Kieselgel chromatographiert.
Ausbeute: 9,4 g (74,4 % d. Th.)

| Elementaranalyse: | | | |
|---|---|---|---|
| ber. | C 63,33 | H 6,80 | N 10,87 |
| gef. | C 63,14 | H 6,72 | N 10,98 |

c) 48mer-Gadolinium-DTPA-monoamid auf der Basis des deblockierten 48mer-Amins aus Beispiel 26b

2,53 g (0,2 mmol) des in Beispiel 26b beschriebenen Poly-Benzyloxycarbonylamins werden in Eisessig gelöst und unter Rühren mit 33 prozentigem Bromwasserstoff in Eisessig versetzt. Nach 3 Stunden wird mit Diethylether die begonnene Fällung vervollständigt, das entstandene 48-mer-Amin-hydrobromid mit Ether gewaschen und im Vakuum getrocknet. Der Rückstand wird anschließend in Wasser aufgenommen und durch Zugabe von

1 N-Natronlauge auf pH 9,5 eingestellt. In diese Lösung werden 11,55 g (28,8 mmol) $N^3$-(2,6-Dioxomorpholinoethyl)-$N^6$-(ethoxycarbonylmethyl)-3,6-diazaoctandisäure (Beispiel 13a der EP 0331 616) in fester Form zugegeben, wobei der pH durch weitere Zugabe von Natronlauge bei 9,5 konstant gehalten wird. Nach Beendigung der Zugabe wird zur Verseifung des DTPA-ethylesters mit 5 N Natronlauge ein pH > 13 eingestellt und über Nacht bei Raumtemperatur gerührt. Dann wird mit konz. Salzsäure auf pH 5 eingestellt, mit 5,22 g (14,4 mmol) $Gd_2O_3$ versetzt, 30 Minuten bei 80°C gerührt, nach dem Abkühlen auf pH 7 eingestellt und über eine YM3 AMICON-Ultrafiltrationsmembran entsalzt. Das Retentat wird schließlich membranfiltriert und gefriergetrocknet.
Ausbeute: 5,90 g (83 % d. Th.)
$H_2O$-Gehalt (Karl-Fischer): 8,3 %
Gd-Bestimmung (AAS): 21,6 %

| Elementaranalyse (berechnet auf wasserfreie Substanz): | | | | | |
|---|---|---|---|---|---|
| ber. | C 35,12 | H 4,27 | Gd 23,14 | N 10,39 | Na 3,24 |
| gef. | C 34,98 | H 4,50 | Gd 22,91 | N 10,54 | Na 3,09 |

**Beispiel 27**

a) 10-(Benzyloxycarbonylmethyl)-1,4,7-tris(tert.-butyloxycarbonylmethyl)-1,4,7,10-tetraazacyclododecan (als Natriumbromid-Komplex)

20 g (38,87 mmol) 1,4,7-tris(tert.-butyloxy-carbonylmethyl (DO3A-tris-tert.-Butylester, hergestellt nach EP 0 299 795, Beispiel 22a) werden in 100 ml Acetonitril gelöst. Dann werden 11,45 g (50 mmol) Bromessigsäurcbenzylester und 10,6 g (100 mmol) Natriumcarbonat zugegeben und 12 Stunden bei 60°C gerührt. Man filtriert von den Salzen ab,

dampft das Filtrat im Vakuum zur Trockne ein und chromatographiert den Rückstand an Kieselgel (Laufmittel: Methylenchlorid/Methanol = 20:1).
Ausbeute: 21,72 g (73 % d. Th.) eines farblosen amorphen Pulvers

| Elementaranalyse: | | | | | |
|---|---|---|---|---|---|
| ber. | C 54,90 | H 7,63 | N 7,32 | Na 3,00 | Br 10,44 |
| gef. | C 54,80 | H 7,72 | N 7,21 | Na 2,89 | Br 10,27 |

b) 10-(Carboxymethyl)-1,4,7-tris(tert.-butyloxycarbonylmethyl)-1,4,7,10-tetraazacyclododecan (als Natriumbromid-Komplex)

20 g (26,12 mmol) der Titelverbindung aus Beispiel 27a werden in 300 ml Isopropanol gelöst und 3 g Palladiumkatalysator (10 % Pd/C) zugegeben. Man hydriert über Nacht bei Raumtemperatur. Man filtriert vom Katalysator ab und dampft das Filtrat zur Trockne ein. Ausbeute: 17,47 g (99 % d. Th.) eines farblosen amorphen Pulvers

| Elementaranalyse: | | | | | |
|---|---|---|---|---|---|
| ber. | C 49,78 | H 7,76 | N 8,29 | Na 4,44 | Br 11,83 |
| gef. | C 49,59 | H 7,59 | N 8,17 | Na 4,40 | Br 11,70 |

c) 24-mer N-(2-DO3Ayl-acetyl)-Kaskadenpolyamid auf der Basis des 24-mer Amins N,N,N',N',N",N"-Hexakis[2-(trilysylamino)-ethyl]-trimesinsäuretriamid

6,0 g (1 mmol) des in Beispiel 1d beschriebenen Poly-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervollständigt, das entstandene 24-Amin-hydrobromid mit Ether gewaschen und im Vakuum getrocknet.

32,43 g (48 mmol) der im vorstehenden Beispiel 27b beschriebenen Säure werden in DMF gelöst, mit 7,35 g (48 mmol) 1-Hydroxybenzotriazol, mit 15,41 g (48 mmol) TBTU (Peboc Limited, UK) und mit 49,3 ml (288 mmol) N-Ethyldiisopropylamin versetzt und 20 Minuten bei Raumtemperatur gerührt. Diese Lösung wird anschließend mit den oben beschriebenen (1 mmol) 24-Aminhydrobromid versetzt und 4 Tage bei Raumtemperatur gerührt. Die Lösung wird im Vakuum eingeengt, das verbleibende Öl im Eisbad gekühlt und mit Trifluoressigsäure versetzt, über Nacht bei Raumtemperatur gerührt und anschließend mit Diethylether gefällt. Der Niederschlag wird im Vakuum getrocknet, in Wasser aufgenommen, auf pH 7 eingestellt, über eine YM3 Amicon®-Ultrafiltrationsmembran von niedermolekularen Anteilen gereinigt und das Retentat schließlich membranfiltriert und gefriergetrocknet.
Ausbeute: 12,5 g (83 % d.Th.)
$H_2O$-Gehalt (Karl-Fischer): 9,7 %

| Elementaranalyse (bezogen auf wasserfreie Substanz): | | | | |
|---|---|---|---|---|
| ber. | C 45,21 | H 5,97 | N 14,49 | Na 12,15 |
| gef. | C 45,04 | H 6,15 | N 14,26 | Na 11,97 |

d) 24-mer-Gd-Komplex des N-(DO3Ayl-acetyl)-Kaskadenpolyamids auf der Basis des 24-mer Amins N,N,N',N',N",N"-Hexakis[2-(trilysylamino)-ethyl]-trimesinsäuretriamid

6,81 g (0,5 mmol) der im vorstehenden Beispiel 27c) beschriebenen Komplexbildnersäure werden in Wasser mit verd. Salzsäure auf pH 3 gestellt, mit 2,17 g (6 mmol) $Gd_2O_3$ versetzt, 30 Minuten bei 80°C gerührt, nach dem Abkühlen auf pH 7 eingestellt und über eine YM3 AMICON®-Ultrafiltrationsmembran entsalzt. Das Retentat wird anschließend zur Abtrennung von nichtneutralen Nebenprodukten über einen IRA 67 ($OH^-$-Form) Anionenaustauscher und über IRC 50 ($H^+$-Form) Kationenaustauscher gegeben und die erhaltene Lösung schließlich membranfiltriert und gefriergetrocknet.
Ausbeute: 5,44 g (63,9 % d. Th.)
$H_2O$-Gehalt (Karl-Fischer): 7,5 %
Gd-Bestimmung (AAS): 21,95 %

| Elementaranalyse (bezogen auf wasserfreie Substanz): | | | | |
|---|---|---|---|---|
| ber. | C 39,13 | H 5,16 | N 12,54 | Gd 23,97 |
| gef. | C 38,90 | H 5,29 | N 12,41 | Gd 23,55 |

**Beispiel 28**

a) 10-(4-carboxy-2-oxo-3-azabutyl)-1,4,7-tris(tert.-butoxycarbonylmethyl)-1,4,7,10-tetra-azacyclododecan

Zu 10 g (14,80 mmol) der Titelverbindung aus Beispiel 27b) in 100 ml Dimethylformamid gibt man 1,73 g (15 mmol) N-Hydroxysuccinimid und kühlt auf 0°C. Dann gibt man 4,13 g (20 mmol) Dicyclohexylcarbodiimid zu und rührt 1 Stunde bei 0°C dann 2 Stunden bei Raumtemperatur. Man kühlt auf 0°C ab und gibt anschließend 5,1 g (50 mmol) Triethylamin und 2,25 g (30 mmol) Glycin zu. Man rührt über Nacht bei Raumtemperatur. Man filtriert vom ausgefallenen Harnstoff ab und dampft das Filtrat im Vakuum zur Trockne ein. Der Rückstand wird mit Wasser aufgenommen und 2 mal mit Methylenchlorid extrahiert. Die organische Phase wird über Magnesiumsulfat getrocknet und im Vakuum eingedampft. Der Rückstand wird an Kieselgel chromatographiert (Laufmittel: Methylenchlorid/Methanol = 15:1).
Ausbeute: 8,20 g (88 % d. Th.) eines farblosen Feststoffes

| Elementaranalyse: | | | |
|---|---|---|---|
| ber. | C 57,21 | H 8,80 | N 11,12 |
| gef. | C 57,10 | H 8,91 | N 11,03 |

b) 24-mer N-(5-DO3Ayl-4-oxo-3-azapentanoyl)-Kaskadenpolyamid auf der Basis des 24-mer Amins N,N,N',N',N",N"-Hexakis[2-(trilysylamino)-ethyl]-trimesinsäuretriamid

6,0 g (1 mmol) des in Beispiel 1d beschriebenen Poly-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervallständigt, das entstandene 24-Amin-hydrobromid mit Ether gewaschen und im Vakuum getrocknet.

30,23 g (48 mmol) der im vorstehenden Beispiel 28a) beschriebenen Säure werden in DMF gelöst, mit 7,35 g (48 mmol) 1-Hydroxybenzotriazol, mit 15,41 g (48 mmol) TBTU (Peboc Limited, UK) und mit 49,3 ml (288 mmol) N-Ethyldiisopropylamin versetzt und 20 Minuten bei Raumtemperatur gerührt. Diese Lösung wird anschließend mit den oben beschriebenen (1 mmol) 24-Aminhydrobromid versetzt und 4 Tage bei Raumtemperatur gerührt. Die Lösung wird im Vakuum eingeengt, das verbleibende Öl im Eisbad gekühlt und mit Trifluoressigsäure versetzt, über Nacht bei Raumtemperatur gerührt und anschließend mit Diethylether gefällt. Der Niederschlag wird im Vakuum getrocknet, in Wasser aufgenommen, auf pH 7 eingestellt, über eine YM3 Amicon® -Ultrafiltrationsmembran von niedermolekularen Anteilen gereinigt und das Retentat schließlich membranfiltriert und gefriergetrocknet.
Ausbeute: 12,9 g (79,2 % d.Th.)
$H_2O$-Gehalt (Karl-Fischer): 7,9 %

| Elementaranalyse (bezogen auf wasserfreie Substanz): | | | | |
|---|---|---|---|---|
| ber. | C 44,93 | H 5,91 | N 15,41 | Na 11,04 |
| gef. | C 44,77 | H 6,15 | N 15,60 | Na 10,86 |

c) 24-mer-Gd-Komplex des N-(5-DO3Ayl-4-oxo-3-azapentanoyl)-Kaskadenpolyamids auf der Basis des 24-mer Amins N,N,N',N',N",N"-Hexakis[2-(trilysylamino)-ethyl]-trimesin-säuretriamid

8,14 g (0,5 mmol) der im vorstehenden Beispiel 28b beschriebenen Komplexbildnersäure werden in Wasser mit verd. Salzsäure auf pH 3 gestellt, mit 2,17 g (6 mmol) $Gd_2O_3$ versetzt, 30 Minuten bei 80°C gerührt, nach dem Abkühlen auf pH 7 eingestellt und über eine YM3 AMICON®-Ultrafiltrationsmembran entsalzt. Das Retentat wird anschließend zur Abtrennung von nichtneutralen Nebenprodukten über einen IRA 67 ($OH^-$-Form) Anionenaustauscher und über IRC 50 ($H^+$-Form) Kationenaustauscher gegeben und die erhaltene Lösung schließlich membranfiltriert und gefriergetrocknet.
Ausbeute: 6,3 g (67,7 % d. Th.)
$H_2O$-Gehalt (Karl-Fischer): 8,0 %

Gd-Bestimmung (AAS): 19,6 %

| Elementaranalyse (bezogen auf wasserfreie Substanz): | | | | |
|---|---|---|---|---|
| ber. | C 39,37 | H 5,18 | N 13,50 | Gd 22,05 |
| gef. | C 39,44 | H 5,02 | N 13,73 | Gd 21,79 |

**Beispiel für einen in-vivo Vergleich mit einem extrazellulären Kontrastmittel**

Die Eignung der im Beispiel 7d beschriebenen Verbindung als blood-pool-agent wird im folgenden Versuch gezeigt.

Als Versuchstiere dienen fünf 300-350 g schwere männliche (Schering-SPF-)Ratten. Vor dem Versuch wird das Abdomen eröffnet, der Darm verlagert und dann durch das hintere Bauchfell hindurch mit einer chirurgischen Nadel die Nierengefäße (arteriell+venös) beider Seiten abgebunden. Anschließend wird die Bauchhöhle wieder verschlossen. Danach werden je Tier 0.3 ml (jeweils 50 mmol/L) der folgenden Kontrastmittel-Lösung intravenös appliziert: Gemisch aus je 1 Teil der Verbindung aus Beispiel 7d, im folgenden Verbindung 1 genannt, und dem Dysprosium-Komplex des 10-(1-Hydroxymethyl-2,3-dihydroxypropyl)-1,4,7-tris(carboxymethyl)-1,4,7,10-tetraazacyclododecans, hergestellt analog der Vorschrift in der Europ. Patentanmeldung EP 448 191, im folgenden Verbindung 2 genannt. Über einen Katheter in der Arteria carotis communis werden Blutproben zu folgenden Zeitpunkten entnommen: 15, 30, 45, 60, 90 sec, 3, 5, 10, 15 min p.i. In den gewonnenen Blutproben werden jeweils parallel die Konzentrationen an Gadolinium (Gd) und Dysprosium (Dy) mittels Atomemissionsspektrometrie (ICP-AES) gemessen. Der im Blutraum verbliebene Anteil der injizierten Kontrastmittel Verbindung 1 (Gd) und Verbindung 2 (Dy, Vergleichssubstanz) kann durch die unterschiedliche Markierung im gleichen Tier verglichen werden. Da eine renale Ausscheidung nicht möglich ist, kann der Abfall der Blutkonzentration nur auf eine Verteilung in den Bluträumen und auf die Diffusion in das interstitielle Gewebe zurückzuführen sein.

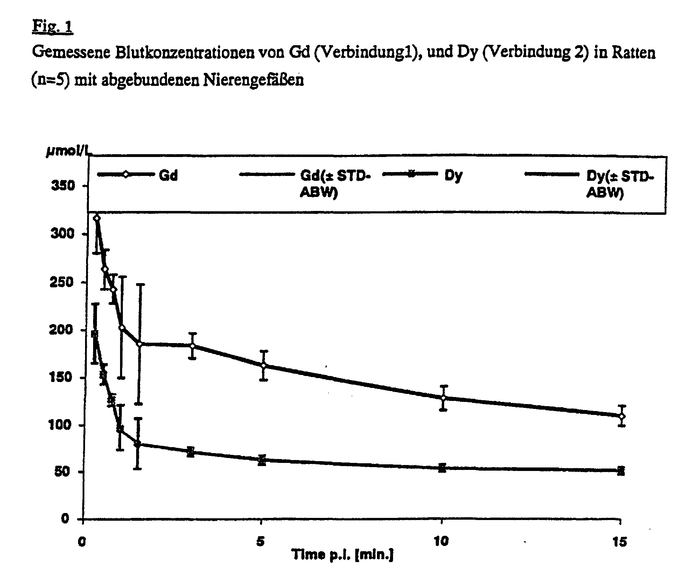

Ergebnisse: Die Diffusion von Verbindung 1 in das Interstitium ist im Vergleich zu einem extrazellulären Kontrastmittel Verbindung 2 deutlich verlangsamt (siehe Figur 1).

Das extrazelluläre Kontrastmittel (Verbindung 2) diffundiert so schnell in die interstitiellen Räume des Körpers, daß bereits nach 1 Minute p.i. ein Equilibrium erreicht wird (angezeigt durch konstanten Blutspiegel). Im Gegensatz dazu werden beim Kaskadenpolymer (Verbindung 1) nicht nur stets höhere Blutkonzentrationen gemessen (Hinweis auf kleineres Verteilungsvolumen), sondern es wird auch über den gesamten Untersuchungszeitraum von 15 Minuten noch kein Equilibrium erreicht (Hinweis auf nur sehr langsam verlaufende Diffusion ins interstitielle Gewebe). Das bedeutet, daß sich Verbindung 1 als Blutpool-Kontrastmittel verhält.

**Patentansprüche**

1. Kaskaden-Polymer-Komplexe enthaltend

a) komplexbildende Liganden der allgemeinen Formel I

$$A\text{-}\{X\text{-}[Y\text{-}(Z\text{-}\langle W\text{-}K_w\rangle_z)_y]_x\}_a \qquad \text{(I)},$$

worin

| | |
|---|---|
| A | für einen stickstoffhaltigen Kaskadenkern der Basismultiplizität a, |
| X und Y | unabhängig voneinander für eine direkte Bindung oder eine Kaskadenreproduktionseinheit der Reproduktionsmultiplizität x bzw. y, |
| Z und W | unabhängig voneinander für eine Kaskadenreproduktionseinheit der Reproduktionsmultiplizität z bzw. w, |
| K | für den Rest eines Komplexbildners, |
| a | für die Ziffern 2 bis 12, |
| x, y, z und w | unabhängig voneinander für die Ziffern 1 bis 4 stehen, |

mit der Maßgabe, daß mindestens zwei Reproduktionseinheiten unterschiedlich sind und daß für das Produkt der Multiplizitäten gilt

16≤a·x·y·z·w≤64,

b) mindestens 16 Ionen eines Elements der Ordnungszahlen 20 bis 29, 39, 42, 44 oder 57-83,

c) gegebenenfalls Kationen anorganischer und/oder organischer Basen, Aminosäuren oder Aminosäureamide sowie

d) gegebenenfalls acylierte terminale Aminogruppen,

**dadurch gekennzeichnet, daß** die Kaskadenreproduktionseinheiten X, Y, Z und W unabhängig voneinander für
E ,

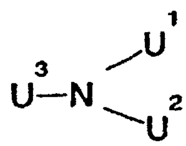

,

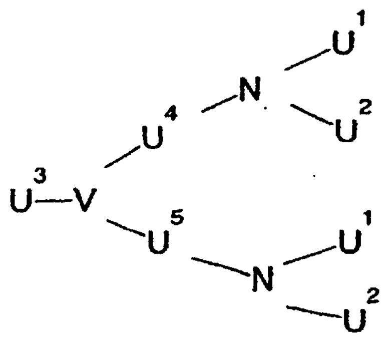

,

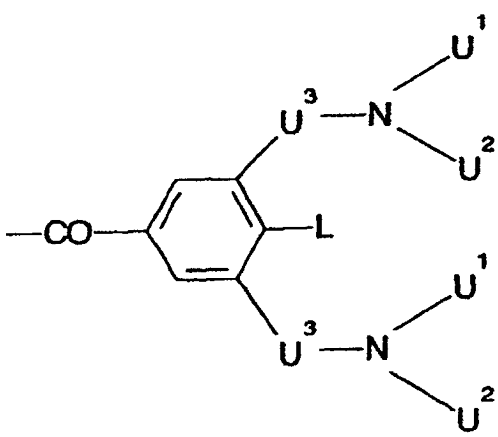

stehen,
worin

$U^1$ für $Q^1$ oder E,
$U^2$ für $Q^2$ oder E mit

E in der Bedeutung der Gruppe

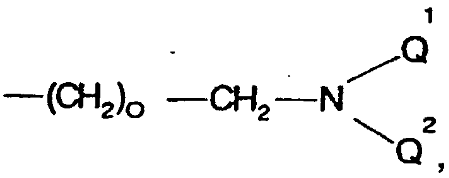

wobei

- o für die Ziffern 1 bis 6,
- $Q^1$ für ein Wasserstoffatom oder $Q^2$ ,
- $Q^2$ für eine direkte Bindung,
- M für eine $C_1$-$C_{10}$-Alkylenkette, die gegebenenfalls durch 1-3 Sauerstoffatome unterbrochen ist und/ oder gegebenenfalls mit 1 bis 2 Oxogruppen substituiert ist,

$U^3$ für eine $C_1$-$C_{20}$-Alkylenkette, die gegebenenfalls durch 1 bis 10 Sauerstoffatome und/oder 1 bis 2 -$N(CO)_q$-$R^2$-, 1 bis 2 Phenylen- und/oder 1 bis 2 Phenylenoxyreste unterbrochen ist und/oder gegebenenfalls durch 1 bis 2 Oxo-, Thioxo-, Carboxy-, $C_1$-$C_5$-Alkylcarboxy-, $C_1$-$C_5$-Alkoxy-, Hydroxy-, $C_1$-$C_5$-alkylgruppen substituiert ist, wobei

- q für die Ziffern 0 oder 1 und
- $R^2$ für ein Wasserstoffatom, einen Methyl- oder einen Ethylrest, der gegebenenfalls mit 1 - 2 Hydroxy- oder 1 Carboxygruppe(n) sustituiert ist,
- L für ein Wasserstoffatom oder die Gruppe

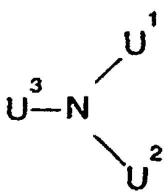

V für die Methingruppe

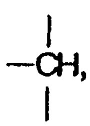

wenn gleichzeitig $U^4$ eine direkte Bindung oder die Gruppe M bedeutet und $U^5$ eine der Bedeutungen von $U^3$ besitzt oder

V für die Gruppe

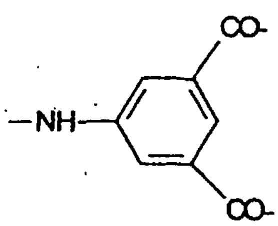

,

wenn gleichzeitig $U^4$ und $U^5$ identisch sind und die direkte Bindung oder die Gruppe M bedeuten,

stehen.

2. Kaskaden-Polymer-Komplexe gemäß Anspruch 1, **dadurch gekennzeichnet, daß** A ein Stickstoffatom,

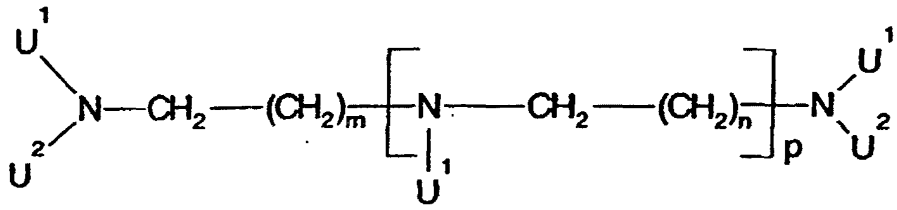

,

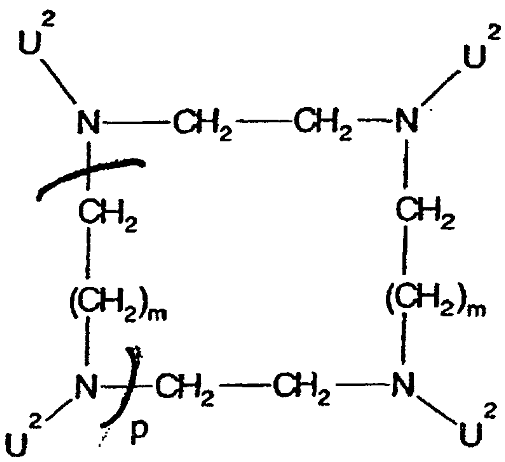

,

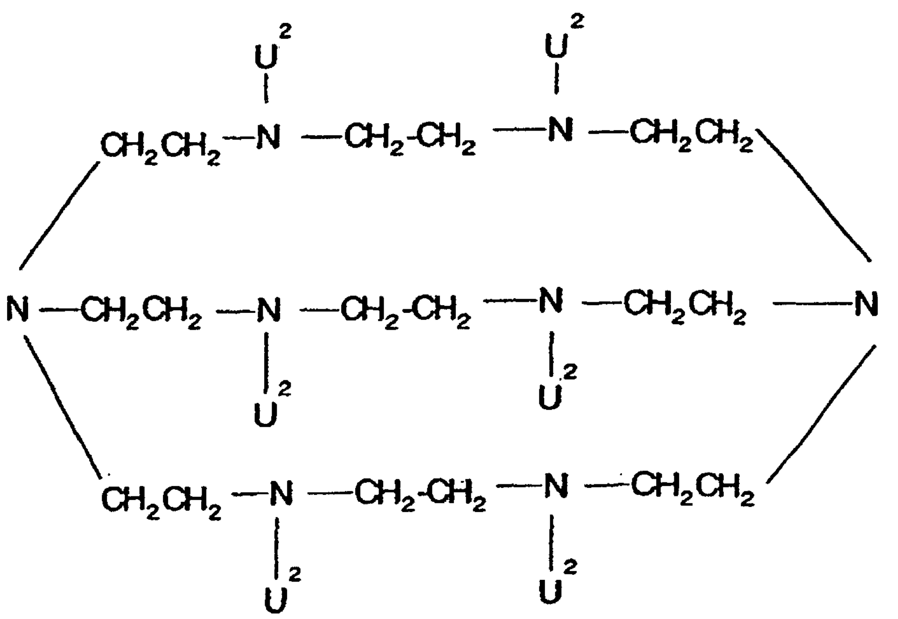

,

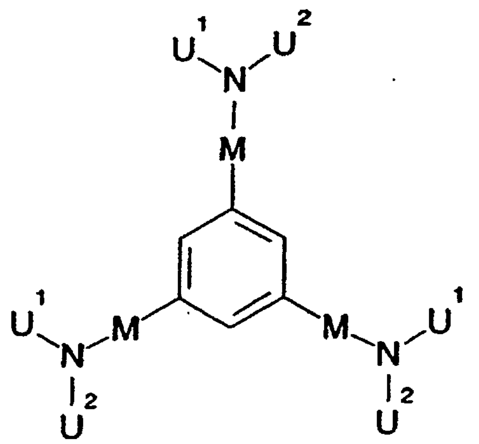

,

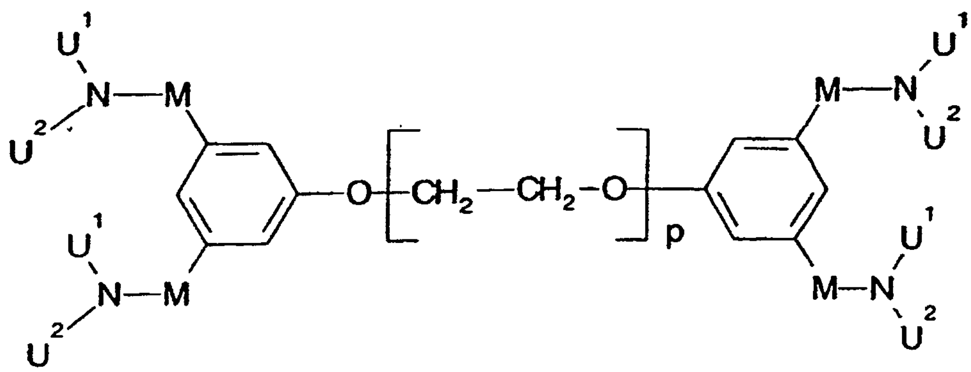

,

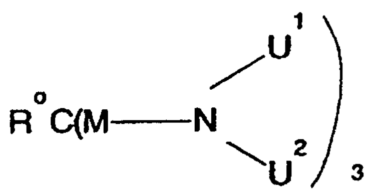

,

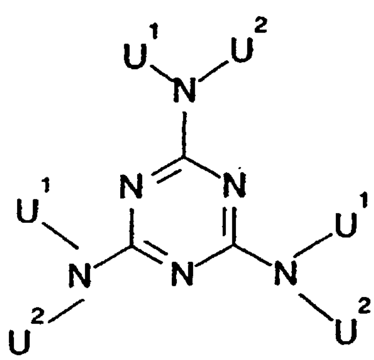

,

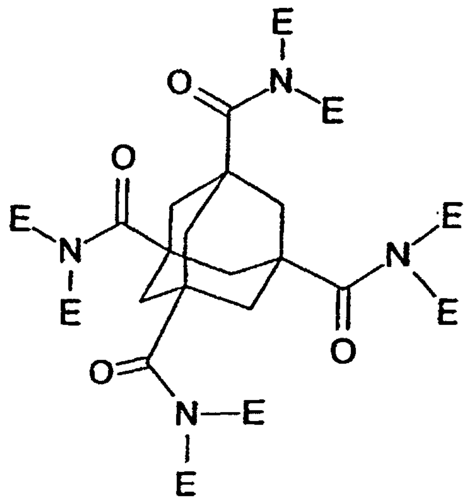

bedeutet, worin

m und n für die Ziffern 1 bis 10,
p für die Ziffern 0 bis 10,
$U^1$ für $Q^1$ oder E,
$U^2$ für $Q^2$ oder E mit

E in der Bedeutung der Gruppe

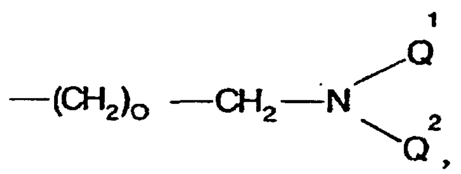

wobei

o für die Ziffern 1 bis 6,
$Q^1$ für ein Wasserstoffatom oder $Q^2$ und
$Q^2$ für eine direkte Bindung

M für eine $C_1$-$C_{10}$-Alkylenkette, die gegebenenfalls durch 1 bis 3 Sauerstoffatome unterbrochen ist und/oder gegebenenfalls mit 1 bis 2 Oxogruppen substituiert ist,
$R^o$ für einen verzweigten oder unverzweigten $C_1$-$C_{10}$-Alkylrest, eine Nitro-, Amino-, Carbonsäuregruppe oder für

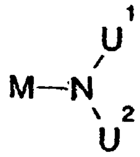

stehen,
wobei die Anzahl $Q^2$ der Basismultiplizität a entspricht.

**3.** Kaskaden-Polymer-Komplexe gemäß Anspruch 1, **dadurch gekennzeichnet, daß** der an die terminalen Stickstoffatome der letzten Generation der Reproduktionseinheit W gebundene Komplexbildner-Rest K für einen Rest der allgemeinen Formeln IA oder IB

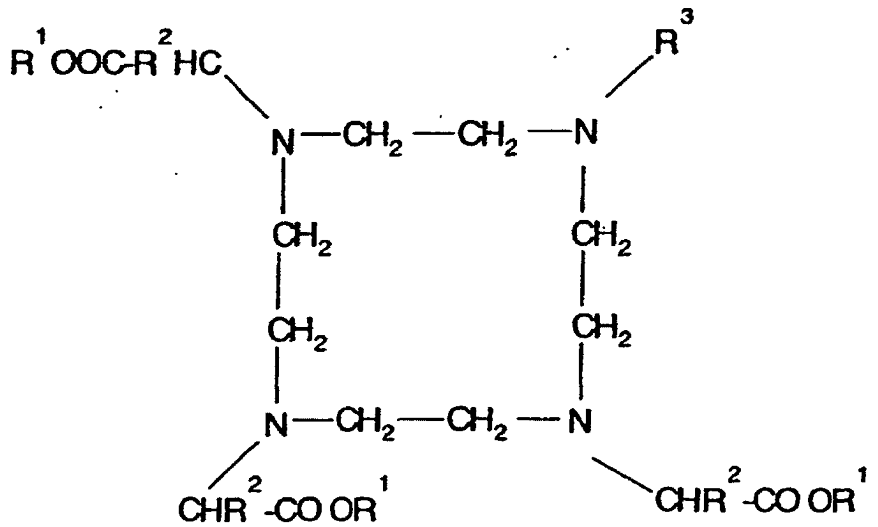

(IA),

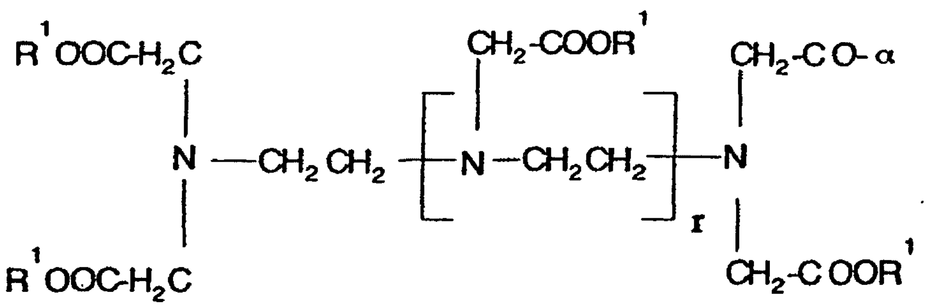

(IB)

steht, worin

- $R^1$ unabhängig voneinander für ein Wasserstoffatom oder ein Metallionenäquivalent der Ordnungszahlen 20 - 29, 39, 42 - 44 oder 57 - 83,
- $R^2$ für ein Wasserstoffatom, einen Methyl- oder einen Ethylrest, der gegebenenfalls mit 1 - 2 Hydroxy- oder 1 Carboxygruppe(n) substituiert ist,
- $R^3$ für eine $-CH_2-CH(OH)-U^6-T-$ oder $-CH_2-CO-U^7$-Gruppe,
- $U^6$ für eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1-Thioharnstoff-, 1 - 2 Carboxyalkylimino-, 1 - 2 Estergruppen, 1 - 10 Sauerstoff-, 1 - 5 Schwefel- und/oder 1 - 5 Stickstoff-Atom(e) enthaltende und/ oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppe(n) substituierte geradkettige, verzweigte, gesättigte oder ungesättigte $C_1$-$C_{20}$-Alkylengruppe, wobei die gegebenenfalls enthaltenden Phenylengruppen durch 1 - 2 Carboxy-, 1 - 2 Sulfon- oder 1 - 2 Hydroxygruppen substituiert sein können,
- $U^7$ für eine direkte Bindung oder den Rest $-NR^2-U^6-T$

T für eine -CO-α, -NHCO-α- oder -NHCS-α-Gruppe,

α für die Bindungsstelle an die terminalen Stickstoffatome der letzten Generation, der Reproduktionseinheit W und

r für die Ziffem 0, 1, 2 oder 3

stehen.

**4.** Kaskaden-Polymer-Komplexe gemäß Anspruch 3 ,**dadurch gekennzeichnet, daß** die für $U^6$ stehende $C_1$-$C_{20}$-Alkylenkette die Gruppen
$-CH_2-$, $-CH_2NHCO-$, $-NHCOCH_2O-$, $-NHCOCH_2OC_6H_4-$, $-N(CH_2CO_2H)-$,
$-NHCOCH_2C_6H_4-$, $-NHCSNHC_6H_4-$, $-CH_2OC_6H_4-$, $-CH_2CH_2O-$,
enthält und/oder durch die Gruppen -COOH, $-CH_2COOH$ substituiert ist.

**5.** Kaskaden-Polymer-Komplexe gemäß Anspruch 3, **dadurch gekennzeichnet, daß** $U^6$ für eine
$-CH_2-$, $-CH_2CH_2-$, $-CH_2CH_2CH_2-$, $-C_6H_4-$, $-C_6H_{10}-$, $-CH_2C_6H_5-$,
$-CH_2NHCOCH_2CH(CH_2CO_2H)-C_6H_4-$,
$-CH_2NHCOCH_2OCH_2-$,
$-CH_2NHCOCH_2C_6H_4-$,

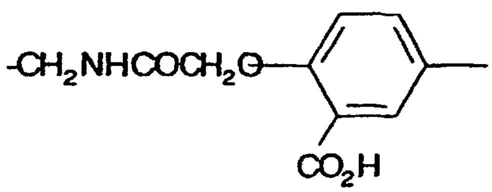

$-CH_2NHCSNH-C_6H_4-CH(CH_2COOH)CH_2-$,
$-CH_2OC_6H_4-N(CH_2COOH)CH_2-$,
$-CH_2NHCOCH_2O(CH_2CH_2O)_4-C_6H_4-$,
$-CH_2O-C_6H_4-$,
$-CH_2CH_2-O-CH_2CH_2-$, $-CH_2CH_2-O-CH_2CH_2-O-CH_2CH_2-$,

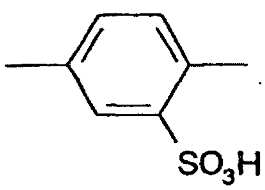

,

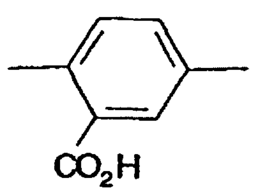

gruppe steht.

**6.** Kaskaden-Polymer-Komplexe gemäß Anspruch 1, **dadurch gekennzeichnet, daß** der in den Kaskadenreproduktionseinheiten X, Y, Z und W enthaltene Rest $U^3$ für
$-CO-$, $-COCH_2OCH_2CO-$, $-COCH_2-$, $-CH_2CH_2-$, $-CONHC_6H_4-$,
$-COCH_2CH_2CO-$, $-COCH_2-CH_2CH_2CO-$, $-COCH_2CH_2CH_2CH_2CO-$,
der Rest $U^4$ für eine direkte Bindung, für $-CH_2CO-$,
der Rest $U^5$ für eine direkte Bindung, für $-(CH_2)_4-$, $-CH_2CO-$, $-CH(COOH)-$,
$CH_2OCH_2CH_2-$, $-CH_2C_6H_4-$, $CH_2-C_6H_4OCH_2CH_2-$,
der Rest E für eine Gruppe

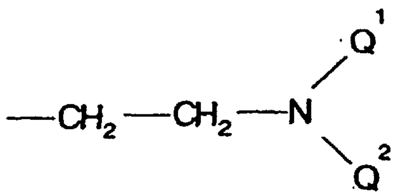

steht.

7. Kaskaden-Polymer-Komplexe gemäß Anspruch1 , **dadurch gekennzeichnet, daß** die Kaskadenreproduktionseinheiten X, Y, Z und W unabhängig voneinander für
$-CH_2CH_2NH-$; $-CH_2CH_2N<$ ;
$-COCH(NH-)(CH_2)_4NH-$; $-COCH(N<)(CH_2)_4N<$ ;
$-COCH_2OCH_2CON(CH_2CH_2NH-)_2$; $-COCH_2OCH_2CON(CH_2CH_2N<)_2$ ;
$-COCH_2N(CH_2CH_2NH-)_2$; $-COCH_2N(CH_2CH_2N<)_2$;
$-COCH_2NH-$; $-COCH_2N<$ ;
$-COCH_2CH_2CON(CH_2CH_2NH-)_2$; $-COCH_2CH_2CON(CH_2CH_2N<)_2$ ;
$-COCH_2OCH_2CONH-C_6H_4-CH[CH_2CON(CH_2CH_2NH-)_2]_2$;
$-COCH_2OCH_2CONH-C_6H_4-CH[CH_2CON(CH_2CH_2N<)_2]_2$;
$-COCH_2CH_2CO-NH-C_6H_4-CH[CH_2CON(CH_2CH_2NH-)_2]_2$;
$-COCH_2CH_2CO-NH-C_6H_4-CH[CH_2CON(CH_2CH_2N<)_2]_2$;
$-CONH-C_6H_4-CH[CH_2CON(CH_2CH_2NH-)_2]_2$;
$-CONH-C_6H_4-CH[CH_2CON(CH_2CH_2N<)_2]_2$;
$-COCH(NH-)CH(COOH)NH-$; $-COCH(N<)CH(COOH)N<$ ;

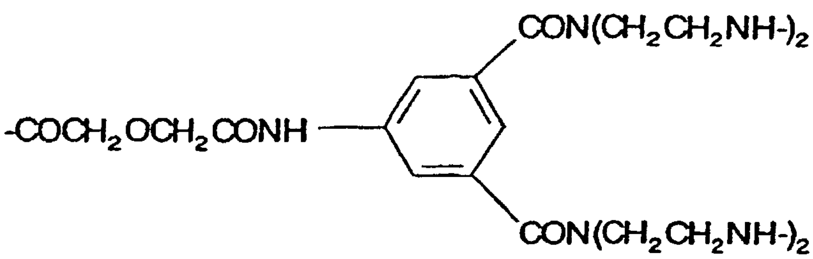

;

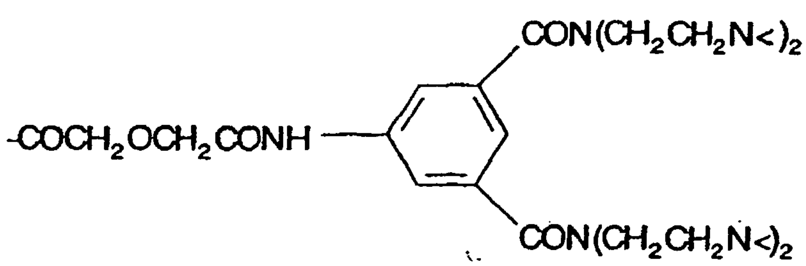

;

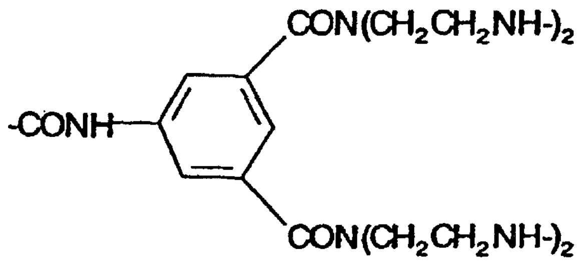

;

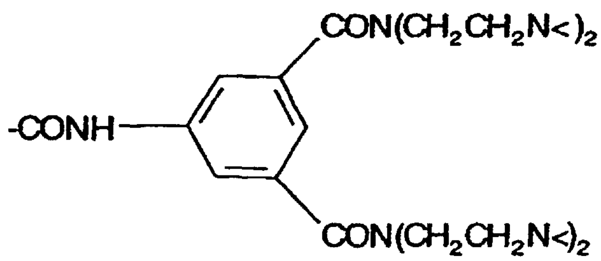

;

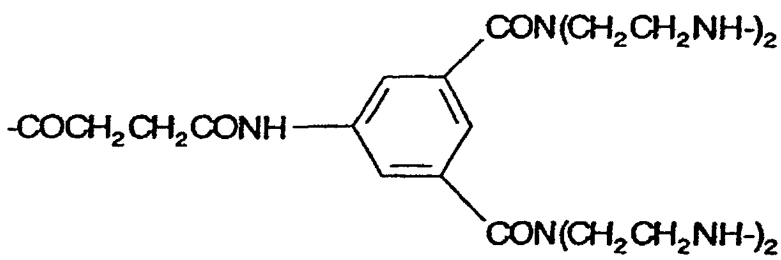

;

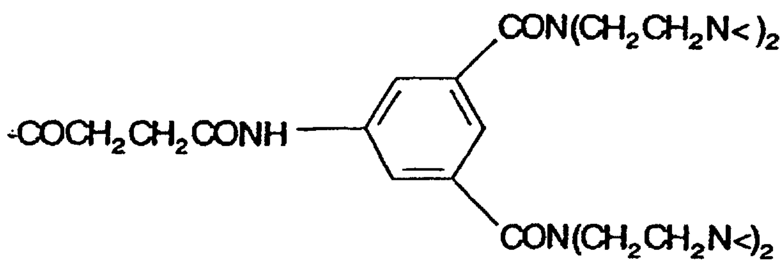

;

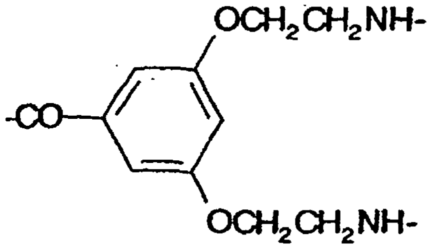

;

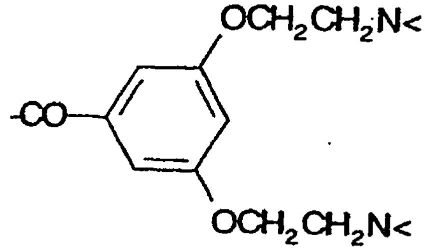

;

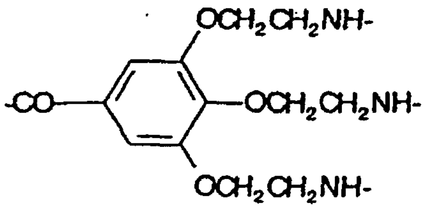 ; 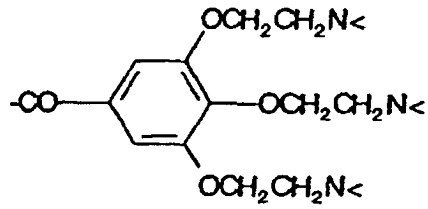 ;

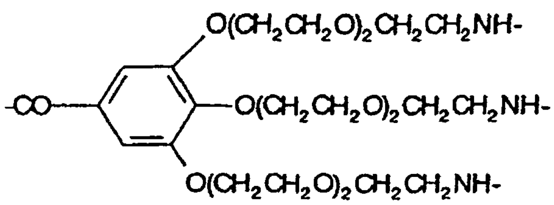 ; 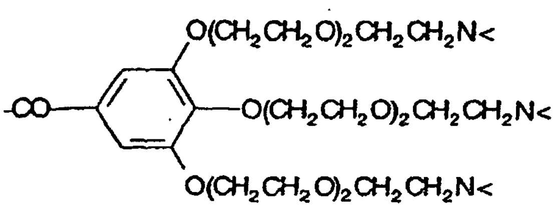 ;

stehen.

**8.** Kaskaden-Polymer-Komplexe gemäß Anspruch 2, **dadurch gekennzeichnet, daß**

| | |
|---|---|
| m | für die Ziffern 1 - 3, |
| n | für die Ziffern 1 - 3, |
| o | für die Ziffer 1, |
| p | für die Ziffern 0 - 3, |
| M | für eine $-CH_2-$, -CO- oder $-CH_2CO$-Gruppe und |
| $R^o$ | für eine $-CH_2NU^1U^2$, $CH_3$- oder $NO_2$-Gruppe |

stehen.

**9.** Pharmazeutische Mittel enthaltend mindestens einen Kaskaden-Polymer-Komplex nach Anspruch 1, gegebenenfalls mit den in der Galenik üblichen Zusätzen.

**10.** Verwendung von mindestens einem Polymer-Komplex nach Anspruch 1 für die Herstellung von Mitteln für die NMR- oder Röntgendiagnostik.

**11.** Verfahren zur Herstellung von Kaskaden-Polymer-Komplexen gemäß den Ansprüchen 1 bis 8', **dadurch gekennzeichnet, daß** man Verbindungen der allgemeinen Formel I'

$$A\text{-}\{X\text{-}[Y\text{-}(Z\text{-}\langle W\text{-}\beta_w\rangle_z)_y]_x\}_a \qquad (I'),$$

worin

| | |
|---|---|
| A | für einen stickstoffhaltigen Kaskadenkern der Basismultiplizität a, |
| X und Y | unabhängig voneinander für eine direkte Bindung oder eine Kaskadenreproduktionseinheit der Reproduktionsmultiplizität x bzw. y, |
| Z und W | unabhängig voneinander für eine Kaskadenreproduktionseinheit der Reproduktionsmultiplizität z bzw. w, |
| a | für die Ziffern 2 bis 12, |
| x, y, z und w | unabhängig voneinander für die Ziffern 1 bis 4 und |
| β | für die Bindungsstelle der terminalen NH-Gruppen der letzten Generation, der Reproduktionseinheit W stehen |

mit der Maßgabe, daß mindestens zwei Reproduktionseinheiten unterschiedlich sind und daß für das Produkt der Multiplizitäten gilt

$$16 \leq a \cdot x \cdot y \cdot z \cdot w \leq 64,$$

mit einem Komplex oder Komplexbildner K' der allgemeinen Formel I'A oder I'B

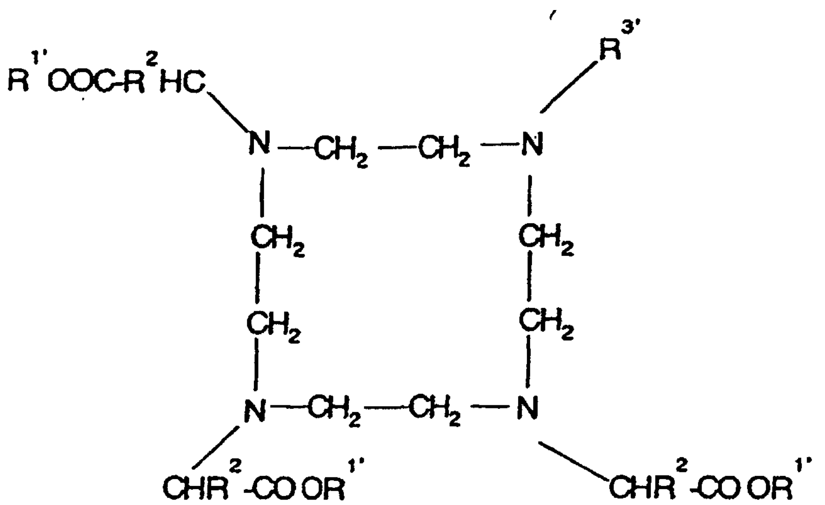

(I'A)

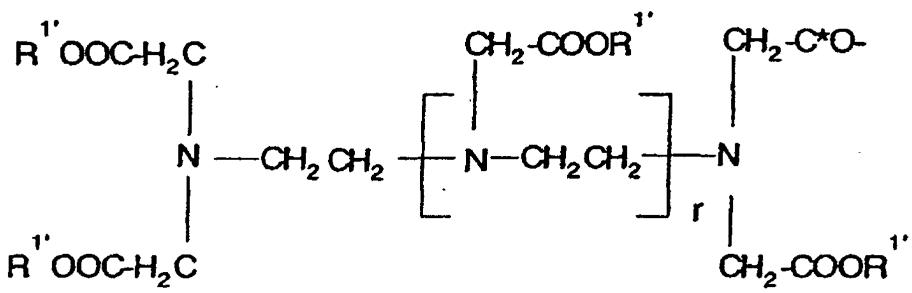

(I'B);

wobei

R1' unabhängig voneinander für ein Wasserstoffatom, ein Metallionenäquivalent der Ordnungszahlen 20 - 29, 39, 42 - 44 oder 57 - 83 oder eine Säureschutzgruppe,

$R^2$ für ein Wasserstoffatom, einen Methyl- oder einen Ethylrest, der gegebenenfalls mit 1 - 2 Hydroxy- oder 1 Carboxygruppe(n) substituiert ist,

$R^{3'}$ für eine $-CH_2-CH(OH)-U^6-T'$ oder $-CH_2-CO-U^{7'}$-Gruppe,

$U^6$ für eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1-Thioharnstoff-, 1 - 2 Carboxyalkylimino-, 1 - 2 Estergruppen, 1 - 10 Sauerstoff-, 1 - 5 Schwefel- und/oder 1 - 5 Stickstoff-Atom(e) enthaltende und/ oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppe(n) substituierte geradkettige, verzweigte, gesättigte oder ungesättigte $C_1$-$C_{20}$-Alkylengruppe, wobei die gegebenenfalls enthaltenden Phenylengruppen durch 1 - 2 Carboxy-, 1 - 2 Sulfon- oder 1 - 2 Hydroxygruppen substituiert sein können,

$U^{7'}$ für eine direkte Bindung oder den Rest $-NR^2-U^6-T'$

T' für eine -C*O-, -COOH-, -N=C=O- oder -N=C=S-Gruppe,

C*O für eine aktivierte Carboxylgruppe und

r für die Ziffern 0, 1, 2 oder 3

stehen

mit der Maßgabe, daß - sofern K' für einen Komplex steht - mindestens zwei (bei zweiwertigen Metallen) bzw. drei (bei dreiwertigen Metallen) der Substituenten $R^1$ für ein Metallionenäquivalent der oben genannten Elemente stehen und daß gewünschtenfalls weitere Carboxyl-gruppen in Form ihrer Salze mit anorganischen und/ oder organischen Basen, Aminosäuren oder Aminosäureamiden vorliegen,

umsetzt, gegebenenfalls vorhandene Schutzgruppen abspaltet, die so erhaltenen Kaskaden-Polymere - sofern K' für einen Komplexbildner steht - in an sich bekannter Weise mit mindestens einem Metalloxid oder Metallsalz eines Elements der Ordnungszahlen 20 - 29, 39, 42, 44 oder 57 - 83 umsetzt und gegebenenfalls anschließend in den so erhaltenen Kaskaden-Polymer-Komplexen noch vorhandene acide Wasserstoffatome ganz oder teilweise durch Kationen von anorganischen und/oder organischen Basen, Aminosäuren oder Aminosäureamiden substituiert und gegebenenfalls noch vorhandene freie terminale Aminogruppen gewünschtenfalls - vor oder nach der Metallkomplexierung - acyliert.

**12.** Verfahren zur Herstellung der pharmazeutischen Mittel gemäß Anspruch 9, **dadurch gekennzeichnet, daß** man den in Wasser oder physiologischer Salzlösung gelösten oder suspendierten Kaskaden-Polymer-Komplex, gegebenenfalls mit den in der Galenik üblichen Zusätzen, in eine für die enterale oder parenterale Applikation geeignete Form bringt.

**Claims**

**1.** A cascade polymer complex containing

a) a complexing ligand of formula I

$$A\text{-}\{X\text{-}[Y\text{-}(Z\text{-}<W\text{-}K_W>_Z)_y]_x\}_a \qquad (I),$$

in which

| | |
|---|---|
| A | stands for a nitrogen-containing cascade nucleus of base multiplicity a, |
| X and Y | independently of one another, stand for a direct bond or a cascade reproduction unit of reproduction multiplicity x or y, |
| Z and W, | independently of one another, stand for a cascade reproduction unit of reproduction multiplicity z or w, |
| K | stands for the radical of a complexing agent, |
| a | stands for a number from 2 to 12, |
| x, y, z and w, | independently of one another, stand for a number 1 to 4, |

provided that at least two reproduction units are different and that

$16 \leq a \cdot x \cdot y \cdot z \cdot w \leq 64$,

holds true for the product of the multiplicities,

b) at least 16 ions of an element of atomic numbers 20 to 29, 39, 42, 44 or 57-83,

c) optionally cations of inorganic and/or organic bases, amino acids or amino acid amides and

d) optionally acylated terminal amino groups,

wherein the cascade reproduction units X, Y, Z and W, independently of one another, stand for E,

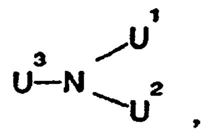

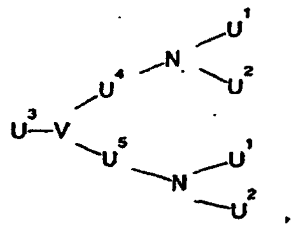

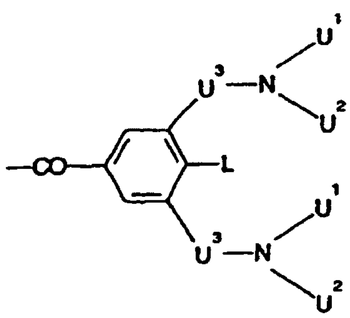

in which

$U^1$ stands for $Q^1$ or E,

$U^2$ stands for $Q^2$ or E with

E meaning the group

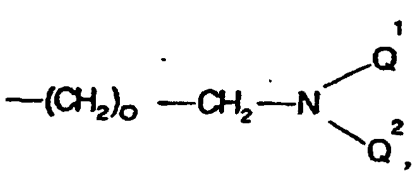

in which

o stands for a number from 1 to 6,

$Q^1$ stands for a hydrogen atom or $Q^2$,

$Q^2$ stands for a direct bond,

M stands for a $C_1$-$C_{10}$ alkylene chain which is optionally interrupted by 1 to 3 oxygen atoms and/or is optionally substituted by 1 or 2 oxo groups,

$U^3$ stands for a $C_1$-$C_{20}$ alkylene chain which optionally is interrupted by 1 to 10 oxygen atoms, and/or 1 to 2 -$N(CO)_q$-$R^2$ radicals, 1 to 2 phenylene radicals and/or 1 to 2 phenylenoxy radicals, and/or optionally is substituted by 1 to 2 oxo, thioxo, carboxy, $C_1$-$C_5$ alkylcarboxy, $C_1$-$C_5$ alkoxy, hydroxy or $C_1$-$C_5$ alkyl groups, in which

q stands for numbers 0 or 1 and

$R^2$ stands for a hydrogen atom, a methyl or an ethyl radical, which optionally is substituted with 1-2 hydroxy or 1 carboxy group(s),

L stands for a hydrogen atom or the group

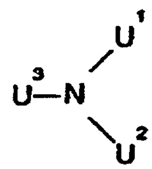

V stands for methine group

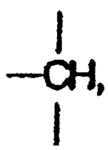

if simultaneously $U^4$ means a direct bond or group M and $U^5$ has one of the meanings of $U^3$ or

V stands for group

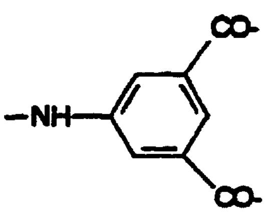

,

if simultaneousy $U^4$ and $U^5$ are identical and mean the direct bond or group M.

**2.** A cascade polymer complex according to claim 1,
wherein A means a nitrogen atom,

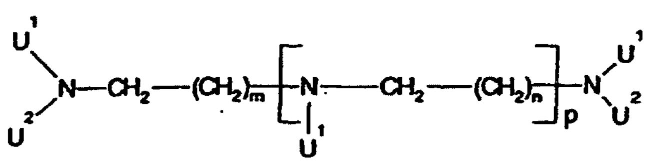

,

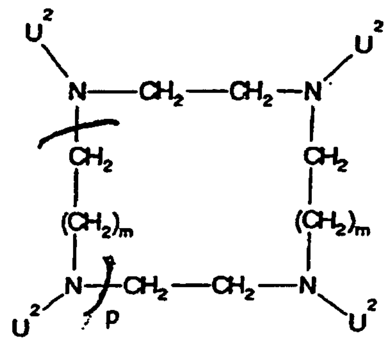

,

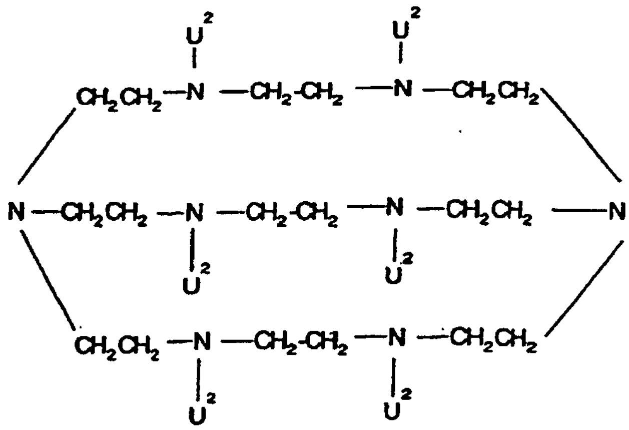

,

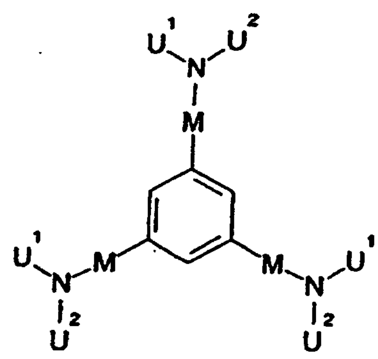

,

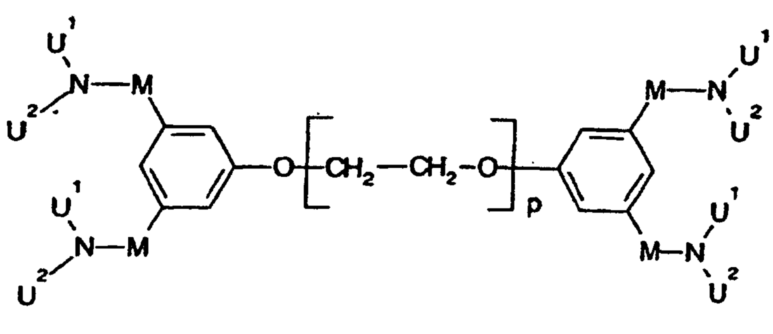

,

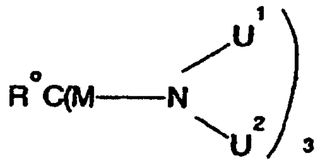

,

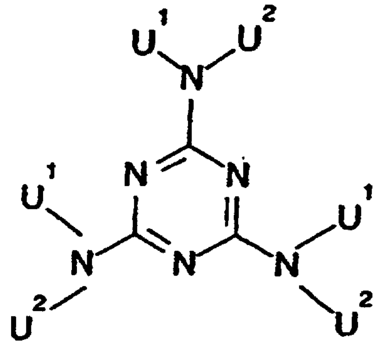

,

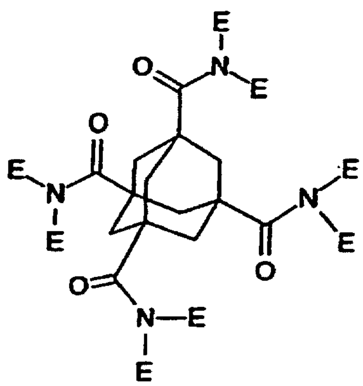

in which

- m and n stand for a number from 1 to 10,
- p stands for a number from 0 to 10,
- $U^1$ stands for $Q^1$ or E,
- $U^2$ stands for $Q^2$ or E with
- E meaning the group

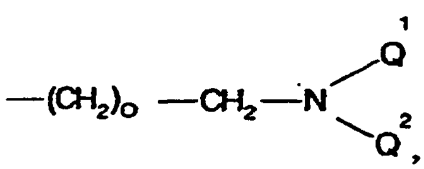

,

in which

o stands for a number from 1 to 6,

$Q^1$ stands for a hydrogen atom or $Q^2$ and

$Q^2$ stands for a direct bond,

M stands for a $C_1$-$C_{10}$ alkylene chain which optionally is interrupted by 1 to 3 oxygen atoms and optionally is substituted with 1 to 2 oxo groups, and

$R^o$ stands for a branched or unbranched $C_1$-$C_{10}$ alkyl radical, a nitro, amino or carboxylic acid group or for

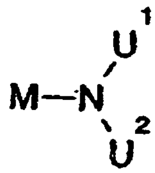

wherein the number of $Q^2$ groups is equal to the base multiplicity a.

**3.** A cascade polymer complex according to claim 1, wherein complexing agent radical K bound to the terminal nitrogen atoms of the last generation of reproduction unit W stands for a radical of formula IA or IB

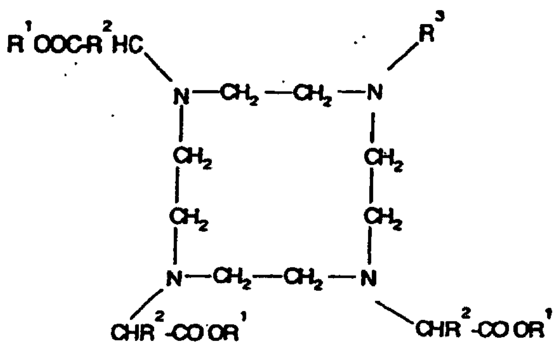

(IA),

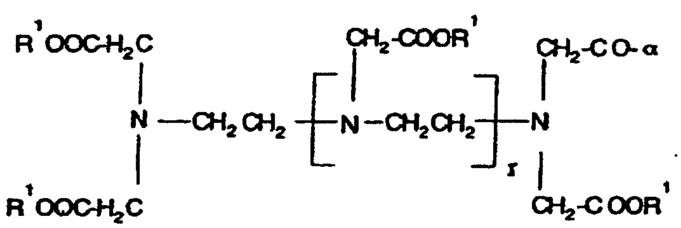

(IB)

in which

$R^1$ independently of one another, stand for a hydrogen atom or a metal ion equivalent of atomic numbers 20-29, 39, 42-44 or 57-83,

$R^2$ stands for a hydrogen atom, a methyl or an ethyl radical which optionally is substituted with 1-2 hydroxy or 1 carboxy group(s),

$R^3$ stands for a-$CH_2$-CH (OH)-$U^6$-T or -$CH_2$-CO-$U^7$ group,

$U^6$ stands for a straight-chain, branched, saturated or unsaturated $C_1$-$C_{20}$ alkylene group optionally containing 1-5 imino, 1-3 phenylene, 1-3 phenylenoxy, 1-3 phenylenimino, 1-5 amide, 1-2 hydrazide, 1-5 carbonyl, 1-5 ethylenoxy, 1 urea, 1 thiourea, 1-2 carboxyalkylimino, 1-2 ester groups, or 1-10 oxygen, 1-5 sulfur or 1-5 nitrogen atom(s); and optionally substituted by 1-5 hydroxy, 1-2 mercapto, 1-5 oxo, 1-5 thioxo, 1-3 carboxy, 1-5 carboxyalkyl, 1-5 ester and/or 1-3 amino group(s), and the phenylene groups that are optionally contained therein can be substituted by 1-2 carboxy, 1-2 sulfone or 1-2 hydroxy groups,

$U^7$ stands for a direct bond or radical -$NR^2$-$U^6$-T,

T stands for a -CO-$\alpha$, -NHCO-$\alpha$ or -NHCS-$\alpha$ group,

$\alpha$ stands for the bonding site to the terminal nitrogen atoms of the last generation, of reproduction unit W and

r stands for numbers 0, 1, 2 or 3.

**4.** A cascade polymer complex according to claim 3, wherein the $C_1$-$C_{20}$ alkylene chain that stands for $U^6$ contains the groups -$CH_2$-, -$CH_2NHCO$-, -$NHCOCH_2O$-, -$NHCOCH_2OC_6H_4$-, -$N(CH_2CO_2H)$-, -$NHCOCH_2C_6H_4$-, -$NHCSNHC_6H_4$-, -$CH_2OC_6H_4$-, or -$CH_2CH_2O$- and/or is substituted by groups -COOH, -$CH_2COOH$.

**5.** A cascade polymer complex according to claim 3, wherein $U^6$ stands for a -$CH_2$-, -$CH_2CH_2$-, -$CH_2CH_2CH_2$-, -$C_6H_4$-, -$C_6H_{10}$-, -$CH_2C_6H_5$-, -$CH_2NHCOCH_2CH(CH_2CO_2H)$-$C_6H_4$-, -$CH_2NHCOCH_2OCH_2$-, -$CH_2NHCOCH_2C_6H_4$-,

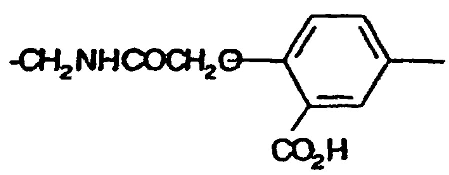

-$CH_2NHCSNH$-$C_6H_4$-$CH(CH_2COOH)CH_2$-, -$CH_2OC_6H_4$-$N(CH_2COOH)CH_2$-, -$CH_2NHCOCH_2O(CH_2CH_2O)_4$-$C_6H_4$-, -$CH_2O$-$C_6H_4$-, -$CH_2CH_2$-O-$CH_2CH_2$-, -$CH_2CH_2$-O-$CH_2CH_2$-O-$CH_2CH_2$-,

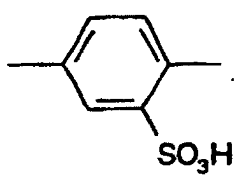

,

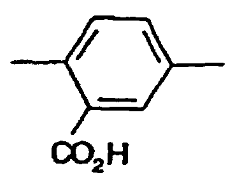

group.

**6.** A cascade polymer complex according to claim 1, wherein radical $U^3$ contained in the cascade reproduction units X, Y, Z and W stands for -CO-, -$COCH_2OCH_2CO$-, -$COCH_2$-, -$CH_2CH_2$-, -$CONHC_6H_4$-, -$COCH_2CH_2CO$-, -$COCH_2$-$CH_2CH_2CO$-, or -$COCH_2CH_2CH_2CH_2CO$-, radical $U^4$ stands for a direct bond, or -$CH_2CO$-, and radical $U^5$ stands for a direct bond, -$(CH_2)_4$-, -$CH_2CO$-, -CH(COOH)-, $CH_2OCH_2CH_2$-, -$CH_2C_6H_4$-, $CH_2$-$C_6H_4OCH_2CH_2$- and the radical E stands for a group

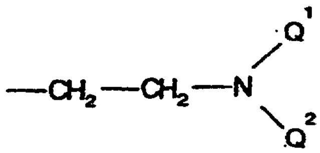

7. A cascade polymer complex according to claim 1, wherein cascade reproduction units X, Y, Z and W, independently of one another, stand for $-CH_2CH_2NH-$; $-CH_2CH_2N<$; $-COCH(NH-)(CH_2)_4NH-$; $-COCH(N<)(CH_2)_4N<$; $-COCH_2OCH_2CON(CH_2CH_2NH-)_2$; $-COCH_2OCH_2CON(CH_2CH_2N<)_2$; $-COCH_2N(CH_2CH_2NH-)_2$; $-COCH_2N(CH_2CH_2N<)_2$; $-COCH_2NH-$; $-COCH_2N<$; $-COCH_2CH_2CON(CH_2CH_2NH-)_2$; $-COCH_2CH_2CON(CH_2CH_2N<)_2$; $-COCH_2OCH_2CONH-C_6H_4-CH[CH_2CON(CH_2CH_2NH-)_2]_2$; $-COCH_2OCH_2CONH-C_6H_4-CH[CH_2CON(CH_2CH_2N<)_2]_2$; $-COCH_2CH_2CO-NH-C_6H_4-CH[CH_2CON(CH_2CH_2NH-)_2]_2$; $-COCH_2CH_2CO-NH-C_6H_4-CH[CH_2CON(CH_2CH_2N<)_2]_2$; $-CONH-C_6H_4-CH[CH_2CON(CH_2CH_2NH-)_2]_2$; $-CONH-C_6H_4-CH[CH_2CON(CH_2CH_2N<)_2]_2$; $-COCH(NR-)CH(COOH)NH-$; $-COCH(N<)CH(COOH)N<$;

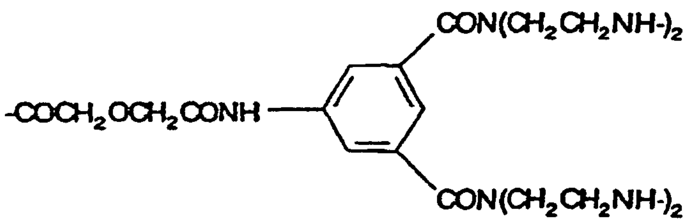

;

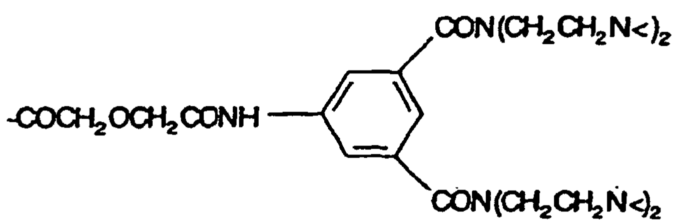

;

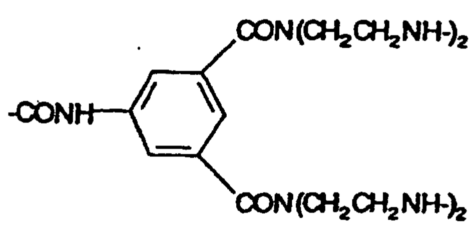

;

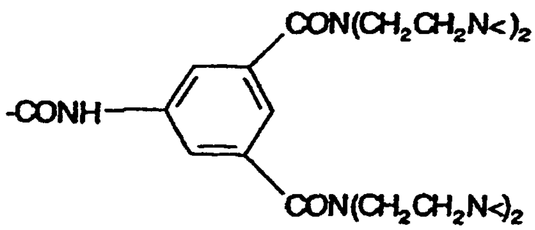

;

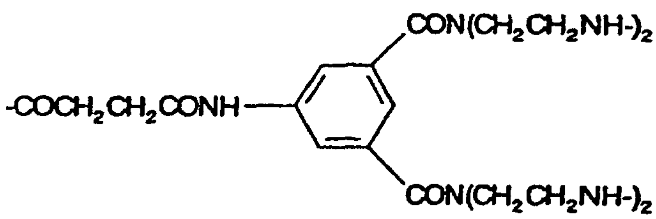

;

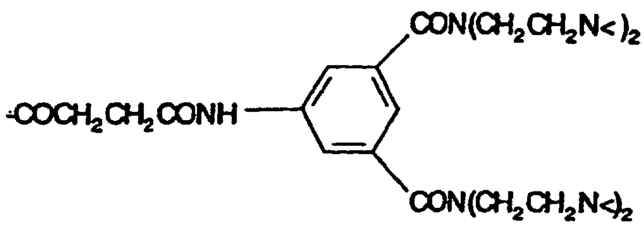

;

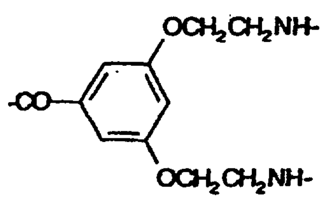

;

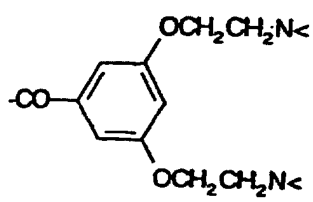

;

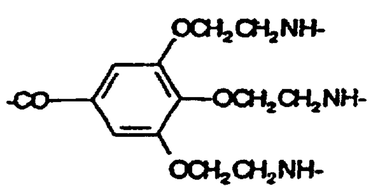

;

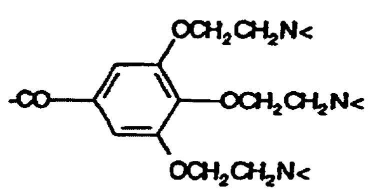

;

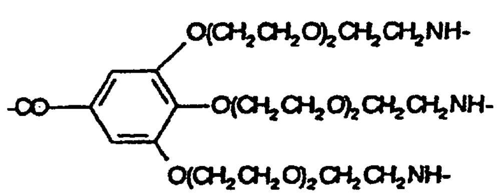

;

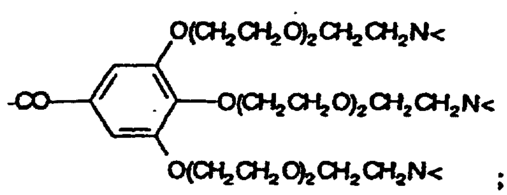

;

**8.** A cascade polymer complex according to claim 2, wherein

m stands for a number from 1-3,

n stands for a number from 1-3,

o stands for 1,

p stands for a number from 0-3,

M stands for a $-CH_2$, -CO or $-CH_2CO$ group and

$R^o$ stands for a $-CH_2NU^1U^2$, $CH_3$ or $NO_2$ group.

**9.** A pharmaceutical agent containing at least one cascade polymer complex according to claim 1, optionally with the additives usual in galenicals.

**10.** Use of at least one polymer complex according to claim 1 for the production of agents for NMR diagnosis or diagnostic radiology.

**11.** A process for the production of a cascade polymer complex according to claims 1 to 8, wherein a compound of general formula I'

$$A\text{-}\{X\text{-}[Y\text{-}(Z\text{-}\leq W\text{-}\beta_w >_z)_y]_x\}_a \qquad (I'),$$

in which

A stands for a nitrogen-containing cascade nucleus of base multiplicity a,

X and Y, independently of one another, stand for a direct bond or a cascade reproduction unit of reproduction multiplicity x or y,

Z and W, independently of one another, stand for a cascade reproduction unit of reproduction multiplicity z or w,

a stands for numbers 2 to 12,

x, y, z and w, independently of one another, stand for numbers 1 to 4, and

β stands for the bonding site of the terminal NH groups of the last generation, of reproduction unit W provided that at least two reproduction units are different and that

$$16 \leq a \cdot x \cdot y \cdot z \cdot w \leq 64$$

holds true for the product of multiplicities, is reacted with a complex or complexing agent K' of general formula I'A or I'B

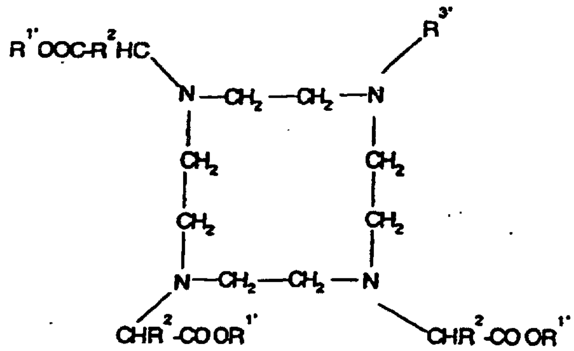

(I'A).

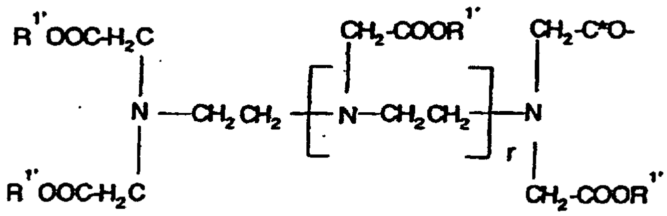

(I'B);

in which

$R^{1'}$, independently of one another, stand for a hydrogen atom, a metal ion equivalent of atomic numbers 20-29, 39, 42-44, or 57-83 or an acid protective group,

$R^2$ stands for a hydrogen atom, a methyl or an ethyl radical which optionally is substituted with 1-2 hydroxy or 1 carboxy group(s),

$R^{3'}$ stands for a $-CH_2-CH(OH)-U^6-T'$ or $-CH_2-CO-U^{7'}$ group,

$U^6$ stands for a straight-chain, branched, saturated or unsaturated $C_1$-$C_{20}$ alkylene group optionally containing 1-5 imino, 1-3 phenylene, 1-3 phenylenoxy, 1-3 phenylenimino, 1-5 amide, 1-2 hydrazide, 1-5 carbonyl, 1-5 ethylenoxy, 1 urea, 1 thiourea, 1-2 carboxyalkylimino, 1-2 ester groups; 1-10 oxygen, 1-5 sulfur and/or 1-5 nitrogen atom(s) and optionally substituted by 1-5 hydroxy, 1-2 mercapto, 1-5 oxo, 1-5 thioxo, 1-3 carboxy, 1-5 carboxyalkyl, 1-5 ester and/or 1-3 amino group(s), and the phenylene groups that are optionally contained therein can be substituted by 1-2 carboxy, 1-2 sulfone or 1-2 hydroxy groups,

$U^{7'}$ stands for a direct bond or radical $-NR^2-U^6-T'$,

T' stands for a -C*O, -COOH, -N=C=O or -N=C=S group,

C*O stands for an activated carboxyl group and

r stands for numbers 0, 1, 2 or 3 provided that if K' stands for a complex at least two, in the case of divalent metals, or three, in the case of trivalent metals, of substituents R' stand for a metal ion equivalent of the above-mentioned elements and that, optionally, other carboxyl groups are present in the form of their salts with inorganic bases and/or organic bases, amino acids or amino acid amides, any optionally present protective groups are cleared, the thus obtained cascade polymers-if K' stands for a complexing agent-are reacted with at least one metal oxide or metal salt of an element of atomic numbers 20-29, 39, 42, 44, or 57-83 and optionally then in the cascade polymer complexes thus obtained, acid hydrogen atoms that are still present are completely or partially substituted by cations of inorganic bases, organic bases, amino acids, or amino acid amides and optionally free terminal amino groups that are still present are acylated if desired - before or after the metal complexing.

**12.** A process for the production of a pharmaceutical agent according to claim 9, wherein the cascade polymer complex, dissolved or suspended in water or physiological salt solution, optionally with the additives usual in galenicals, is

brought into a form suitable for enteral or parenteral administration.

## Revendications

**1.** Complexes de polymères en cascade contenant

a) des ligands complexants de formule générale I

$$A\text{-}\{X\text{-}[Y\text{-}(Z\text{-}\langle W\text{-}K_w\rangle z)_y]_x\}_a \qquad (I),$$

dans laquelle

| | |
|---|---|
| A | représente un noyau de cascade azoté de multiplicité de base a, |
| X et Y | représentent, indépendamment l'un de l'autre, une liaison directe ou une unité de reproduction en cascade, de multiplicité de reproduction x ou y, |
| Z et W | représentent, indépendamment l'un de l'autre, une unité de reproduction en cascade de multiplicité de reproduction z ou w, |
| K | représente le reste d'un complexant, |
| a | représente les nombres 2 à 12, |
| x, y, z et w | représentent, indépendamment les uns des autres, les nombres 1 à 4, |

étant entendu qu'au moins 2 unités de reproduction sont différentes et que pour le produit des multiplicités, on a :

$16 \leq a.x.y.z.w \leq 64$,

b) au moins 16 ions d'un élément des nombres atomiques 20 à 29, 39, 42, 44 ou 57-83,

c) éventuellement les cations de bases minérales et/ou de bases organiques, d'aminoacides ou d'amides d'aminoacides, ainsi que

d) éventuellement des groupes amino terminaux acylés,

**caractérisés en ce que** les unités de reproduction en cascade X, Y, Z et W représentent, indépendamment les unes des autres,

E,

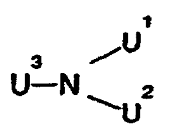

,

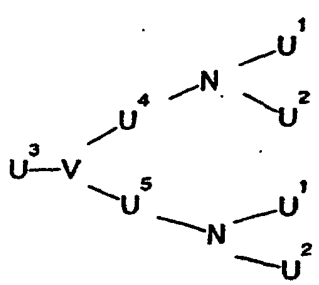

,

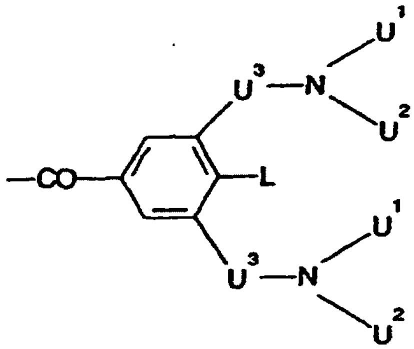

où

$U^1$ représente $Q^1$ ou E,
$U^2$ représente $Q^2$ ou E,
E représentant le groupe

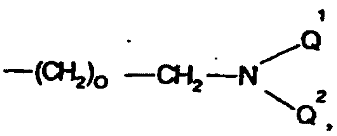

où

o représente les nombres 1 à 6,
$Q^1$ représente un atome d'hydrogène ou $Q^2$,
$Q^2$ représente une liaison directe,
M représente une chaîne alkylène en $C_1$-$C_{10}$ qui est éventuellement interrompue par 1-3 atomes d'oxygène et/ou est éventuellement substituée par 1 ou 2 groupes oxo,
$U^3$ représente une chaîne alkylène en $C_1$-$C_{20}$ qui est éventuellement interrompue par 1 à 10 atomes d'oxygène et/ou 1 ou 2 radicaux $-N(CO)_q-R^2-$, 1 ou 2 radicaux phénylène et/ou 1 ou 2 radicaux phénylène-oxy et/ou est éventuellement substituée par 1 ou 2 groupes oxo, thioxo, carboxy, alkyl($C_1$-$C_5$)carboxy, alcoxy($C_1$-$C_5$) hydroxy, alkyle en $C_1$-$C_5$,

q représentant le nombre 0 ou 1 et
$R^2$ représentant un atome d'hydrogène, un radical méthyle ou éthyle, qui est éventuellement substitué par 1 ou 2 groupes hydroxy ou un groupe carboxy,

L représente un atome d'hydrogène ou le groupe

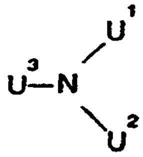

V représente le groupe méthine

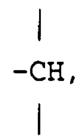

V si en même temps $U^4$ représente une liaison directe ou le groupe M, et $U^5$ a l'une des significations de $U^3$, représente le groupe

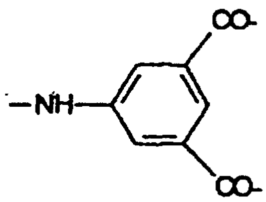

,

si en même temps $U^4$ et $U^5$ sont identiques et représentent une liaison directe ou le groupe M.

**2.** Complexes de polymères en cascade selon la revendication 1, **caractérisés en ce que** A représente un atome d'azote,

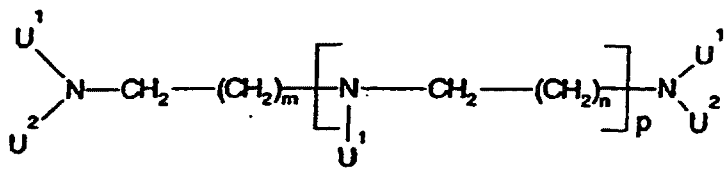

,

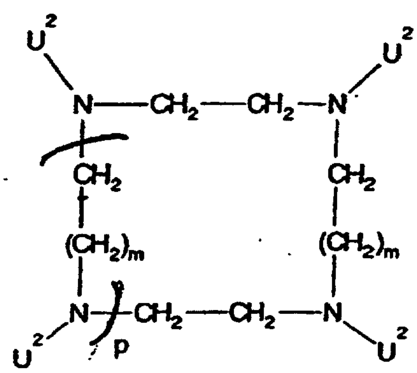

,

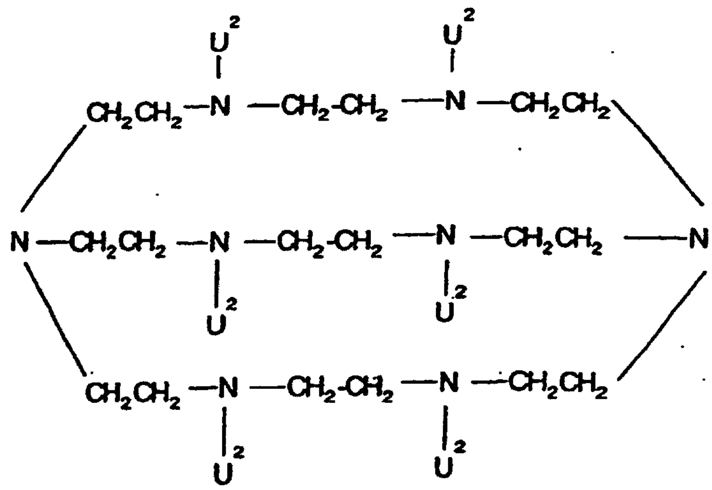

,

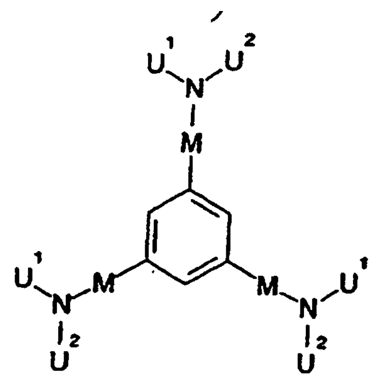

,

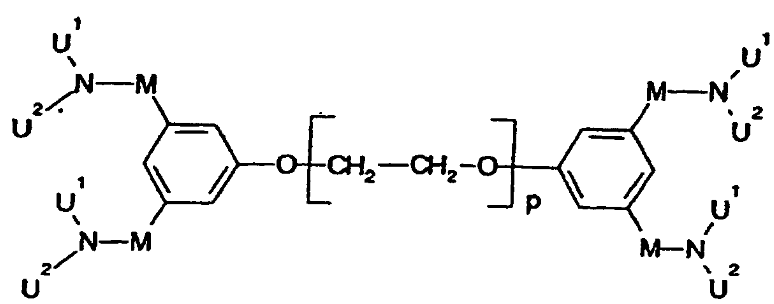

,

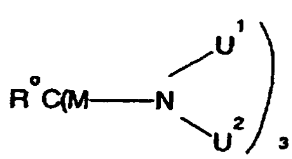

,

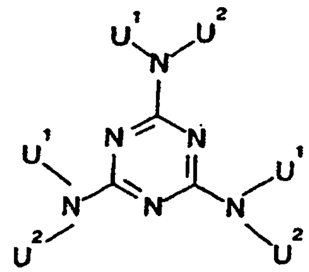

,

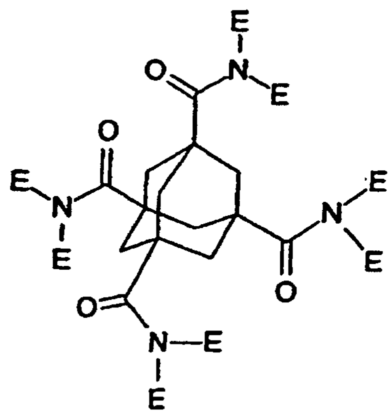

où

m et n représentent les nombres 1 à 10

p représente les nombres 0 à 10,

$U^1$ représente $Q^1$ ou E,

$U^2$ représente $Q^2$ ou E, où

E a la signification du groupe

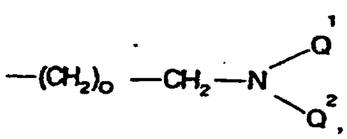

o représentant les nombres 1 à 6,

$Q^1$ représentant un atome d'hydrogène ou $Q^2$, et

$Q^2$ représentant une liaison directe,

M représente une chaîne alkylène en $C_1$-$C_{10}$ qui est éventuellement interrompue par 1 à 3 atomes d'oxygène et/ou est éventuellement substituée par 1 ou 2 groupes oxo,

$R^o$ représente un radical alkyle en $C_1$-$C_{10}$ ramifié ou non ramifié, un groupe nitro, amino, carboxy ou

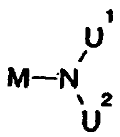

le symbole $Q^2$ correspondant à la multiplicité de base a.

**3.** Complexes de polymères en cascade selon la revendication 1, **caractérisés en ce que** le reste de complexant K lié aux atomes d'azote terminaux de la dernière génération d'une unité de reproduction W représente un radical de formule générale IA ou IB

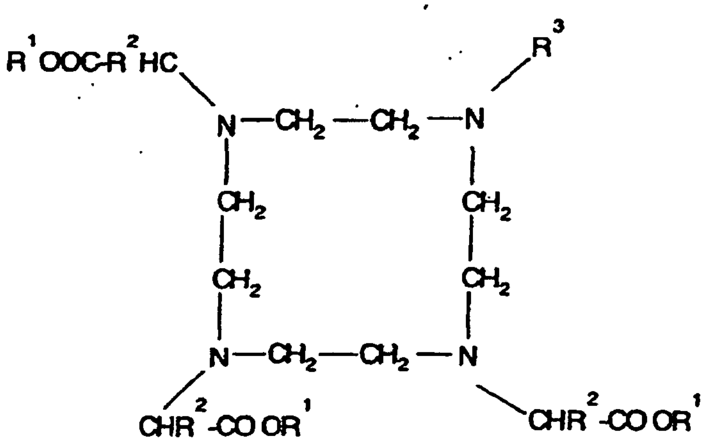

(IA),

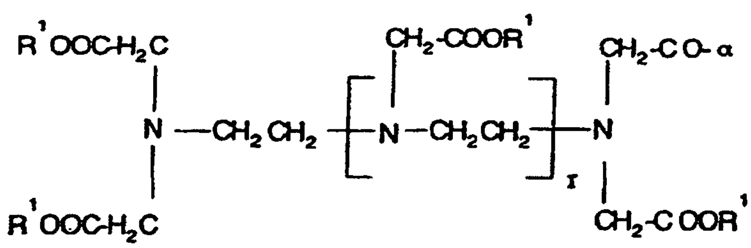

(IB)

formules dans lesquelles

$R^1$ représentent, indépendamment les uns des autres, un atome d'hydrogène ou un équivalent d'ion métallique des nombres atomiques 20-29, 39, 42-44 ou 57-83,

$R^2$ représente un atome d'hydrogène, un radical méthyle ou éthyle qui est éventuellement substitué par 1-2 groupes hydroxy ou un groupe carboxy,

$R^3$ représente un groupe $-CH_2-CH(OH)-U^6-T-$ ou $-CH_2-CO-U^7$,

$U^6$ représente un groupe alkylène en $C_1-C_{20}$ à chaîne droite ou ramifiée, saturé ou insaturé, contenant éventuellement 1-5 groupes imino, 1-3 groupes phénylène, 1-3 groupes phénylène-oxy, 1-3 groupes phénylène-imino, 1-5 groupes amido, 1-2 groupes hydrazide, 1-5 groupes carbonyle, 1-5 groupes éthylène-oxy, un groupe urée, un groupe thio-urée, 1-2 groupes carboxyalkylimino, 1-2 groupes ester, 1-10 atomes d'oxygène, 1-5 atomes de soufre et/ou 1-5 atomes d'azote et/ou éventuellement substitué par 1-5 groupes hydroxy, 1-2 groupes mercapto, 1-5 groupes oxo, 1-5 groupes thioxo, 1-3 groupes carboxy, 1-5 groupes carboxyalkyle, 1-5 groupes ester et/ou 1-3 groupes amino, les groupes phénylène éventuellement contenus pouvant être substitués par 1-2 groupes carboxy, 1-2 groupes sulfone ou 1-2 groupes hydroxy,

$U^7$ représente une liaison directe ou le radical $-NR^2-U^6-T$,

T représente un groupe -CO-α, -NHCO-α ou -NHCS-α,

α représente la position de liaison aux atomes d'azote terminaux de la dernière génération de l'unité de reproduction W, et

r représente le nombre 0, 1, 2 ou 3.

**4.** Complexes de polymères en cascade selon la revendication 3, **caractérisés en ce que** la chaîne alkylène en $C_1$-$C_{20}$ représentée par $U^6$ contient les groupes $-CH_2-$, $-CH_2NHCO-$, $-NHCOCH_2O-$, $NHCOCH_2OC_6H_4-$, $-N(CH_2CO_2H)-$, $-NHCOCH_2C_6H_4-$, $-NHCSNHC_6H_4-$, $-CH_2OC_6H_4-$, $-CH_2CH_2O-$ et/ou est substituée par les groupes $-COOH$, $-CH_2COOH$.

**5.** Complexes de polymères en cascade selon la revendication 3, **caractérisés en ce que** $U^6$ représente un groupe

$-CH_2-$, $-CH_2CH_2-$, $-CH_2CH_2CH_2-$, $-C_6H_4-$, $-C_6H_{10}-$, $-CH_2C_6H_5-$,
$-CH_2NHCOCH_2CH(CH_2CO_2H)-C_6H_4-$,
$-CH_2NHCOCH_2OCH_2-$,
$-CH_2NHCOCH_2C_6H_4-$,

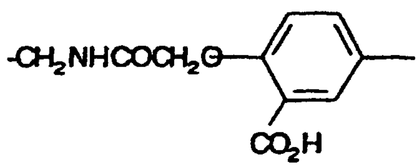

$-CH_2NHCSNH-C_6H_4-CH(CH_2COOH)CH_2-$,
$-CH_2OC_6H_4-N(CH_2COOH)CH_2-$,
$-CH_2NHCOCH_2O(CH_2CH_2O)_4-C_6H_4-$,
$-CH_2O-C_6H_4-$,
$-CH_2CH_2-O-CH_2CH_2-$, $-CH_2CH_2-O-CH_2CH_2-O-CH_2CH_2-$,

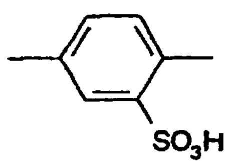 , 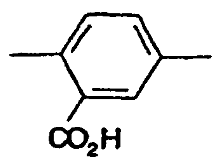 .

**6.** Complexes de polymères en cascade selon la revendication 1, **caractérisés en ce que** le radical $U^3$ contenu dans les unités de reproduction en cascade X, Y, Z et W représente $-CO-$, $-COCH_2OCH_2CO-$, $-COCH_2-$, $-CH_2CH_2-$, $-CONHC_6H_4-$, $-COCH_2CH_2CO-$, $-COCH_2-CH_2CH_2CO-$, $-COCH_2CH_2CH_2CH_2CO-$,
le radical $U^4$ représente une liaison directe ou $-CH_2CO-$, le radical $U^5$ représente une liaison directe ou $-(CH_2)_4-$, $-CH_2CO-$, $-CH(COOH)-$, $-CH_2OCH_2CH_2-$, $-CH_2C_6H_4-$, $-CH_2-C_6H_4OCH_2CH_2-$,
le radical E représente un groupe

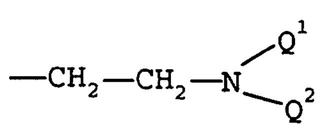

**7.** Complexes de polymères en cascade selon la revendication 1, **caractérisés en ce que** les unités de reproduction en cascade X, Y, Z et W représentent, indépendamment les unes des autres,

$-CH_2CH_2NH-$; $-CH_2CH_2N<$ ;
$-COCH(NH-)(CH_2)_4NH-$; $-COCH(N<)(CH_2)_4N<$ ;
$-COCH_2OCH_2CON(CH_2CH_2NH-)_2$; $-COCH_2OCH_2CON(CH_2CH_2N<)_2$ ;
$-COCH_2N(CH_2CH_2NH-)_2$; $-COCH_2N(CH_2CH_2N<)_2$;

$-COCH_2NH-$; $-COCH_2N<$ ;
$-COCH_2CH_2CON(CH_2CH_2NH-)_2$; $-COCH_2CH_2CON(CH_2CH_2N< )_2$ ;
$-COCH_2OCH_2CONH-C_6H_4-CH[CH_2CON(CH_2CH_2NH-)_2]_2$;
$-COCH_2OCH_2CONH-C_6H_4-CH[CH_2CON(CH_2CH_2N<)_2]_2$;
$-COCH_2CH_2CO-NH-C_6H_4-CH[CH_2CON(CH_2CH_2NH-)_2]_2$;
$-COCH_2CH_2CO-NH-C_6H_4-CH[CH_2CON(CH_2CH_2N<)_2]_2$;
$-CONH-C_6H_4-CH[CH_2CON(CH_2CH_2NH-)_2]_2$;
$-CONH-C_6H_4-CH[CH_2CON(CH_2CH_2N<)_2]_2$;
$-COCH(NH-)CH(COOH)NH-$; $-COCH(N<)CH(COOH)N<$ ;

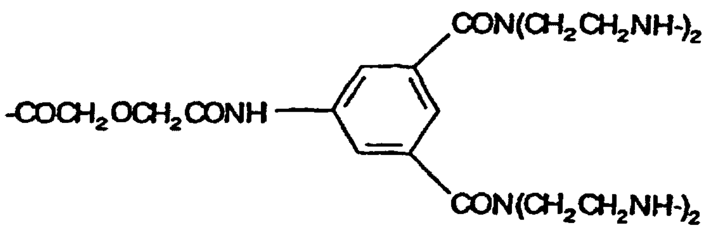
;

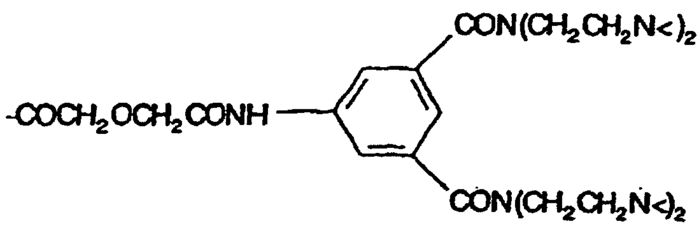
;

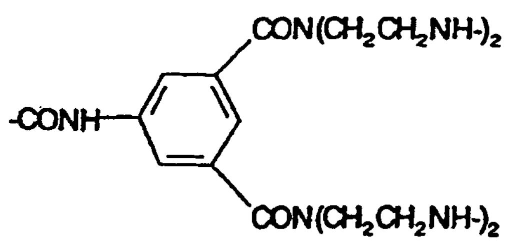
;

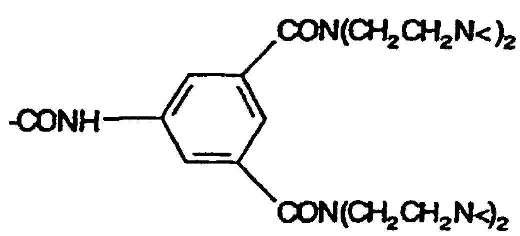
;

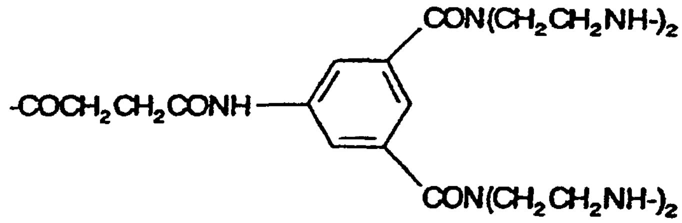

;

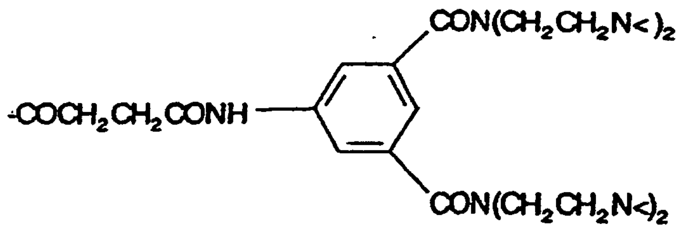

;

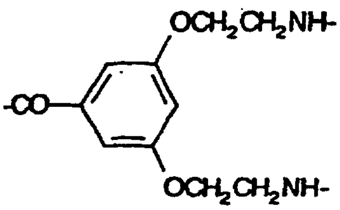

;

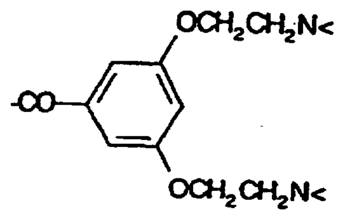

;

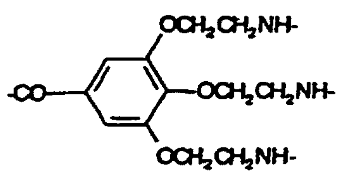

;

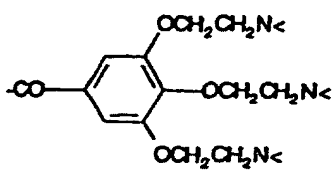

;

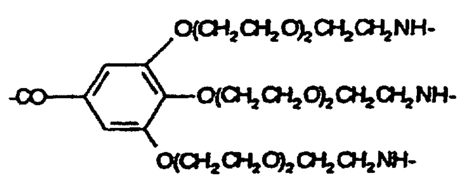

;

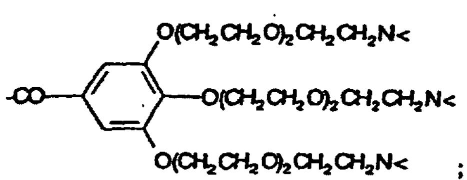

;

**8.** Complexes de polymères en cascade selon la revendication 2, **caractérisés en ce que**

- m représente un nombre allant de 1 à 3,
- n représente un nombre allant de 1 à 3,
- o représente le nombre 1,
- p représente le nombre 0-3,
- M représente un groupe $-CH_2-$, -CO- ou $-CH_2CO-$, et
- $R^o$ représente un groupe $-CH_2NU^1U^2$, $CH_3-$ ou $NO_2-$.

**9.** Produits pharmaceutiques contenant au moins un complexe de polymère en cascade selon la revendication 1, éventuellement avec les additifs usuels en formulation galénique.

10. Utilisation d'au moins un complexe de polymère selon la revendication 1 pour la préparation de produits pour le diagnostic radiographique ou par RMN.

11. Procédé pour la préparation de complexes de polymères en cascade selon les revendications 1 à 8, **caractérisé en ce qu'**on fait réagir des composés de formule générale I'

$$A\text{-}\{X\text{-}[Y\text{-}(Z\text{-}\langle W\text{-}\beta_w\rangle_z)_y]_x\}_a \qquad (I'),$$

dans laquelle

| | |
|---|---|
| A | représente un noyau de cascade azoté de multiplicité de base a, |
| X et Y | représentent, indépendamment l'un de l'autre, une liaison directe ou une unité de reproduction en cascade, de multiplicité de reproduction x ou y, |
| Z et W | représentent, indépendamment l'un de l'autre, une unité de reproduction en cascade de multiplicité de reproduction z ou w, |
| a | représente les nombres 2 à 12, |
| x, y, z et w | représentent, indépendamment les uns des autres, les nombres 1 à 4, et |
| β | représente la position de liaison du groupe NH- terminal de la dernière génération d'unité de reproduction W, |

étant entendu qu'au moins 2 unités de reproduction sont différentes et que, pour le produit des multiplicités, on a :

$16 \leq a.x.y.z.w \leq 64$,

avec un complexe ou un complexant K' de formule générale I'A ou I'B

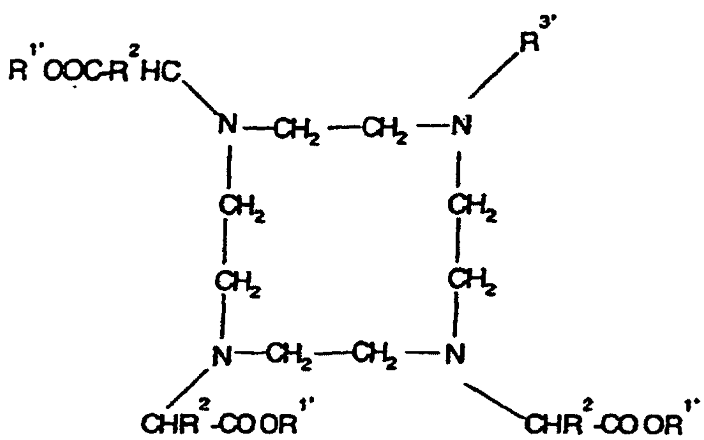

(I'A)

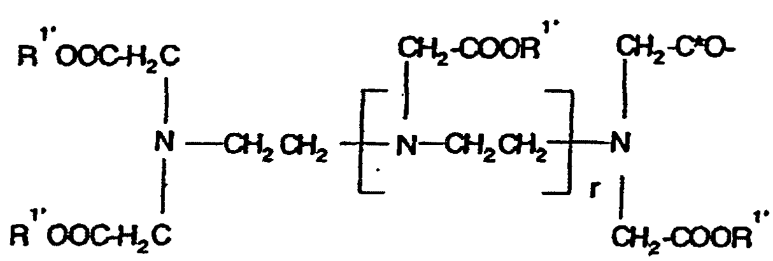

(I'B);

où

R[1'] représentent, indépendamment les uns des autres, un équivalent d'ions métalliques des nombres atomiques 20-29, 39, 42-44 ou 57-83 ou un groupe protecteur d'acide,

$R^2$ représente un atome d'hydrogène, un radical méthyle ou éthyle qui est éventuellement substitué par 1-2 groupes hydroxy ou un groupe carboxy,

$R^{3'}$ représente un groupe $-CH_2-CH(OH)-U^6-T'$ ou $-CH_2-CO-U^{7'}$,

$U^6$ représente un groupe alkylène en $C_1-C_{20}$ à chaîne droite ou ramifiée, saturé ou insaturé, contenant éventuellement 1-5 groupes imino, 1-3 groupes phénylène, 1-3 groupes phénylène-oxy, 1-3 groupes phénylène-imino, 1-5 groupes amino, 1-2 groupes hydrazide, 1-5 groupes carbonyle, 1-5 groupes éthylène-oxy, un groupe urée, un groupe thio-urée, 1-2 groupes carboxyalkylimino, 1-2 groupes ester, 1-10 atomes d'oxygène, 1-5 atomes de soufre et/ou 1-5 atomes d'azote et/ou éventuellement substitué par 1-5 groupes hydroxy, 1-2 groupes mercapto, 1-5 groupes oxo, 1-5 groupes thioxo, 1-3 groupes carboxy, 1-5 groupes carboxyalkyle, 1-5 groupes ester et/ou 1-3 groupes amino, les groupes phénylène éventuellement contenus pouvant être substitués par 1-2 groupes carboxy, 1-2 groupes sulfone ou 1-2 groupes hydroxy,

$U^{7'}$ représente une liaison directe ou le radical $-NR^2-U^6-T'$,

T' représente un groupe -C*O-, -COOH-, -N=C=O- ou -N=C=S-,

C*O représente un groupe carboxy activé et

r représente le nombre 0, 1, 2 ou 3, étant entendu que - lorsque K' représente un complexe - au moins deux (dans le cas de métaux bivalents) ou trois (dans le cas de métaux trivalents) des substituants $R^1$ représentent un équivalent d'ion métallique de l'élément précité et que, si on le désire, d'autres groupes carboxy se trouvent sous forme de leurs sels avec des bases organiques et/ou des bases minérales, des aminoacides ou des amides d'aminoacides,

éventuellement on élimine des groupes protecteurs, on fait réagir les polymères en cascade ainsi obtenus - lorsque K' représente un complexant - d'une façon connue en soi, avec au moins un oxyde métallique ou un sel métallique d'un élément des nombres atomiques 20-29, 39, 42, 44 ou 57-83, et éventuellement ensuite des atomes d'hydrogène acides encore présents dans les complexes de polymères en cascade ainsi obtenus sont remplacés en partie ou en totalité par des cations de bases organiques et/ou de bases minérales, d'aminoacides ou d'amides d'aminoacides, et éventuellement des groupes amino terminaux libres encore présents sont, si on le désire, acylés - avant ou après la complexation de métaux.

**12.** Procédé pour la préparation des produits pharmaceutiques selon la revendication 9, **caractérisé en ce qu'**on met sous une forme appropriée à l'administration entérale ou parentérale le complexe de polymère en cascade dissous ou mis en suspension dans de l'eau ou dans une solution physiologique de sel, éventuellement avec les additifs usuels en formulation galénique.

Fig. 1

Gemessene Blutkonzentrationen von Gd (Verbindung1), und Dy (Verbindung 2) in Ratten (n=5) mit abgebundenen Nierengefäßen